# EUROPEAN PATENT SPECIFICATION

(11) **EP 2 498 599 B1**
(45) Date of publication and mention of the grant of the patent: **20.08.2014**
(21) Application number: 09824133.4
(22) Date of filing: 29.10.2009
(51) Int. Cl.: A01N 33/18, A01N 33/24, A61K 31/445, A61P 29/00, C07D 215/06, C07D 277/04, C07D 209/12, C07D 265/36, C07D 205/04, C07D 243/08, C07D 217/04, C07D 211/14, C07D 295/088, C07D 211/74

(54) **ANALGESIC THAT BINDS FILAMIN A**
FILAMIN-A-BINDENDES ANALGETIKUM
ANALGÉSIQUE QUI SE LIE À LA FILAMINE A

(30) Priority: 28.10.2009 US 607883; 04.05.2009 US 435284; 31.10.2008 US 263257
(43) Date of publication of application: 19.09.2012
(73) Proprietor: Pain Therapeutics, Inc., San Mateo, CA 94404 (US)
(72) Inventor: BURNS BARBIER, Lindsay, Austin, Texas 78731 (US); WANG, Hoau-Yan, Philadelphia PA 19128 (US); LIN, Nan-Horng, Vernon Hills IL 60061 (US); BLASKO, Andrei, San Bruno CA 94066 (US)
(74) Representative: Carpmaels & Ransford LLP
(86) International application number: PCT/US2009/062579
(87) International publication number: WO 2010/051374

(56) References cited:
- WO-A2-2004/071417
- US-A1- 2005 075 505
- US-A1- 2007 111 970
- ZHANG W ET AL: "Fluorous electrophilic scavengers for solution-phase parallel synthesis", TETRAHEDRON LETTERS, ELSEVIER, AMSTERDAM, NL, vol. 44, no. 10, 3 March 2003 (2003-03-03) , pages 2065-2068, XP004410072, ISSN: 0040-4039, DOI: 10.1016/S0040-4039(03)00178-3
- K. A. TOENJES ET AL: "Small-Molecule Inhibitors of the Budded-to-Hyphal-Form Transition in the Pathogenic Yeast Candida albicans", ANTIMICROBIAL AGENTS AND CHEMOTHERAPY, vol. 49, no. 3, 1 March 2005 (2005-03-01), pages 963-972, XP055060743, ISSN: 0066-4804, DOI: 10.1128/AAC.49.3.963-972.2005

### SEQUENCE LISTING

<110> PAIN THERAPEUTICS, INC
<120> ANALGESIC THAT BINDS FILAMIN A
<130> P059548EP
<150> US 12/607,883
   <151> 2009-10-28
<150> US 12/435,284
   <151> 2009-05-04
<150> US 12/263,257
   <151> 2008-10-31
<160> 1
<170> PatentIn version 3.5
<210> 1
   <211> 5
   <212> PRT
   <213> Artificial Sequence
<220>
   <223> chemically synthesized FLNA sequence that corresponds to amino acid
   residue positions 2561-2565 of the FLNA protein
<400> 1
   Val Ala Lys Gly Leu
   1 5

## Description

### TECHNICAL FIELD

This invention contemplates a composition and related method for providing long-lasting analgesia and reducing inflammation. More particularly, a compound, composition and method are described that utilize a small molecule to bind filamin A, to reduce inflammation and to preserve Gi/o signaling by the mu opioid receptor, known to interact with filamin A. Preferably, the compound reduces inflammation, preserves mu opioid receptor - Gi/o signaling and also functions as a mu opioid receptor agonist. Most preferably, the compound binds filamin A with picomolar or sub-picomolar affinity.

### BACKGROUND OF THE INVENTION

Best known for cross-linking cytoplasmic actin into dynamic scaffolds to control cell motility, filamins are large cytoplasmic proteins increasingly found to regulate cell signaling by interacting with over 30 different receptors and signaling molecules (Feng et al., 2004 Nat Cell Biol 6:1034-1038; Stossel et al., 2001 Nature 2:138-145), including the mu opioid receptor (MOR) (Onoprishvili et al, 2003 Mol Pharmacol 64:1092-1100). Filamins are dimerized through the last carboxy-terminal repeat near the transmembrane regions, allowing an intracellular V-shaped structure that is critical for function. There are three mammalian isoforms: filamin A (FLNA), B and C.

FLNA controls cell motility by controlling the cycle of actin polymerization and depolymerization, allowing cells to move and to migrate. As actin depolymerization is linked to the inflammatory response, binding to FLNA suppresses inflammation by slowing actin polymerization and cell motility. Femtomolar naloxone and its inactive isomer, both known to bind FLNA (Wang et al., 2008 PLoS One 3:e1554), have been shown to reduce the microglial inflammatory response; i.e., pro-inflammatory factors and reactive oxygen species, of lipopolysaccaride-activated microglial cells (Liu et al, 2000 JPET 293:607-617; Qin et al., 2005 FASEB J 19:550-557). The glial inflammatory response has been implicated in neuropathic pain (Hutchinson et al., 2008 Eur J Neurosci 28:20-29) as well as the inflammatory neurotoxicity of neurodegenerative disease (Liu et al., 2003 JPET 304:1-7).

A second function of binding to FLNA is a beneficial regulation of opioid receptor signaling; i.e., a maintenance of coupling to Gi and Go proteins. MOR preferentially couples to pertussis toxin-sensitive G proteins, Gi/o (inhibitory/other), and inhibits the adenylyl cyclase/cAMP pathway (Laugwitz et al., 1993 Neuron 10:233-242; Connor et al., 1999 Clin Exp Pharmacol Physiol 26:493-499). Analgesia results from these MOR-linked inhibitory G protein (Gi/o) signaling cascades and related ion channel interactions that suppress cellular activities by hyperpolarization.

Adaptive responses of opioid receptors contribute to the development of analgesic tolerance and physical dependence, and possibly also to components of opioid addiction. A critical adaptive response of the MOR is a switch in G protein coupling from its native Gi/o proteins to stimulatory Gs proteins, resulting in opposite effects on the cell upon activation as well as analgesic tolerance and physical dependence (Wang et al., 2005 Neuroscience 135:247-261). Prevention of this G protein coupling switch by agents that bind filamin A (Wang et al, 2008 FLoS One 3:e1554), a scaffolding protein known to interact with MOR, can alleviate unwanted adaptive responses to continued opioid administration.

A chronic opioid-induced switch to Gs coupling by MOR can cause excitatory signaling, by activation of adenylyl cyclase, in place of the usual inhibitory signaling or inhibition of adenylyl cyclase (Crain et al., 1992 Brain Res 575:13-24; Crain et al., 2000 Pain 84:121-131; Gintzler et al., 2001 Mol Neurobiol 21:21-33; Wang et al., 2005 Neuroscience 135:247-261). This switch in G protein coupling from Gi/o to Gs (Wang et al., 2005 Neuroscience 135:247-261; Chakrabarti et al., 2005 Mol Brain Res 135:217-224) may be a result of the decreased efficiency of coupling to the native G proteins, the usual index of desensitization (Sim et al., 1996 J Neurosci 16:2684-2692) and still commonly considered the reason for analgesic tolerance.

The chronic opioid-induced MOR - G protein coupling switch is accompanied by stimulation of adenylyl cyclase II and IV by MOR-associated Gβγ dimers (Chakrabarti et al., 1998 Mol Pharmacol 54:655-662; Wang et al., 2005 Neuroscience 135:247-261). The interaction of the Gβγ dimer with adenylyl cyclase had previously been postulated to be the sole signaling change underlying the excitatory effects of opiates (Gintzler et al., 2001 Mol Neurobiol 21:21-33). It has further been shown that the Gβγ that interacts with adenylyl cyclases originates from the Gs protein coupling to MOR and not from the Gi/o proteins native to MOR (Wang et al., 2006 J Neurobiol 66:1302-1310).

Thus, MORs are normally inhibitory G protein-coupled receptors that couple to Gi or Go proteins to inhibit adenylyl cyclase and decrease production of the second messenger cAMP, as well as to suppress cellular activities via ion channel-mediated hyperpolarization. Opioid analgesic tolerance and dependence are also associated with that switch in G protein coupling by MOR from Gi/o to Gs (Wang et al., 2005 Neuroscience 135:247-261). This switch results in activation of adenylyl cyclase that provides essentially opposite, stimulatory, effects on the cell.

Controlling this switch in G protein coupling by MOR is the scaffolding protein FLNA, and compounds that bind a particular segment of FLNA with high affinity, like naloxone (NLX) and naltrexone (NTX), can prevent this switch (Wang et al, 2008 PLoS One 3:e1554) and the associated analgesic tolerance and dependence(Wang et al., 2005 Neuroscience 135:247-261). This switch in G protein coupling also occurs acutely, though transiently, and is potentially linked to the acute rewarding or addictive effects of opioid drugs, through CREB activation as a result of increased cAMP accumulation (Wang et al., 2009 PLoS ONE 4(1):e4282).

Ultra-low-dose NLX or NTX have been shown to enhance opioid analgesia, minimize opioid tolerance and dependence (Crain et al., 1995 Proc Natl Acad Sci USA 92:10540-10544; Powell et al. 2002. JPET 300:588-596), as well as to attenuate the addictive properties of opioids (Leri et al., 2005 Pharmacol Biochem Behav 82:252-262; Olmstead et al., 2005 Psychopharmacology 181:576-581). An ultra-low dose of opioid antagonist was an amount initially based on *in vitro* studies of nociceptive dorsal root ganglion neurons and on *in vivo* mouse studies. It has long been hypothesized that ultra-low-dose opioid antagonists enhance analgesia and alleviate tolerance/dependence by blocking the excitatory signaling opioid receptors that underlie opioid tolerance and hyperalgesia (Crain et al., 2000 Pain 84:121-131). Later research has shown that the attenuation of opioid analgesic tolerance, dependence and addictive properties by ultra-low-dose, defined herein, naloxone or naltrexone, occurs by preventing the MOR - Gs coupling that results from chronic opiate administration (Wang et al., 2005 Neuroscience 135:247-261), and that the prevention of MOR - Gs coupling is a result of NLX or NTX binding to filamin A at approximately 4 picomolar affinity (Wang et al, 2008 PLoS One 3:e1554).

Found in all cells of the brain, CREB is a transcription factor implicated in addiction as well as learning and memory and several other experience-dependent, adaptive (or maladaptive) behaviors (Carlezon et al., 2005 Trends Neurosci 28:436-445). In general, CREB is inhibited by acute opioid treatment, an effect that is completely attenuated by chronic opioid treatment, and activated during opioid withdrawal (Guitart et al., 1992 J Neurochem 58:1168-1171). However, a regional mapping study showed that opioid withdrawal activates CREB in locus coeruleus, nucleus accumbens and amygdala but inhibits CREB in lateral ventral tegemental area and dorsal raphe nucleus (Shaw-Luthman et al., 2002 J Neurosci 22:3663-3672).

In the striatum, CREB activation has been viewed as a homeostatic adaptation, attenuating the acute rewarding effects of drugs (Nestler, 2001 Am J Addict 10:201-217; Nestler, 2004 Neuropharmacology 47:24-32). This view is supported by nucleus accumbens overexpression of CREB or a dominant-negative mutant respectively reducing or increasing the rewarding effects of opioids in the conditioned place preference test (Barot et al., 2002 Proc Natl Acad Sci USA 99:11435-11440). In conflict with this view, however, is the finding that reducing nucleus accumbens CREB via antisense attenuated cocaine reinforcement as assessed in self-administration (Choi et al., 2006 Neuroscience 137:373-383). Clearly, CREB activation is implicated in addiction, but whether it directly contributes to the acute rewarding effects of drugs or initiates a homeostatic regulation thereof appears less clear.

The several-fold increase in pS¹³³CREB reported by Wang et al., 2009 PLoS ONE 4(1):e4282 following acute, high-dose morphine may indicate acute dependence rather than acute rewarding effects. However, the transient nature of the MOR-Gs coupling correlating with this CREB activation suggests otherwise. In fact, the correlation of pS¹³³CREB with the Gs coupling by MOR following this acute high-dose morphine exposure, as well as the similar treatment effects on both, suggest that this alternative signaling mode of MOR can contribute to the acute rewarding or addictive effects of opioids. This counterintuitive notion can explain the apparent paradox that ultra-low-dose NTX, while enhancing the analgesic effects of opioids, decreases the acute rewarding or addictive properties of morphine or oxycodone as measured in conditioned place preference or self-administration and reinstatement paradigms.

In considering analgesic tolerance, opioid dependence, and opioid addiction together as adaptive regulations to continued opioid exposure, a treatment that prevents MOR's signaling adaptation of switching its G protein partner can logically attenuate these seemingly divergent behavioral consequences of chronic opioid exposure.

Even though ultra-low-dose NTX blocks the conditioned place preference to oxycodone or morphine (Olmstead et al., 2005 Psychopharmacology 181:576-581), its co-self-administration only reduces the rewarding potency of these opioids but does not abolish self-administration outright (Leri et al., 2005 Pharmacol Biochem Behav 82:252-262). It is possible that a direct stimulatory effect on VTA neurons, as opposed to the proposed disinhibition via inhibition of GABA interneurons (Spanagel et al., 1993 Proc Natl Acad Sci USA 89:2046-2050), can play some role in opioid reward. A MOR-Gs coupling mediation of reward, increasing with increasing drug exposure, is in keeping with current theories that the escalation of drug use signifying drug dependence can not indicate a "tolerance" to rewarding effects but instead a sensitization to rewarding effects (Zernig et al., 2007 Pharmacology 80:65-119).

The results reported in Wang et al., 2009 PLoS ONE 4(1):e4282 demonstrated that acute, high-dose morphine causes an immediate but transient switch in G protein coupling by MOR from Go to Gs similar to the persistent switch caused by chronic morphine. Ultra-low-dose NLX or NTX prevented this switch and attenuated the chronic morphine-induced coupling switch by MOR. The transient nature of this acute altered coupling suggests the receptor eventually recovers and couples to its native G protein.

With chronic opioid exposure, the receptor can lose the ability to recover and continue to couple to Gs, activating the adenylyl cyclase/cAMP pathway, upregulating protein kinase A, and phosphorylating CREB as one downstream effector example. The persistently elevated phosphorylated CREB can then shape the expression of responsive genes including those closely related to drug addiction and tolerance. Importantly, the equivalent blockade of Gs coupling and pS¹³³CREB by the pentapeptide binding site of naloxone (NLX) and naltrexone (NTX) on FLNA further elucidates the mechanism of action of ultra-low-dose NLX and NTX in their varied effects.

These data further strengthen the regulation of MOR-Gs coupling by FLNA and that binding to FLNA or using a FLNA peptide decoy for MOR can prevent the altered MOR coupling, thereby attenuating tolerance, dependence and addictive properties associated with opioid drugs.

The combination of ultra-low-dose opioid antagonists with opioid agonists formulated together in one medication has been shown to alleviate many of these undesirable aspects of opioid therapy (Burns, 2005 Recent Developments in Pain Research 115-136, ISBN:81-308-0012-8). This approach shows promise for an improvement in analgesic efficacy, and animal data suggests reduced addictive potential. The identification of the cellular target of ultra-low-dose NLX or NTX in their inhibition of mu opioid receptor - Gs coupling as a pentapeptide segment of filamin A (Wang et al., 2008 PLoS ONE 3(2):e1554) has led to development of assays to screen against this target to create a new generation of pain therapeutics that can provide long-lasting analgesia with minimal tolerance, dependence and addictive properties. Importantly, the non-opioid cellular target of ultra-low-dose NLX or NTX, FLNA, provides potential for developing either a therapeutic combination of which one component is not required to be ultra-low-dose, or a single-entity novel analgesic.

The present invention identifies a compound that binds to filamin A (FLNA; the high-affinity binding site of naloxone [NLX] and naltrexone [NTX]), to reduce cell motility and inflammation as well as to prevent the Gi/o-to-Gs coupling switch of MOR and is similar to or more active than DAMGO in activating MOR.

Zhang et al. (2003 Tetrahedron Letters 44:2065-2068) describe a synthesis of β-hydroxyamine analogs from amine and epoxide starting materials. Fluorous isatoic anhydride and isocyanate scavengers were used in the procedure.

Toenjes et al. (WO 2004/071417 and 2005 Antimicrob. Agents Chemother. 49:963-972) describe β-hydroxyamine analogs that inhibit the budded-to-hyphal transition of *Candida albicans* and their use in antifungal therapies.

### BRIEF SUMMARY OF THE INVENTION

The present invention contemplates an analgesic compound, a composition containing that compound and a method of reducing pain in a host mammal in need thereof by administering a composition containing such a compound. A contemplated compound corresponds in structure to Formula **A** or its pharmaceutically acceptable salt 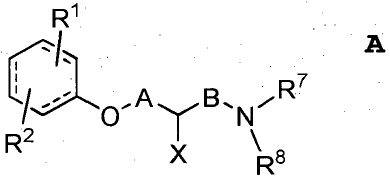 In a compound of Formula **A,** R¹ and R² are the same or different and are independently H, halogen, C₁-C₁₂ hydrocarbyl, C₁-C₆ acyl, C₁-C₆ hydrocarbyloxy, CF₃ and NR³R⁴ wherein R³ and R⁴ are the same or different and are H, C₁-C₄ hydrocarbyl, C₁-C₄ acyl, C₁-C₄ hydrocarbylsulfonyl, or R³ and R⁴ together with the depicted nitrogen form a 5-7-membered ring that optionally contains 1 or 2 additional hetero atoms that independently are nitrogen, oxygen or sulfur. **A** and **B** are the same or different and are CH₂, CDH or CD_{2.} X is OH or NR⁵R⁶ wherein R⁵ and R⁶ are the same or different and are H, C₁-C₄ hydrocarbyl, C₁-C₄ acyl, C₁-C₄ hydrocarbylsulfonyl, or R⁵ and R⁶ together with the depicted nitrogen form a 5-7-membered ring that optionally contains 1 or 2 additional hetero atoms that independently are nitrogen, oxygen or sulfur. In NR⁷R⁸, R⁷ and R⁸ together with the depicted nitrogen form a ring structure **W. W** contains 4 to 14 atoms in the ring structure including the depicted nitrogen, and preferably up to 12 atoms. **W** contains: a) 1, 2 or 3 further hetero atoms that are independently oxygen, nitrogen or sulfur, and b) one or more substituent groups bonded to one or more ring atoms, in which the one or more substituents contain a total of up to 8 atoms, and preferably up to 6 atoms, selected from the group consisting of carbon, nitrogen, oxygen and sulfur, and mixtures thereof. A dotted line (----) represents an optional double bond. In regard to a contemplated compound, R¹ and R² are other than methyl and isopropyl, respectively, when **W** is dimethyl-N-morpholinyl and the optional double bonds are absent.

A preferred compound of Formula **A** is a compound of Formula **I** in which **A, B, X,** W and R¹ and R² are as defined above.

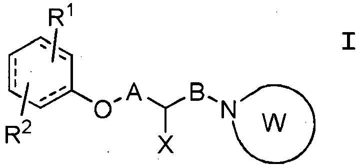

In one preferred embodiment, a contemplated compound corresponds in structure to Formula **Ia** 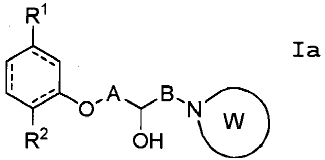 Here, R¹ and R² are the same or different and are independently H, or C₁-C₆ hydrocarbyl; **A** and **B** are the same or different and are CH₂, CDH or CD_{2;} X is OH or X is OH or NR⁵R⁶ wherein R⁵ and R⁶ are the same or different and are H, C₁-C₄ hydrocarbyl, C₁-C₄ acyl, C₁-C₄ hydrocarbylsulfonyl, or R⁵ and R⁶ together with the depicted nitrogen form a 5-7-membered ring that optionally contains 1 or 2 additional hetero atoms that independently are nitrogen, oxygen or sulfur. **W** is a ring structure that contains 4 to 12 atoms in the ring structure including the depicted nitrogen, and: a) contains 1, 2 or 3 further hetero atoms that are independently oxygen, nitrogen or sulfur, and b) includes one or more substituent groups bonded to one or more ring atoms, in which the one or more substituent contain a total of up to 8 atoms selected from the group consisting of carbon, nitrogen, oxygen and sulfur, and mixtures thereof. The dotted line (----) represents 1, 2, or 3 optional double bonds, and R¹ and R² are other than methyl and isopropyl, respectively, when **W** is dimethyl-N-morpholinyl, and the optional double bonds are absent.

In one preferred embodiment, a compound of Formulas **I** and **Ia** has the structure of Formula **II,** whereas in another preferred embodiment, a compound of Formulas **I** and **Ia** has the structure of a compound of Formula **III.** 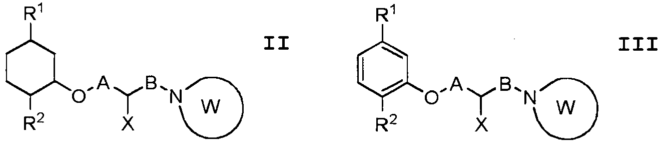 In a compound of both Formulas **II** and **III, A, B, W and X** are as previously defined for a compound of Formulas **I** and **Ia.** R¹ and R² for a compound of Formula **II** are defined as R¹ and R² for a compound of Formula **Ia,** whereas R¹ and R² for a compound of Formula **III** are defined as R¹ and R² for a compound of Formula **I.**

More preferably, the R¹ and R² groups of a compound of Formula **II** contain 3 to 5 carbon atoms. For some compounds of Formula **III,** R¹ is H and R² is halogen, C₁-C₆ hydrocarbyl, C₁-C₆ acyl, C₁-C₆ hydrocarbyloxy or NR³R⁴, whereas for others, both R groups are other than H.

In a compound of either Formula **II** or Formula **III, W** can contain 1 or 2 further hetero atoms that are independently oxygen, nitrogen or sulfur.

A particularly preferred compound of Formulas **II** and **III** has a structure of Formulas **IIa** and **IIIa,** wherein the other groups **A, B, W,** R¹ and R² are as defined above.

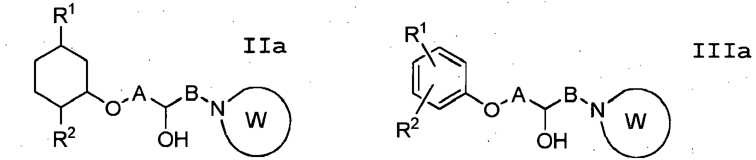

A pharmaceutically acceptable salt of a compound of each of the above Formulas is also contemplated.

A pharmaceutical composition is also contemplated. That composition comprises an above compound of Formula **A** or a pharmaceutically acceptable salt thereof dissolved or dispersed in a physiologically tolerable carrier. The compound or salt is present in an effective analgesic amount. The composition is preferably in solid form as in a tablet of capsule.

Compounds for use in a method of reducing one or both of pain and inflammation in a host mammal in need thereof are also contemplated. A contemplated use comprises administering to a host mammal host in need thereof a pharmaceutical composition containing an effective amount of a compound of Formula **A** or its pharmaceutically acceptable salt dissolved or dispersed in a physiologically tolerable carrier to reduce one or both of pain and inflammation in the host. A contemplated composition is typically administered a plurality of times over a period of days, and is preferably administered a plurality of times in one day. That administration can be perorally or parenterally.

The present invention has several benefits and advantages.

One benefit is anti-inflammatory action combined with analgesia by a compound with a novel mechanism of action for both that does not have a narcotic structure.

An advantage of the invention is that analgesia can be provided by administration of a contemplated composition either perorally or parenterally.

A further benefit of the invention is that as indicated by the initial data, a contemplated compound provides the analgesic effects characteristic of opioid drugs but does not cause analgesic tolerance or dependence.

Another advantage of the invention as also indicated by the initial data is that a contemplated compound provides the analgesic effects and does not have the addictive potential of opioid drugs.

Still further benefits and advantages will be apparent to a skilled worker from the description that follows.

### ABBREVIATIONS AND SHORT FORMS

The following abbreviations and short forms are used in this specification.
"MOR" means µ opioid receptor
"FLNA" means filamin A
"NLX" means naloxone
"NTX" means naltrexone
"Gi/o" means G protein inhibitory/other subtype, inhibits adenylyl cyclase
"Gs" means G protein stimulatory subtype, stimulates adenylyl cyclase
"Gβγ" means G protein beta gamma subunit
"cAMP" means cyclic adenosine monophosphate
"CREB" means cAMP Response Element Binding protein
"IgG" means Immunoglobulin G

### DEFINITIONS

In the context of the present invention and the associated claims, the following terms have the following meanings:

The articles "a" and "an" are used herein to refer to one or to more than one (i.e., to at least one) of the grammatical object of the article. By way of example, "an element" means one element or more than one element.

As used herein, the term "hydrocarbyl" is a short hand term to include straight and branched chain aliphatic as well as alicyclic groups or radicals that contain only carbon and hydrogen. Thus, alkyl, alkenyl and alkynyl groups are contemplated, whereas aromatic hydrocarbons such as phenyl and naphthyl groups, which strictly speaking are also hydrocarbyl groups, are referred to herein as aryl groups, substituents, moieties or radicals, as discussed hereinafter. An aralkyl group such as benzyl or phenethyl is deemed a hydrocarbyl group. Where a specific aliphatic hydrocarbyl substituent group is intended, that group is recited; i.e., C₁-C₄ alkyl, methyl or dodecenyl. Exemplary hydrocarbyl groups contain a chain of 1 to about 12 carbon atoms, and preferably one to about 7 carbon atoms, and preferably 1 to about 7 carbon atoms, and more preferably 1 to 4 carbon atoms of an alkyl group.

A particularly preferred hydrocarbyl group is an alkyl group. As a consequence, a generalized, but more preferred substituent can be recited by replacing the descriptor "hydrocarbyl" with "alkyl" in any of the substituent groups enumerated herein.

Examples of alkyl radicals include methyl, ethyl, n-propyl, isopropyl, n-butyl, isobutyl, sec-butyl, tert-butyl, pentyl, iso-amyl, hexyl, octyl, decyl, dodecyl and the like. Examples of suitable alkenyl radicals include ethenyl (vinyl), 2-propenyl, 3-propenyl, 1,4-pentadienyl, 1,4-butadienyl, 1-butenyl, 2-butenyl, 3-butenyl, decenyl and the like. Examples of alkynyl radicals include ethynyl, 2-propynyl, 3-propynyl, decynyl, 1-butynyl, 2-butynyl, 3-butynyl, and the like.

Usual chemical suffix nomenclature is followed when using the word "hydrocarbyl" except that the usual practice of removing the terminal "yl" and adding an appropriate suffix is not always followed because of the possible similarity of a resulting name to one or more substituents. Thus, a hydrocarbyl ether is referred to as a "hydrocarbyloxy" group rather than a "hydrocarboxy" group as may possibly be more proper when following the usual rules of chemical nomenclature. Illustrative hydrocarbyloxy groups include methoxy, ethoxy, and cyclohexenyloxy groups. On the other hand, a hydrocarbyl group containing a -C(O)-functionality is referred to as a hydrocarboyl (acyl) group and that containing a -C(O)O- is a hydrocarboyloxy group inasmuch as there is no ambiguity. Exemplary hydrocarboyl and hydrocarboyloxy groups include acyl and acyloxy groups, respectively, such as acetyl and acetoxy, acryloyl and acryloyloxy.

A "carboxyl substituent is a -C (O) OH group. A C₁-C₆ hydrocarbyl carboxylate is a C₁-C₆ hydrocarbyl ester of a carboxyl group. A carboxamide is a -C(O)NR³R⁴ substituent, where the R groups are defined elsewhere. Illustrative R³ and R⁴ groups that together with the depicted nitrogen of a carboxamide form a 5-7-membered ring that optionally contains 1 or 2 additional hetero atoms that independently are nitrogen, oxygen or sulfur, include morpholinyl, piprazinyl, oxathiazolyl, 1,2,3-triazolyl, 1,2,4-triazolyl, pyrazolyl, 1,2,4-oxadiazinyl and azepinyl groups.

As a skilled worker will understand, a substituent that cannot exist such as a C₁ alkenyl or alkynyl group is not intended to be encompassed by the word "hydrocarbyl", although such substituents with two or more carbon atoms are intended.

The term "aryl", alone or in combination, means a phenyl or naphthyl radical that optionally carries one or more substituents selected from hydrocarbyl, hydrocarbyloxy, halogen, hydroxy, amino, nitro and the like, such as phenyl, p-tolyl, 4-methoxyphenyl, 4-(tert-butoxy)phenyl, 4-fluorophenyl, 4-chlorophenyl, 4-hydroxyphenyl, and the like. The term "arylhydrocarbyl", alone or in combination, means a hydrocarbyl radical as defined above in which one hydrogen atom is replaced by an aryl radical as defined above, such as benzyl, 2-phenylethyl and the like. The term "arylhydrocarbyloxycarbonyl", alone or in combination, means a radical of the formula -C(O)-O-arylhydrocarbyl in which the term "arylhydrocarbyl" has the significance given above. An example of an arylhydrocarbyloxycarbonyl radical is benzyloxycarbonyl. The term "aryloxy" means a radical of the formula aryl-O- in which the term aryl has the significance given above. The term "aromatic ring" in combinations such as substituted-aromatic ring sulfonamide, substituted-aromatic ring sulfinamide or substituted-aromatic ring sulfenamide means aryl or heteroaryl as defined above.

As used herein, the term "binds" refers to the adherence of molecules to one another, such as, but not limited to, peptides or small molecules such as the compounds disclosed herein, and opioid antagonists, such as naloxone or naltrexone.

As used herein, the term "selectively binds" refers to binding as a distinct activity. Examples of such distinct activities include the independent binding to filamin A or a filamin A binding peptide, and the binding of a compound discussed above to a µ opioid receptor.

As used herein, the term "FLNA-binding compound" refers to a compound that binds to the scaffolding protein FLNA, or more preferably to a polypeptide comprising residues -Val-Ala-Lys-Gly-Leu-(SEQ ID NO:1) of the FLNA sequence that correspond to amino acid residue positions 2561-2565 of the FLNA protein sequence as noted in the sequence provided at the web address: UniProtKB/Swiss-Prot entry P21333, FLNA-HUMAN, Filamin-A protein sequence. A FLNA-binding compound can inhibit the MOR-Gs coupling caused by agonist stimulation of MOR via interactions with FLNA, preferably in the 24^{th} repeat region. When co-administered with an opioid agonist, a FLNA-binding compound can enhance the analgesic effects and improve the treatment of pain.

As used herein, the term "candidate FLNA-binding compound" refers to a substance to be screened as a potential FLNA-binding compound. In preferred instances a FLNA-binding compound is also an opioid agonist. Additionally, a FLNA-binding compound can function in a combinatory manner similar to the combination of an opioid agonist and ultra-low-dose antagonist, wherein both FLNA and MOR are targeted by a single entity.

As used herein, the term "opioid receptor" refers to a G protein coupled receptor, located in the central nervous system that interacts with opioids. More specifically, the µ opioid receptor is activated by morphine causing analgesia, sedation, nausea, and many other side effects known to one of ordinary skill in the art.

As used herein, the term "opioid agonist" refers to a substance that upon binding to an opioid receptor can stimulate the receptor, induce G protein coupling and trigger a physiological response. More specifically, an opioid agonist is a morphine-like substance that interacts with MOR to produce analgesia.

As used herein, the term "opioid antagonist" refers to a substance that upon binding to an opioid receptor inhibits the function of an opioid agonist by interfering with the binding of the opioid agonist to the receptor.

As used herein an "analgesia effective amount" refers to an amount sufficient to reduce pain and inflammation a recipient host.

As used herein the term "ultra-low-dose" or "ultra-low amount" refers to an amount of compound that when given in combination with an opioid agonist is sufficient to enhance the analgesic potency of the opioid agonist. More specifically, the ultra-low-dose of an opioid antagonist is admixed with an opioid agonist in an amount about 1000- to about 10,000,000-fold less, and preferably about 10,000- to about 1,000,000-fold less than the amount of opioid agonist.

As used herein an "FLNA-binding effective amount" refers to an amount sufficient to perform the functions described herein, such as reduction or prevention of inflammation, inhibition of MOR-Gs coupling, prevention of the cAMP desensitization measure, inhibition of CREB S¹³³ phosphorylation and inhibition of any other cellular indices of opioid tolerance and dependence, which functions can also be ascribed to ultra-low-doses of certain opioid antagonists such as naloxone or naltrexone. When a polypeptide or FLNA-binding compound of the invention interacts with FLNA, an FLNA-binding effective amount can be an ultra-low amount or an amount higher than an ultra-low-dose as the polypeptide or FLNA-binding compound will not antagonize the opioid receptor and compete with the agonist, as occurs with known opioid antagonists such as naloxone or naltrexone in amounts greater than ultra-low-doses. More preferably, when a polypeptide or VAKGL-binding compound of the present invention both interacts with FLNA and is an agonist of the µ opioid receptor, an FLNA-binding effective amount is an amount higher than an ultra-low-dose and is a sufficient amount to activate the µ opioid receptor.

As used herein the phrase "determining inhibition of the interaction of MOR with a Gs protein" refers to monitoring the cellular index of opioid tolerance and dependence caused by chronic or high-dose administration of opioid agonists to mammalian cells. More specifically, the mu opioid receptor - Gs coupling response can be identified by measuring the presence of the Gas (stimulatory) subunit, the interaction of MOR with the G protein complexes and formation of Gs-MOR coupling, the interaction of the Gβγ protein with adenylyl cyclase types II and IV, loss of inhibition or outright enhancement of cAMP accumulation, and the activation of CREB via phosphorylation of S¹³³.

As used herein the term "naloxone/naltrexone positive control" refers to a positive control method comprising steps discussed in a method embodiment, wherein the candidate FLNA-binding compound is a known opioid antagonist administered in an ultra-low amount, preferably naloxone or naltrexone.

As used herein the term "FLNA-binding compound negative control" refers to a negative control method comprising steps discussed in a method embodiment, wherein the candidate FLNA-binding compound is absent and the method is carried out in the presence of only opioid agonist.

As used herein the term "pharmacophore" is not meant to imply any pharmacological activity. The term refers to chemical features and their distribution in three-dimensional space that constitutes and epitomizes the preferred requirements for molecular interaction with a receptor (U.S. Patent No. 6,034,066).

### DETAILED DESCRIPTION OF THE INVENTION

It should be understood that the present disclosure is to be considered as an exemplification of the present invention, and is not intended to limit the invention to the specific embodiments illustrated. It should be further understood that the title of this section of this application ("Detailed Description of the Invention") relates to a requirement of the United States Patent Office, and should not be found to limit the subject matter disclosed herein.

The present invention contemplates a compound that binds to FLNA and also stimulates MOR, a composition containing that compound and a method of its use to provide one or both of pain relief and reduction of inflammation. A contemplated compound can suppress inflammation and inhibit MOR-Gs coupling through interactions with FLNA and/or MOR.

In another aspect of the present invention, a contemplated compound inhibits or prevents the morphine-induced Gs protein coupling by MOR. That prevention of MOR-Gs coupling is believed to occur by preserving a particular interaction of filamin A and MOR. Downstream effects of preventing the MOR-Gs coupling include inhibition of cAMP accumulation and of cAMP Response Element Binding protein (CREB) activation in a manner resembling the activity of ultra-low-dose opioid antagonists naloxone and naltrexone.

In another aspect of the present invention, a FLNA-binding compound prevents or inhibits the MOR-Gs coupling while itself activating MOR.

The data collected in organotypic striatal slice cultures demonstrate that after 7 days of twice daily 1-hour exposures to oxycodone, MOR in striatum switch from Go to Gs coupling (compare vehicle to oxycodone conditions). In contrast, a compound contemplated herein did not cause a switch to Gs coupling despite its ability to stimulate MOR as previously assessed by GTPγS binding that is blocked by beta-funaltrexamine, a specific MOR antagonist. These data imply that these compounds provide the analgesic effects characteristic of opioid drugs but do not cause analgesic tolerance or dependence, and do not have the addictive potential of opioid drugs.

A compound contemplated by the present invention binds to an above-defined FLNA polypeptide as well as stimulates MOR. A contemplated compound corresponds in structure to Formula A 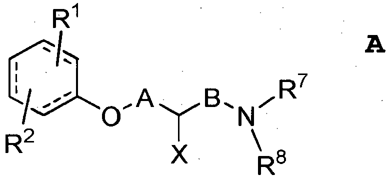 In a compound of Formula A, R¹ and R² are the same or different and are independently H, halogen, C₁-C₁₂ hydrocarbyl, C₁-C₆ acyl, C₁-C₆ hydrocarbyloxy, CF₃ and NR³R⁴, wherein R³ and R⁴ are the same or different and are H, C₁-C₄ hydrocarbyl, C₁-C₄ acyl, C₁-C₄ hydrocarbylsulfonyl, or R³ and R⁴ together with the depicted nitrogen form a 5-7-membered ring that optionally contains 1 or 2 additional hetero atoms that independently are nitrogen, oxygen or sulfur. **A** and **B** are the same or different and are CH₂, CDH or CD₂. X is OH or NR⁵R⁶, wherein R⁵ and R⁶ are the same or different and are H, C₁-C₄ hydrocarbyl, C₁-C₄ acyl, C₁-C₄ hydrocarbylsulfonyl, or R⁵ and R⁶ together with the depicted nitrogen form a 5-7-membered ring that optionally contains 1 or 2 additional hetero atoms that independently are nitrogen, oxygen or sulfur. R⁷ and R⁸ together with the depicted nitrogen (NR⁷R⁸) form a ring structure **W. W** contains 4 to 14 atoms in the ring structure including the depicted nitrogen, and preferably up to 12 atoms, and more preferably up to 8 atoms in the ring structure, wherein **W:** a) contains 1, 2 or 3 further hetero atoms that are independently oxygen, nitrogen or sulfur, or mixtures thereof, and b) contains one or more substituent groups bonded to one or more ring atoms, in which the one or more substituents contain a total of up to 8 atoms, and preferably up to 6 atoms, selected from the group consisting of carbon, nitrogen, oxygen and sulfur, and mixtures thereof. A dotted line (----) represents an optional double bond. In regard to a contemplated compound, R¹ and R² are other than methyl and isopropyl, respectively, when **W** is dimethyl-N-morpholinyl and the optional double bonds are absent. A pharmaceutically acceptable salt of a compound of Formula **A** is also contemplated.

One preferred compound of Formula **A** is a compound of Formula **I** 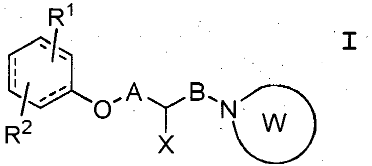 In a compound of Formula **I,** R¹ and R² are the same or different and are independently H, halogen, C₁-C₁₂ hydrocarbyl, C₁-C₆ acyl, C₁-C₆ hydrocarbyloxy, CF₃ and NR³R⁴, wherein R³ and R⁴ are the same or different and are H, C₁-C₄ hydrocarbyl, C₁-C₄ acyl, C₁-C₄ hydrocarbylsulfonyl, or R³ and R⁴ together with the depicted nitrogen form a 5-7-membered ring that optionally contains 1 or 2 additional hetero atoms that independently are nitrogen, oxygen or sulfur. **A** and **B** are the same or different and are CH₂, CDH or CD_{2.} X is OH or NR⁵R⁶, wherein R⁵ and R⁶ are the same or different and are H, C₁-C₄ hydrocarbyl, C₁-C₄ acyl, C₁-C₄ hydrocarbylsulfonyl, or R⁵ and R⁶ together with the depicted nitrogen form a 5-7-membered ring that optionally contains 1 or 2 additional hetero atoms that independently are nitrogen, oxygen or sulfur. **W** is a ring structure that contains up to 12 atoms in the ring structure, and preferably up to 8 atoms in the ring structure, including the depicted nitrogen. **W** contains: a) 1, 2 or 3 further hetero atoms that are independently oxygen, nitrogen or sulfur, and b) one or more substituent groups bonded to one or more ring atoms, in which the one or more substituents contain a total of up to 8 atoms selected from the group consisting of carbon, nitrogen, oxygen and sulfur, and mixtures thereof. A dotted line (----) represents an optional double bond. In regard to a contemplated compound, R¹ and R² are other than methyl and isopropyl, respectively, when **W** is dimethyl-N-morpholinyl and the optional double bonds are absent.

In preferred practice, when one optional double bond is present, three double bonds are present so that the compound is a derivative of benzene. Thus, preferably, unless three double bonds are present none of the double bonds is present and the compound has a saturated ring.

In some preferred embodiments when three double bonds are present, one of R¹ and R² is H so that the compound is a disubstituted benzene derivative. In other embodiments, both of R¹ and R² are halogen, although not necessarily the same halogen. Thus, a compound containing a fluoro and a bromo group is contemplated, as are a compound containing a chloro and a bromo group, a compound containing two fluoro groups, two chloro groups, two bromo groups and two iodo groups.

Where one or both of R¹ and R² is NR³R⁴, it is preferred that R³ and R⁴ are the same C₁-C₄ hydrocarbyl, and more preferably both of R³ and R⁴ are methyl (C₁). It is also preferred that only on e of R¹ and R² be NR³R⁴, and that the other be H.

Where one or both of R¹ and R² is C₁-C₄ acyl, it is preferred that only one of R¹ and R² is C₁-C₄ acyl and the other is H. A preferred C₁-C₄ acyl group is an acetyl group [CH₃C(O)-].

In another preferred embodiment, a contemplated compound corresponds in structure to Formula **Ia** 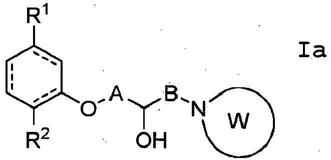 wherein
R¹ and R² are the same or different and are independently H, or C₁-C₆ hydrocarbyl;
**A** and **B** are the same or different and are CH₂, CDH or CD₂;
X is OH or X is OH or NR⁵R⁶, wherein R⁵ and R⁶ are the same or different and are H, C₁-C₄ hydrocarbyl, C₁-C₄ acyl, C₁-C₄ hydrocarbylsulfonyl, or R⁵ and R⁶ together with the depicted nitrogen form a 5-7-membered ring that optionally contains 1 or 2 additional hetero atoms that independently are nitrogen, oxygen or sulfur
**W** is a ring structure that contains 4 to 12, preferably up to 12 atoms in the ring structure, and more preferably up to 8 atoms in the ring structure, including the depicted nitrogen, and contains
   a) 1, 2 or 3 further hetero atoms that are independently oxygen, nitrogen or sulfur, and
   b) one or more substituent groups bonded to one or more ring atoms, in which the one or more substituent contain a total of up to 12 atoms selected from the group consisting of carbon, nitrogen, oxygen and sulfur, and mixtures thereof;
a dotted line (----) represents 1, 2, or 3 optional double bonds,
with the proviso that R¹ and R² are other than methyl and isopropyl, respectively, when **W** is dimethyl-N-morpholinyl ( 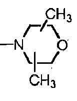 ) and the three optional double bonds are absent.

In one preferred embodiment, a compound of Formula **I** has the structure of Formula **II,** whereas in another preferred embodiment, a compound of Formula **I** has the structure of a compound of Formula **III.** R¹ and R², **A, B, X** and **W** are as previously defined in a compound of both Formulas **II** and **III.**

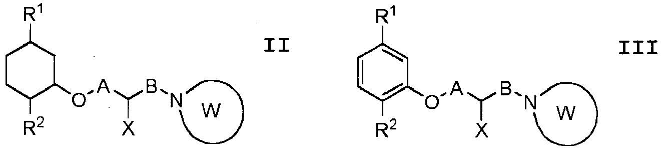

More preferably, the R¹ and R² of a compound of Formula **II** contain 3 to 5 carbon atoms, whereas for a compound of Formula **III,** R¹ is H and R² contains 3 to 5 carbon atoms.

Exemplary **W** substituents are illustrated below, wherein the wavy line indicates the position of the bond between W and the remainder of the compound. It is to be understood that the phrase "and mixtures thereof" is meant to encompass those substituents that contain two or more different hetero atoms as are seen below.

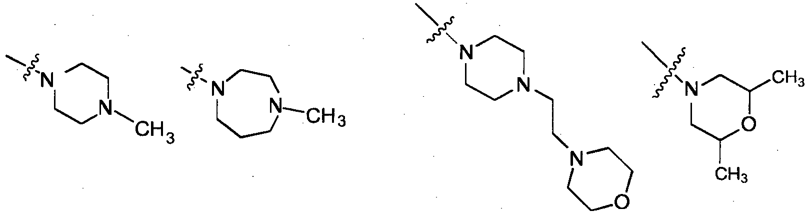

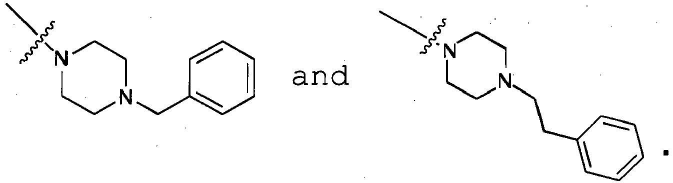

Use of a compound of Formula **II** selected from the group consisting of one or a mixture of the following in a contemplated method of reducing pain is particularly preferred

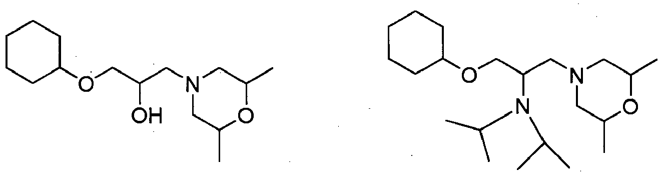

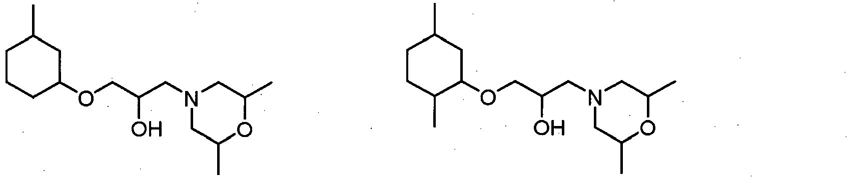

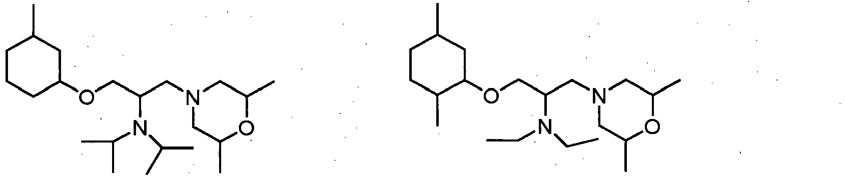

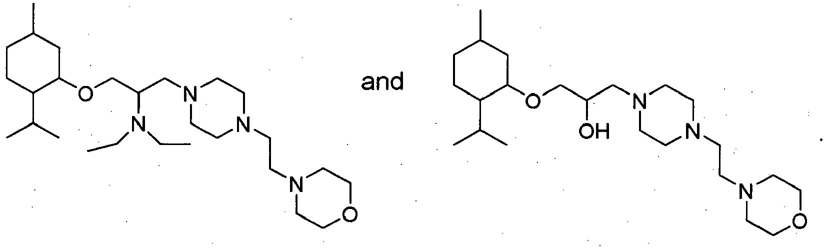

Of the above compounds, the following compounds of Formula **IIa** are themselves particularly preferred:

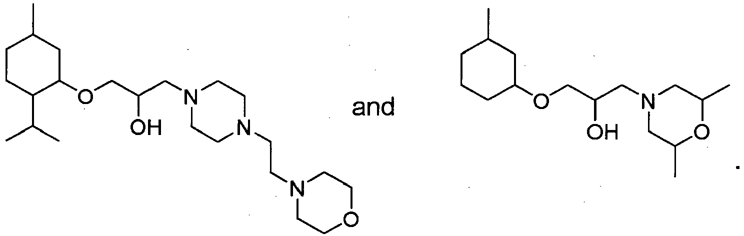

Of the above compounds, the following compound that is referred to in the assays described hereinafter as compound A0011 is more particularly preferred.

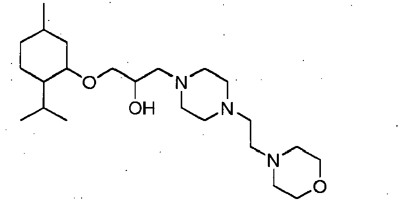

A particularly preferred compound of Formula IIIa is selected from the group consisting of 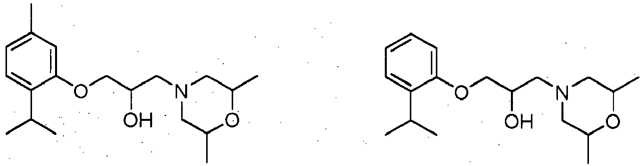 and

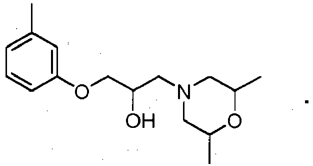

As can be seen from the above definitions, a contemplated compound can contain deuterated carbon atoms on either side of the "X" substituent. Deuterated compounds can be useful in studying the mechanism of drug interactions with living organisms for the elucidation of metabolic and biosynthetic pathways. Deuteration can also extend the half-life of a contemplated compound *in vivo* because a C-D bond is stronger than a C-H bond thereby requiring more energy input for bond cleavage. See, Blake et al., 1975 J. Pharm. Sci. 64(3):367-391; and Nelson et al., 2003 Drug Metab. Dispos. 31(12):1481-1498, and the citations therein.

A deuterated compound can be readily prepared using well-known chemistry. For example, epichlorohydrin that is generally used in the synthesis of a contemplated compound is commercially available in deuterated forms.

In another aspect, a contemplated compound is selected in part using a method for determining the ability of a candidate FLNA-binding compound, other than naloxone or naltrexone, to inhibit the interaction of the µ opioid receptor with filamin A (FLNA) and thereby prevent the µ opioid receptor from coupling to Gs proteins (Gs). That method comprises the steps of: (a) admixing the candidate FLNA-binding compound (alone if such FLNA-binding compound also stimulates MOR or with a MOR agonist otherwise) with mammalian cells that contain the µ opioid receptor and FLNA in their native conformations and relative orientations, the opioid agonist being present in an agonist effective amount and/or being administered in a repeated, chronic manner the FLNA-binding compound being present in an FLNA-binding effective amount; and (b) determining inhibition of the interaction of the µ oπτoτδ receptor with the G protein by analysis of the presence or the absence of the Gas subunit of Gs protein, wherein the absence of the Gas subunit indicates inhibition of the interaction of the µ oπτoτδ receptor with the Gs protein.

In one aspect, the analysis of Gs protein coupling by the µ opioid receptor and downstream effects elicited by admixing mammalian cells with a before-defined compound can be conducted by any one or more of several methods such as for example co-immunoprecipitation of Gα proteins with MOR, Western blot detection of MOR in immunoprecipitates, and densitometric quantification of Western blots.

### Pharmaceutical Composition

A compound of the invention can be provided for use by itself, or as a pharmaceutically acceptable salt. Although substituent groups can provide an acid functionality, a contemplated compound of any of Formulas **I-III** is an amine and can typically be used in the form of an acid addition salt derived from an inorganic or organic acid. Exemplary salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate and undecanoate.

Other compounds useful in this invention that contain acid functionalities can also form salts. Examples include salts with alkali metals or alkaline earth metals, such as sodium, potassium, calcium or magnesium or with organic bases or basic quaternary ammonium salts.

The reader is directed to Berge, 1977 J. Pharm. Sci. 68(1):1-19 for lists of commonly used pharmaceutically acceptable acids and bases that form pharmaceutically acceptable salts with pharmaceutical compounds.

In some cases, the salts can also be used as an aid in the isolation, purification or resolution of the compounds of this invention. In such uses, the acid used and the salt prepared need not be pharmaceutically acceptable.

A contemplated composition can be used in the manufacture of a medicament that is useful at least for lessening or reducing pain in a mammal that is in need, such as somatic, visceral, neuropathic or sympathetic pain, including musculoskeletal pain, inflammatory pain, burn pain, and pain from syndromes such as fibromyalgia and complex regional pain syndrome (CRPS). A contemplated composition can also be used in the manufacture of a medicament that is useful in reducing inflammation. Inasmuch as pain and inflammation are not always coincident, a contemplated composition is referred to as being used to reduce one or both of pain and inflammation, or a similar phrase.

A pharmaceutical composition is contemplated that contains an analgesia effective amount of a compound of Formula **I,** Formula **Ia,** Formula **II,** Formula **III,** Formula **IIa,** or Formula **IIIa** dissolved or dispersed in a physiologically tolerable carrier. Such a composition can be administered to mammalian cells *in vitro* as in a cell culture, or *in vivo* as in a living, host mammal in need.

A contemplated composition is typically administered a plurality of times over a period of days. More usually, a contemplated composition is administered a plurality of times in one day.

As is seen from the data that follow, a contemplated compound is active in the assays studies at micromolar amounts. In the laboratory mouse tail flick test, contemplated compound A0011 exhibited peak activity at about ten minutes using a dose of 56 mg/kg. Morphine administered at the same dose exhibited a slightly greater antinociceptive effect at twenty minutes. Further data are provided hereinafter. It is thus seen that the contemplated compounds are quite active and potent, and that a skilled worker can readily determine an appropriate dosage level, particularly in view of the relative activity of a contemplated compound compared to orally administered morphine.

A contemplated composition described herein can be used in the manufacture of a medicament that is useful at least for lessening or reducing pain in a mammal that is in need.

A contemplated pharmaceutical composition can be administered orally (perorally), parenterally, by inhalation spray in a formulation containing conventional nontoxic pharmaceutically acceptable carriers, adjuvants, and vehicles as desired. The term parenteral as used herein includes subcutaneous injections, intravenous, intramuscular, intrasternal injection, or infusion techniques. Formulation of drugs is discussed in, for example, Hoover, John E., Remington's Pharmaceutical Sciences, Mack Publishing Co., Easton, Pennsylvania; 1975 and Liberman, H.A. and Lachman, L., Eds., Pharmaceutical Dosage Forms, Marcel Decker, New York, N.Y., 1980.

Injectable preparations, for example, sterile injectable aqueous or oleaginous suspensions can be formulated according to the known art using suitable dispersing or wetting agents and suspending agents. The sterile injectable preparation can also be a sterile injectable solution or suspension in a nontoxic parenterally acceptable diluent or solvent, for example, as a solution in 1,3-butanediol. Among the acceptable vehicles and solvents that can be employed are water, Ringer's solution, and isotonic sodium chloride solution, phosphate-buffered saline. Liquid pharmaceutical compositions include, for example, solutions suitable for parenteral administration. Sterile water solutions of an active component or sterile solution of the active component in solvents comprising water, ethanol, or propylene glycol are examples of liquid compositions suitable for parenteral administration.

In addition, sterile, fixed oils are conventionally employed as a solvent or suspending medium. For this purpose any bland fixed oil can be employed including synthetic mono- or diglycerides. In addition, fatty acids such as oleic acid find use in the preparation of injectables. Dimethyl acetamide, surfactants including ionic and non-ionic detergents, polyethylene glycols can be used. Mixtures of solvents and wetting agents such as those discussed above are also useful.

Sterile solutions can be prepared by dissolving the active component in the desired solvent system, and then passing the resulting solution through a membrane filter to sterilize it or, alternatively, by dissolving the sterile compound in a previously sterilized solvent under sterile conditions.

Solid dosage forms for oral administration can include capsules, tablets, pills, powders, and granules. In such solid dosage forms, the compounds of this invention are ordinarily combined with one or more adjuvants appropriate to the indicated route of administration. If administered *per os,* the compounds can be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets can contain a controlled-release formulation as can be provided in a dispersion of active compound in hydroxypropylmethyl cellulose. In the case of capsules, tablets, and pills, the dosage forms can also comprise buffering agents such as sodium citrate, magnesium or calcium carbonate or bicarbonate. Tablets and pills can additionally be prepared with enteric coatings.

A mammal in need of treatment and to which a pharmaceutical composition containing a contemplated compound is administered can be a primate such as a human, an ape such as a chimpanzee or gorilla, a monkey such as a cynomolgus monkey or a macaque, a laboratory animal such as a rat, mouse or rabbit, a companion animal such as a dog, cat, horse, or a food animal such as a cow or steer, sheep, lamb, pig, goat, llama or the like.

Where *in vitro* mammalian cell contact is contemplated, a CNS tissue culture of cells from an illustrative mammal is often utilized, as is illustrated hereinafter. In addition, a non-CNS tissue preparation that contains opioid receptors such as guinea pig ileumcan also be used.

Preferably, the pharmaceutical composition is in unit dosage form. In such form, the composition is divided into unit doses containing appropriate quantities of the active urea. The unit dosage form can be a packaged preparation, the package containing discrete quantities of the preparation, for example, in vials or ampules.

### EXAMPLES

The present invention is described in the following examples which are set forth to aid in the understanding of the invention, and should not be construed to limit in any way the invention as defined in the claims which follow thereafter.

In the following examples, any compound falling outside the scope of the claims is for reference only.

The experiments described herein were carried out on organotypic striatal slices from male Sprague Dawley rats (200 to 250g) purchased from Taconic (Germantown, NY). Rats were housed two per cage and maintained on a regular 12-hour light/dark cycle in a climate-controlled room with food and water available *ad libitum* and sacrificed by rapid decapitation. All data are presented as mean ± standard error of the mean. Treatment effects were evaluated by two-way ANOVA followed by Newman-Keul's test for multiple comparisons. Two-tailed Student's t test was used for *post hoc* pairwise comparisons. The threshold for significance was p<0.05.

The following Table of Correspondence shows the structures of the compounds discussed herein and their identifying numbers.

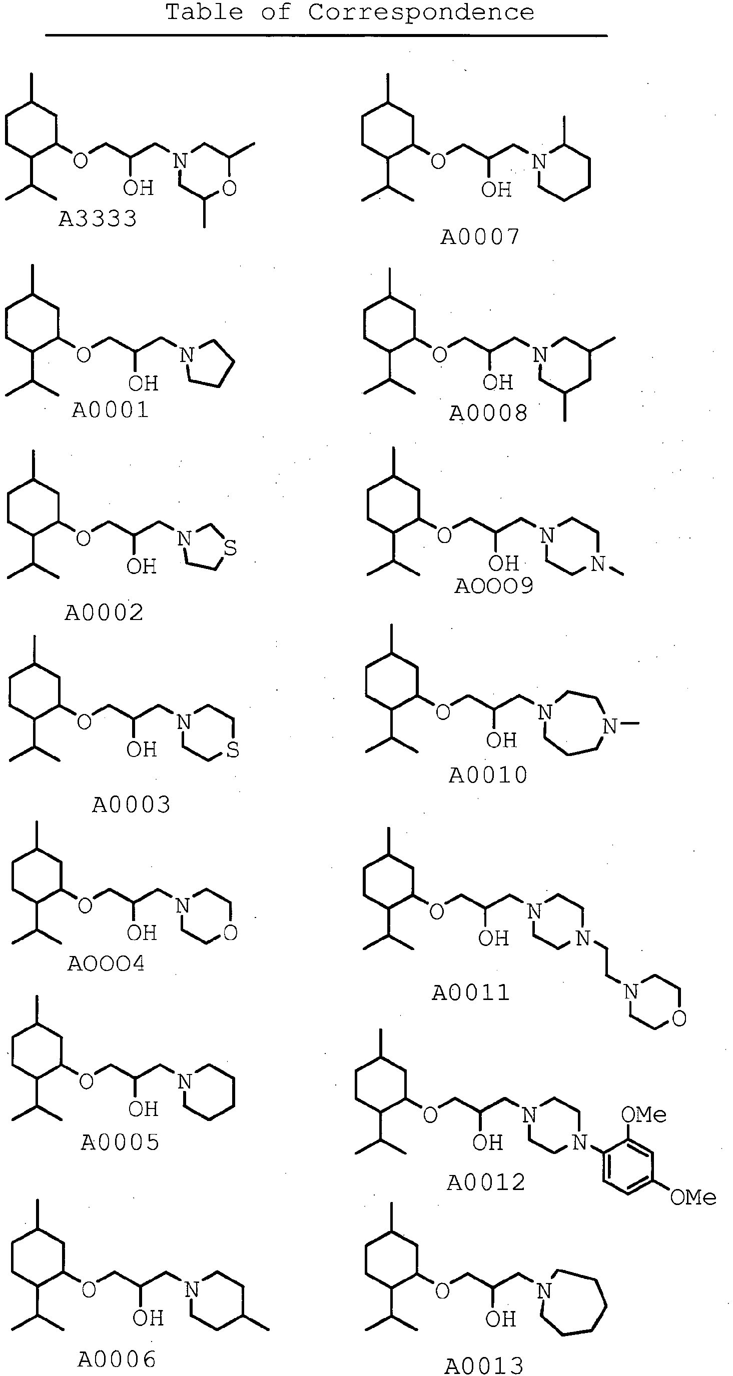

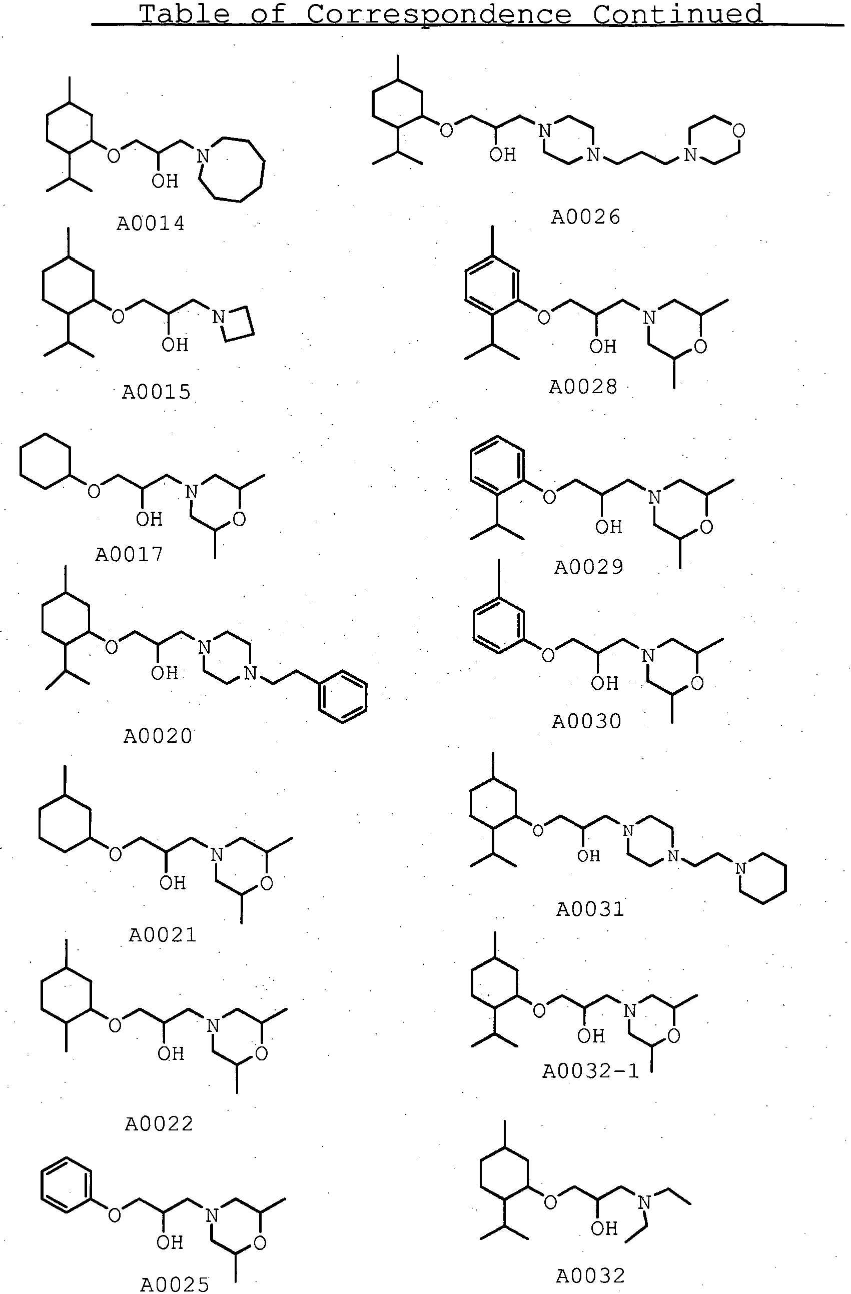

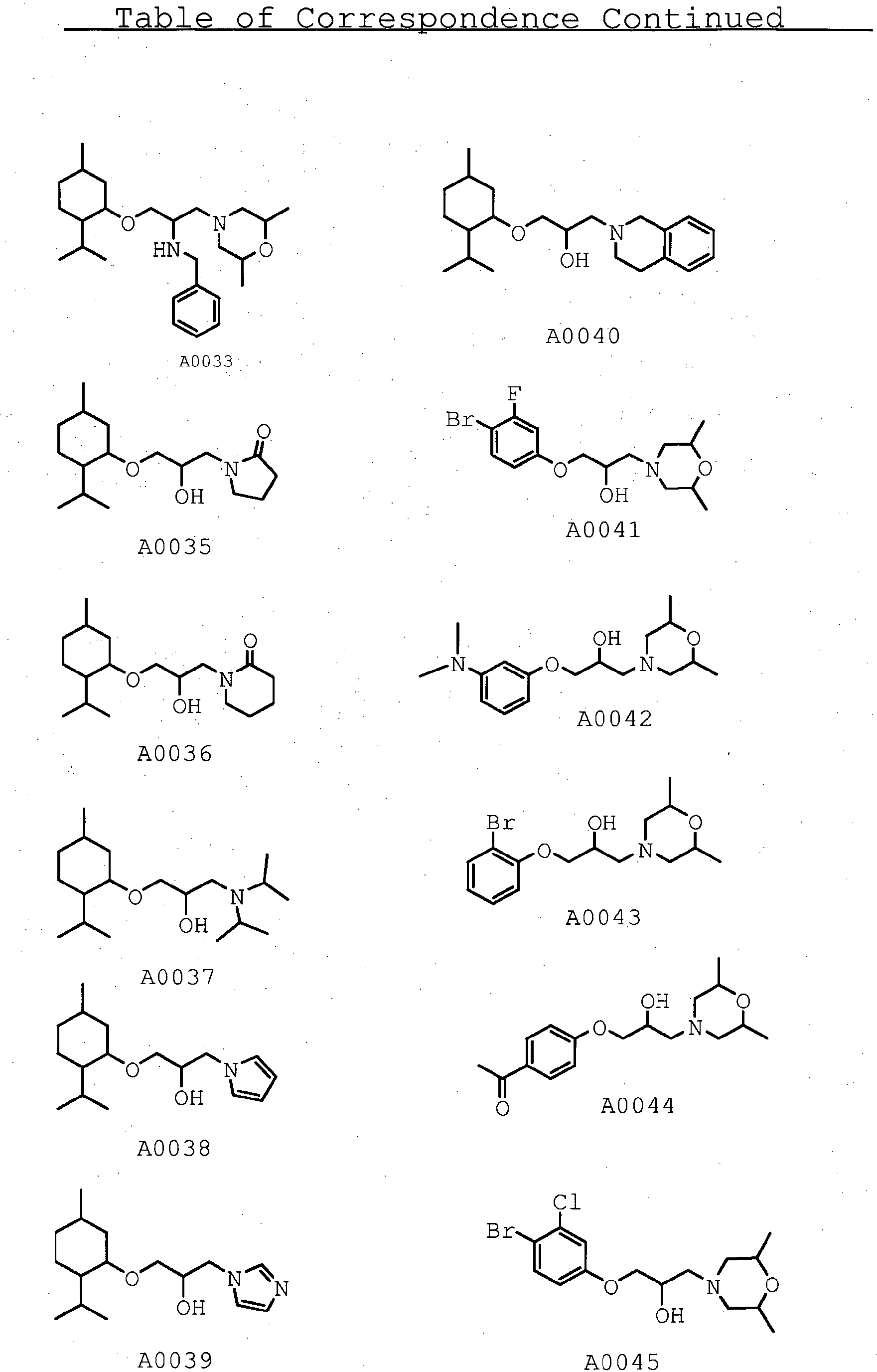

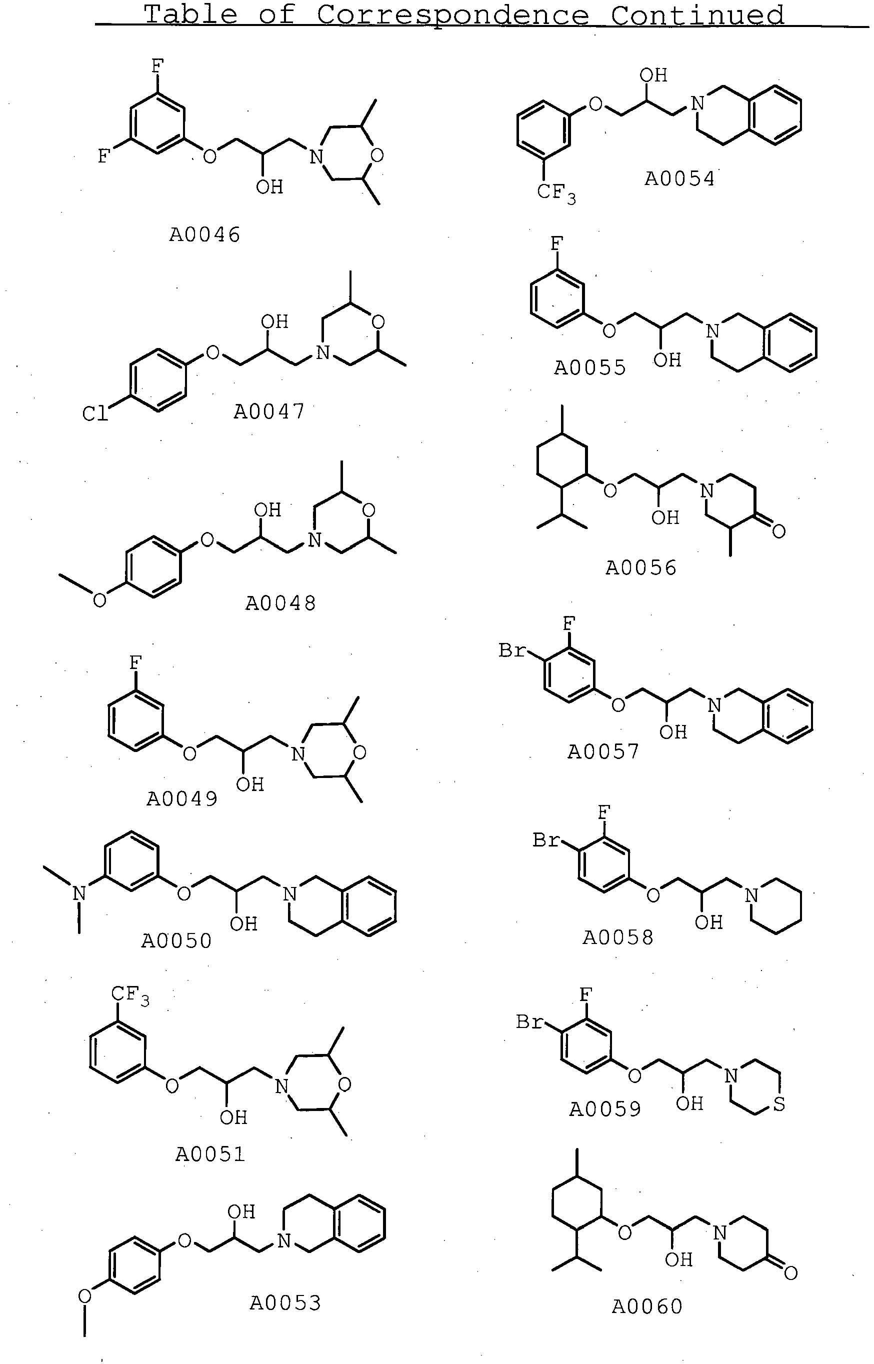

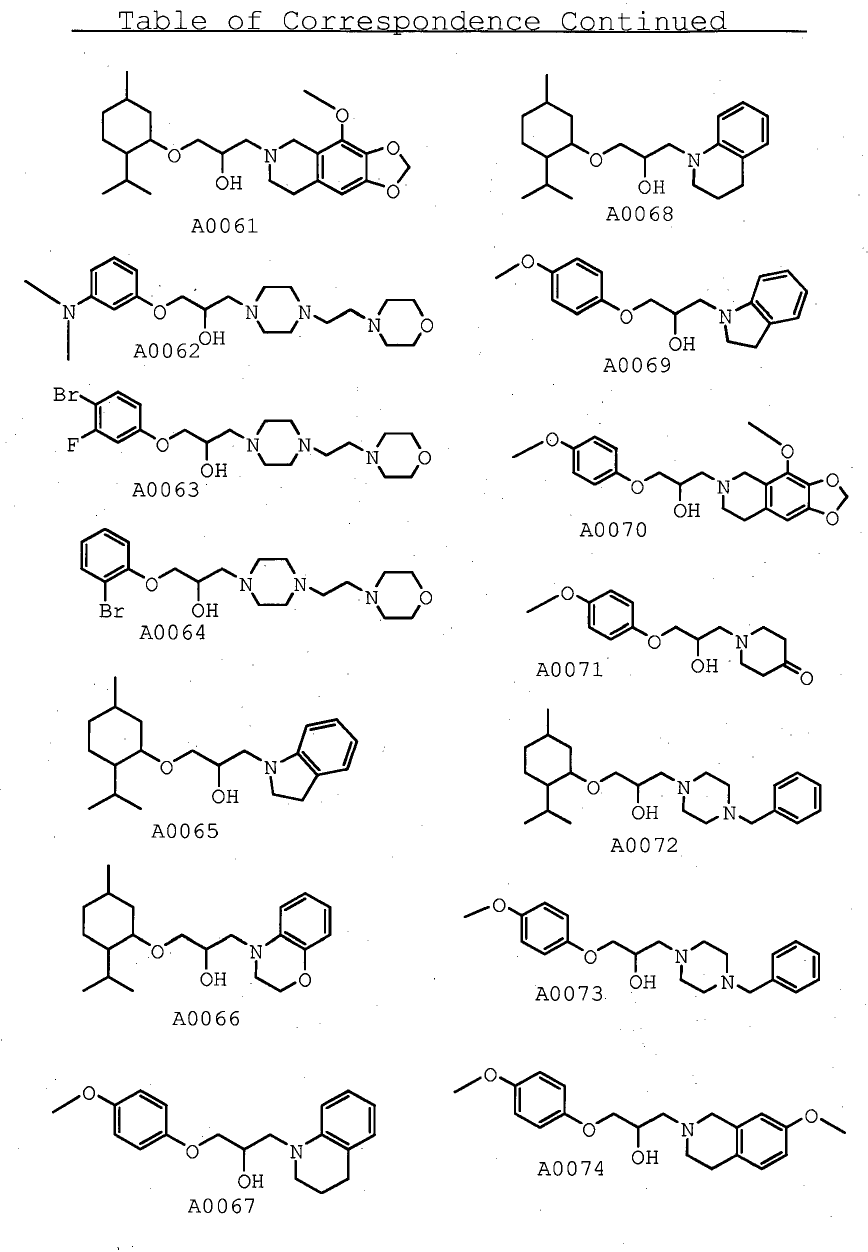

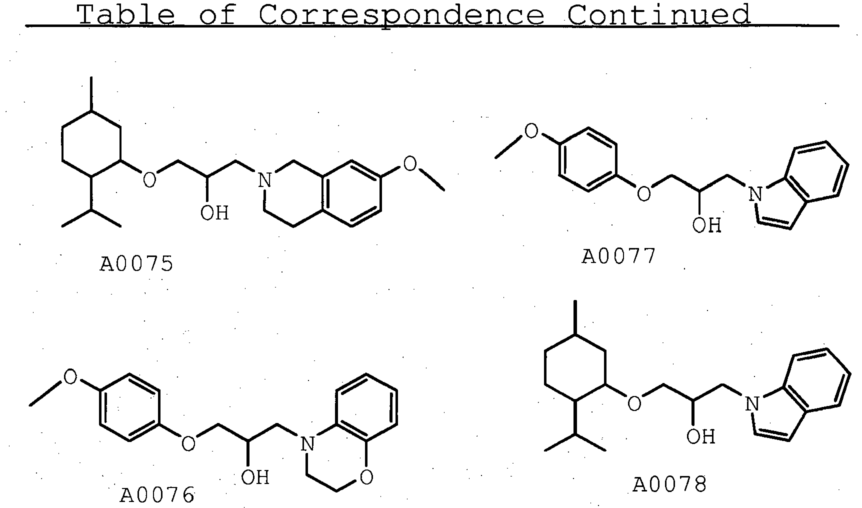

Without departing from the spirit and scope of this invention, one of ordinary skill can make various changes and modifications to the invention to adapt it to various usages and conditions. As such, these changes and modifications are properly, equitably, and intended to be, within the full range of equivalence of the following claims.

### EXAMPLE 1: MOR agonist activity

### using GTPγS binding assay

To assess the µ opiate receptor (MOR) agonist activity of positive compounds from the FLNA screening, compounds were tested in a [³⁵S]GTPγS binding assay using striatal membranes. Our previous study has shown that in striatal membranes, activation of MOR leads to an increase in [³⁵S]GTPγS binding to Gαo (Wang et al., 2005 Neuroscience 135:247-261).

Striatal tissue was homogenized in 10 volumes of ice cold 25 mM HEPES buffer, pH 7.4, which contained 1 mM EGTA, 100 mM sucrose, 50 µg/ml leupeptin, 0.04 mM PMSF, 2 µg/ml soybean trypsin inhibitor and 0.2% 2-mercaptoethanol. The homogenates were centrifuged at 800 X g for 5 minutes and the supernatants were centrifuged at 49,000 X g for 20 minutes. The resulting pellets were suspended in 10 volume of reaction buffer, which contained 25 mM HEPES, pH 7.5, 100 mM NaCl, 50 µg/ml leupeptin, 2 µg/ml soybean trypsin inhibitor, 0.04 mM PMSF and 0.02% 2-mercaptomethanol.

The resultant striatal membrane preparation (200 µg) was admixed and maintained (incubated) at 30°C for 5 minutes in reaction buffer as above that additionally contained 1 mM MgCl₂ and 0.5 nM [³⁵S]GTPγS (0.1 µCi/assay, PerkinElmer Life and Analytical Sciences) in a total volume of 250 µl and continued for 5 minutes in the absence or presence of 0.1 - 10 µM of an assayed compound of interest. The reaction was terminated by dilution with 750 µl of ice-cold reaction buffer that contained 20 mM MgCl₂ and 1 mM EGTA and immediate centrifugation at 16,000 X g for 5 minutes.

The resulting pellet was solubilized by sonicating for 10 seconds in 0.5 ml of immunoprecipitation buffer containing 0.5% digitonin, 0.2% sodium cholate and 0.5% NP-40. Normal rabbit serum (1 µl) was added to 1 ml of lysate and incubated at 25°C for 30 minutes. Nonspecific immune complexes were removed by incubation with 25 µl of protein A/G-conjugated agarose beads at 25°C for 30 minutes followed by centrifugation at 5,000 X g at 4°C for 5 minutes. The supernatant was divided and separately incubated at 25°C for 30 minutes with antibodies raised against Gαo proteins (1:1,000 dilutions).

The immunocomplexes so formed were collected by incubation at 25°C for 30 minutes with 40 µl of agarose-conjugated protein A/G beads and centrifugation at 5,000 X g at 4°C for 5 minutes. The pellet was washed and suspended in buffer containing 50 mM Tris-HCl, pH 8.0, and 1% NP-40. The radioactivity in the suspension was determined by liquid scintillation spectrometry. The specificity of MOR activation of [³⁵S]GTPγS binding to Gαo induced by a selective compound was defined by inclusion of 1 µM β-funaltrexamine (β-FNA; an alkylating derivative of naltrexone that is a selective MOR antagonist). DAMGO (H-Tyr-**D**-Ala-Gly-N-MePhe-Gly-OH; 1 or 10 µM) was used as a positive control.

The results of this study are shown in the Table below.

### FLNA-Binding Compound MOR Agonist Activity

| **FLNA- Binding Compound** | **Concentration of FLNA-Binding Compound as Agonist** | | | | | |
|---|---|---|---|---|---|---|
| | **0.1 µM** | **1 µM** | **1 µM +BFNA** | **%DAMGO (0.1 µM)** | **%DAMGO (1 µM)** | **%DAMGO + BFNA** |
| A3333 | 170.7% | 328.3% | 65.9% | 88.9% | 101.0% | 136.7% |
| A0001 | 94.3% | 181.7% | 22.2% | 63.1% | 78.9% | 83.8% |
| A0002 | 155. 6% | 4 % | 6.5% | 104.1% | 86.6% | 24.5% |
| A0003 | 176.8% | 276.0% | 17.1% | 118.3% | 119.9% | 64.5% |
| A0004 | 97.4% | 144.2% | 86.0% | 55.2% | 55.6% | 130.9% |
| A0005 | 179.7% | 239.2% | 23.5% | 105.0% | 89.6% | 45.1% |
| A0006 | 170.0% | 190.9% | 18.2% | 113.8% | 82.9% | 68.7% |
| A0007 | 102.0% | 221.9% | 40.4% | 68.3% | 96.4% | 152.5% |
| A0008 | 163.8% | 235.0% | 133.9% | 109.6% | 102.1% | 505.3% |
| A0009 | 70.2% | 126.4% | 93.9% | 39.8% | 48.7% | 142.9% |
| A0010 | 277.2% | 319.0% | 190.3% | 161.9% | 119.5% | 365.3% |
| A0011 | 236.3% | 287.5% | 47.0% | 158.2% | 124.9% | 177.4% |
| A0012 | 149.3% | 185.7% | 122.4% | 99.9% | 80.7% | 461.9% |
| A0013 | 102.1% | 164.8% | 86.1% | 57.8% | 63.6% | 131.1% |
| A0014 | 147.0% | 174.9% | 140.8% | 83.2% | 67.5% | 214.3% |
| A0015 | 110.9% | 150.1% | 62.5% | 64.8% | 56.2% | 120.0% |
| A0017 | 161.9% | 246.0% | 65.2% | 96.9% | 100.4% | 187.9% |
| A0020 | 168.6% | 217.4% | 67.4% | 100.9% | 88.7% | 194.2% |
| A0021 | 133.3% | 275.3% | 12.1% | 79.8% | 112.4% | 34.9% |
| A0022 | 154.1% | 216.0% | 28.0% | 90.0% | 80.9% | 53.7% |
| A0025 | 58.6% | 138.7% | 52.2% | 33.2% | 54.5% | 198.5% |
| A0026 | 140.7% | 179.8% | 120.8% | 79.7% | 70.7% | 459.3% |
| A0028 | 143.6% | 187.7% | 116.7% | 81.3% | 73.8% | 443.7% |
| A0029 | 173.8% | 206.5% | 22.3% | 98.4% | 81.2% | 84.8% |
| A0030 | 133.4% | 287.8% | 165.2% | 75.5% | 113.2% | 628.1% |
| A0031 | 178.2% | 297.0% | 150.9% | 100.9% | 116.8% | 573.8% |
| A0032-1 | 187.4% | 324.5% | 224.5% | 95.5% | 117.6% | 303.8% |
| A0032 | 226.9% | 257.8% | 133.0% | 115.6% | 93.4% | 180.0% |
| A0033 | 155.8% | 254.6% | 118.2% | 79.4% | 92.2% | 159.9% |
| A0035 | 120.6% | 158.8% | 88.6% | 61.5% | 57.5% | 119.9% |
| A0036 | 144.1% | 167.5% | 63.2% | 73.4% | 60.7% | 85.5% |
| A0037 | 177.9% | 236.2% | 104.6% | 90.7% | 85.6% | 141.5% |
| A0038 | 176.7% | 234.5% | 107.0% | 90.1% | 85.0% | 144.8% |
| A0039 | 267.8% | 339.6% | 173.5% | 136.5% | 123.0% | 234.8% |
| A0040 | 46.1% | 149.0% | 16.7% | 23.5% | 54.0% | 22.6% |
| A0041 | 212.7% | 283.6% | 50.6% | 108.4% | 102.8% | 68.5% |
| A0042 | 147.5% | 233.1% | 89.5% | 75.2% | 84.5% | 121.1% |
| A0043 | 183.3% | 223.8% | 89.1% | 93.4% | 81.1% | 120.6% |
| A0044 | 176.2% | 209.1% | 134.7% | 89.8% | 75.8% | 182.3% |
| A0045 | 143.9% | 274.2% | 99.2% | 73.3% | 99.3% | 134.2% |
| A0046 | 257.5% | 354.1% | 140.0% | 131.2% | 128.3% | 189.4% |
| A0047 | 233.0% | 255.0% | 116.5% | 118.8% | 92.4% | 157.6% |
| A0048 | 233.7% | 302.9% | 167.2% | 119.1% | 109.7% | 226.3% |
| A0049 | 232.3% | 370.3% | 107.1% | 118.4% | 134.2% | 144.9% |
| A0050 | 151.0% | 189.3% | 81.0% | 77.0% | 68.6% | 109.6% |
| A0051 | 290.4% | 386.6% | 211.6% | 148.0% | 140.1% | 286.3% |
| A0053 | 78.5% | 118.2% | 15.1% | 46.5% | 47.5% | 46.2% |
| A0054 | 74.9% | 159.2% | 114.1% | 44.4% | 63.9% | 348.9% |
| A0055 | 89.8% | 195.2% | 33.5% | 53.2% | 78.4% | 102.4% |
| A0056 | 115.6% | 129.6% | 17.4% | 74.1% | 56.2% | 43.6% |
| A0057 | 124.2% | 192.1% | 44.8% | 79.6% | 83.3% | 112.3% |
| A0058 | 70.7% | 244.3% | 59.9% | 45.3% | 106.0% | 150.1% |
| A0059 | 99.2% | 129.9% | 85.7% | 63.5% | 56.4% | 214.8% |
| A0060 | 99.7% | 158.2% | 14.3% | 63.9% | 68.6% | 35.8% |
| A0061 | 110.3% | 197.1% | 10.7% | 70.7% | 85.5% | 26.8% |
| A0062 | -- | -- | -- | -- | -- | -- |
| A0063 | 122.8% | 245.8% | 310% | 78.7% | 106.6% | 77.7% |
| A0064 | 219.2% | 262.9% | 43.7% | 127.4% | 119.7% | 126.7% |
| A0065 | 197.6% | 266.8% | 44.9% | 126.6% | 115.7% | 112.5% |
| A0066 | 151.9% | 195.6% | 59.2% | 88.3% | 89.0% | 171.6% |
| A0067 | 170.8% | 254.4% | 33.9% | 99.2% | 115.8% | 98.3% |
| A0068 | 73.9% | 110.4% | 98.1% | 36.8% | 35.2% | 182.0% |
| A0069 | 122.7% | 244.2% | 29.5% | 71.3% | 111.2% | 85.5% |
| A0070 | 128.6% | 195.3% | 80.3% | 74.7% | 88.9% | 232.8% |
| A0071 | 225.7% | 310.9% | 239.4% | 128.2% | 122.9% | 1088.2% |
| A0072 | 254.3% | 305.1% | 171.8% | 126.8% | 97.2% | 318.7% |
| A0073 | 201.7% | 325.7% | 185.8% | 100.5% | 103.7% | 344.7% |
| A0074 | -- | -- | -- | -- | -- | -- |
| A0075 | -- | -- | -- | -- | -- | -- |
| A0076 | 79.8% | 172.6% | 41.2% | 46.4% | 78.6% | 119.4% |
| A0077 | 300.1% | 334.7% | 103.5% | 170.5% | 132.3% | 470.5% |
| A0078 | 250.5% | 289.9% | 147.8% | 124.9% | 92.3% | 274.2% |

### EXAMPLE 2: FITC-NLX-based FLNA Screening Assay

### A. Streptavidin-coated 96-well plates

Streptavidin-coated 96-well plates (Reacti-Bind™ NeutrAvidin™ High binding capacity coated 96-well plate, Pierce-ENDOGEN) are washed three times with 200 µl of 50 mM Tris HCl, pH 7.4 according to the manufacturer's recommendation.

### B. N-biotinylated VAKGL pentapeptide (Bn-VAKGL) (SEQ ID NO: 1)

Bn-VAKGL peptide (0.5 mg/plate) is dissolved in 50 µl DMSO and then added to 4450 µl of 50 mM Tris HCl, pH 7.4, containing 100 mM NaCl and protease inhibitors (binding medium) as well as 500 µl superblock in PBS (Pierce-ENDOGEN) [final concentration for DMSO: 1%].

### C. Coupling of Bn-VAKGL peptides to

### streptavidin-coated plate

The washed streptavidin-coated plates are contacted with 5 µg/well of Bn-VAKGL (100 µl) for 1 hour (incubated) with constant shaking at 25°C [50 µl of Bn-VAKGL peptide solution from B + 50 µl binding medium, final concentration for DMSO: 0.5%]. At the end of the incubation, the plate is washed three times with 200 µl of ice-cold 50 mM Tris HCl, pH 7.4.

### D. Binding of FITC-tagged

### naloxone [FITC-NLX] to VAKGL

Bn-VAKGL coated streptavidin plates are incubated with 10 nM fluorescein isothiocyanate-labeled naloxone (FITC-NLX; Invitrogen) in binding medium (50 mM Tris HCl, pH 7.4 containing 100 mM NaCl and protease inhibitors) for 30 minutes at 30°C with constant shaking. The final assay volume is 100 µl. At the end of incubation, the plate is washed twice with 100 µl of ice-cold 50 mM Tris, pH 7.4. The signal, bound-FITC-NLX is detected using a DTX-880 multi-mode plate reader (Beckman).

### E. Screening of Medicinal Chemistry Analogs

The compounds are first individually dissolved in 25% DMSO containing 50 mM Tris HCl, pH 7.4, to a final concentration of 1 mM (assisted by sonication when necessary) and then plated into 96-well compound plates. To screen the medicinal chemistry analogs (new compounds), each compound solution (1 µl) is added to the Bn-VAKGL coated streptavidin plate with 50 µl/well of binding medium followed immediately with addition of 50 µl of FITC-NLX (total assay volume/well is 100 µl). The final screening concentration for each compound is 10 µM.

Each screening plate includes vehicle control (total binding) as well as naloxone (NLX) and/or naltrexone (NTX) as positive controls. Compounds are tested in triplicate or quadruplicate. Percent inhibition of FITC-NLX binding for each compound is calculated [(Total FITC-NLX bound in vehicle - FITC-NLX bound in compound)/Total FITC-NLX bound in vehicle] x 100%]. To assess the efficacies and potencies of the selected compounds, compounds that achieve approximately 60-70% inhibition at 10 µM are screened further at 1 and 0.1 µM concentrations.

The results of this screening assay are shown in the table below.

### FLNA Peptide Binding Assay

| **FLNA-binding Compound** | **Concentration of FLNA-binding Compound** | | |
|---|---|---|---|
| | **0.01 µM** | **0.1 µM** | **1 µM** |
| 3333 | 40.4% | 48.5% | 54.2% |
| A0001 | 39.7% | 45.6% | 52.4% |
| A0002 | 38.7% | 43.7% | 49.9% |
| A0003 | 21.3% | 31.6% | 37.4% |
| A0004 | 40.0% | 43.7% | 47.6% |
| A0005 | 34.2% | 38.2% | 43.8% |
| A0006 | 37.9% | 43.5% | 47.5% |
| A0007 | 39.2% | 46.2% | 52.9% |
| A0008 | 34.5% | 33.5% | 39.8% |
| A0009 | 26.4% | 37.8% | 38.9% |
| A0010 | 36.0% | 36.5% | 39.0% |
| A0011 | 45.7% | 51.1% | 52.8% |
| A0012 | 39.7% | 49.6% | 54.4% |
| A0013 | 30.2% | 40.2% | 47.7% |
| A0014 | 33.8% | 39.7% | 44.7% |
| A0015 | 36.3% | 46.8% | 55.0% |
| A0017 | 29.8% | 38.6% | 44.0% |
| A0020 | 37.8% | 38.8% | 45.8% |
| A0021 | 36.8% | 43.4% | 49.5% |
| A0022 | 41.9% | 49.7% | 56.8% |
| A0025 | 39.0% | 49.8% | 53.2% |
| A0026 | 36.4% | 42.4% | 49.2% |
| A0028 | 39.5% | 43.8% | 50.5% |
| A0029 | 44.4% | 44.4% | 50.8% |
| A0030 | 35.6% | 44.4% | 48.9% |
| A0031 | 40.8% | 47.6% | 52.9% |
| A0032-1 | 35.6% | 43.9% | 50.0% |
| A0032 | 43.0% | 50.3% | 54.5% |
| A0033 | 46.4% | 51.8% | 56.5% |
| A0035 | 40.3% | 45.5% | 54.9% |
| A0036 | 45.6% | 50.1% | 54.4% |
| A0037 | 49.3% | 51.3% | 56.8% |
| A0038 | 46.4% | 52.3% | 56.6% |
| A0039 | 49.0% | 53.5% | 60.3% |
| A0040 | 45.0% | 50.4% | 56.3% |
| A0041 | 45.8% | 51.7% | 56.9% |
| A0042 | 47.2% | 48.3% | 55.8% |
| A0043 | 46.4% | 48.9% | 51.8% |
| A0044 | 32.4% | 36.9% | 39.6% |
| A0045 | 28.1% | 35.0% | 37.8% |
| A0046 | 34.3% | 38.4% | 40.9% |
| A0047 | 40.9% | 42.9% | 44.5% |
| A0048 | 38.5% | 44.0% | 46.9% |
| A0049 | 46.2% | 49.4% | 49.3% |
| A0050 | 42.9% | 49.8% | 52.1% |
| A0051 | 45.9% | 45.4% | 52.1% |
| A0053 | 34.8% | 40.0% | 46.9% |
| A0054 | 28.7% | 35.8% | 41.4% |
| A0055 | 28.1% | 32.4% | 41.8% |
| A0056 | 34.4% | 40.9% | 41.3% |
| A0057 | 29.1% | 37.0% | 43.4% |
| A0058 | 28.9% | 36.6% | 42.1% |
| A0059 | 27.4% | 36.6% | 38.7% |
| A0060 | 32.4% | 39.0% | 42.0% |
| A0061 | 27.5% | 38.9% | 42.8% |
| A0062 | -- | -- | -- |
| A0063 | 21.2% | 31.0% | 38.8% |
| A0064 | 41.8% | 46.2% | 53.6% |
| A0065 | 38.7% | 50.0% | 50.8% |
| A0066 | 36.7% | 45.4% | 53.7% |
| A0067 | 32.7% | 39.1% | 44.3% |
| A0068 | 51.9% | 54.2% | 58.3% |
| A0069 | 32.0% | 40.4% | 46.1% |
| A0070 | 32.9% | 39.1% | 41.7% |
| A0071 | 44.7% | 46.8% | 53.9% |
| A0072 | 45.5% | 52.2% | 59.4% |
| A0073 | 47.3% | 54.8% | 59.7% |
| A0074 | -- | -- | -- |
| A0075 | -- | -- | -- |
| A0076 | 36.1% | 40.0% | 44.9% |
| A0077 | 41.1% | 48.7% | 49.4% |
| A0078 | 50.1% | 55.8% | 57.6% |

### EXAMPLE 3: Tail-flick test

The mouse "tail flick" test was used to assay the relative antinociceptive activity of compositions containing a compound to be assayed. This assay was substantially that disclosed by Xie et al., 2005 J. Neurosci 25:409-416.

The mouse hot-water tail-flick test was performed by placing the distal third of the tail in a water bath maintained at 52°C. The latency until tail withdrawal from the bath was determined and compared among the treatments. A 10 second cutoff was used to avoid tissue damage. Data are converted to percentage of antinociception by the following formula: (response latency - baseline latency)/(cutoff - baseline latency) x 100 to generate dose-response curves. Linear regression analysis of the log dose-response curves was used to calculate the A₅₀ (dose that resulted in a 50% antinociceptive effect) doses and the 95% confidence intervals (CIs). Relative potency was determined as a ratio of the A₅₀ values. The significance of the relative potency and the confidence intervals are determined by applying the t test at p < 0.05.

To assess tolerance to the antinociceptive effect, the compound was administered twice daily for 7 days at an A₉₀ dose (dose that results in a 90% antinociceptive effect in the 52°C warm-water tail-flick test), and the tail-flick test was performed daily after the a.m. dose. A significant reduction in tail-flick latency on subsequent days compared to the Day 1 administration of the A₉₀ dose indicates antinociceptive tolerance.

Orally administered morphine exhibited an A₅₀ value of 61.8 (52.4-72.9) mg/kg, and a mean maximum antinociception amount of about 43% at 56 mg/kg at about 20 minutes. Orally administered compound A0011 exhibited a mean maximum antinociception amount of about 38% at 56 mg/kg at about 10 minutes, whereas compounds A0021, A0049, and A0065 exhibited about 25%, about 45%, and about 25%, respectively, after oral dosing at 56 mg/kg. Compounds A0041 and A0064 exhibited about 12% and about 50%, respectively, after 32 mg/kg dosings after about 10-15 minutes and about 30-50 minutes, respectively.

### EXAMPLE 4: Dependence test

On day 8, 16-20 hours after the last administration of an assay composition, animals were given naloxone to precipitate withdrawal (10 mg/kg, s.c.) before being placed in an observation chamber for 1 hour. A scale adapted from MacRae et al., 1997 Psychobiology 25:77-82 was used to quantify four categories of withdrawal behaviors: "wet dog" shakes, paw tremors, mouth movements, and ear wipes. Scores are summed to yield a total withdrawal score across the 1-hour test.

### EXAMPLE 5: Relative Gs/Go Switching

In this set of studies, the rat brain slice organotypic culture methods were modified from those published previously (Adamchik et al., 2000 Brain Res Protoc 5:153-158; Stoppini et al., 1991 J Neurosci Methods 37:173-182). Striatal slices (200 µM thickness) were prepared using a McIlwain tissue chopper (Mickle Laboratory Engineering Co., Surrey, UK). Slices were carefully transferred to sterile, porous culture inserts (0.4 µm, Millicell-CM) using the rear end of a glass Pasteur pipette. Each culture insert unit contained 2 slices and was placed into one well of the 12-well culture tray. Each well contain 1.5 ml of culture medium composed of 50% MEM with Earl's salts, 2 mM L-glutamine, 25% Earl's balanced salt solution, 6.5 g/l D-glucose, 20% fetal bovine serum, 5% horse serum, 25 mM HEPES buffer, 50 mg/ml streptomycin and 50 mg/ml penicillin. The pH value was adjusted to 7.2 with HEPES buffer.

Cultures were first incubated for 2 days to minimize the impact of injury from slice preparation. Incubator settings throughout the experiment were 36°C with 5% CO₂. To induce tolerance, culture medium was removed and the culture insert containing the slices was gently rinsed twice with warm (37°C) phosphate-buffered saline (pH 7.2) before incubation in 0.1% fetal bovine serum-containing culture medium with 100 µM morphine for 1 hour twice daily (at 9-10 AM and 3-4 PM) for 7 days.

Slices were returned to culture medium with normal serum after each drug exposure. Tissues were harvested 16 hours after the last drug exposure by centrifugation.

For determination of MOR - G protein coupling, slices were homogenized to generate synaptic membranes. Synaptic membranes (400 µg) were incubated with either 10 µM oxycodone or Kreb's-Ringer solution for 10 minutes before solubilization in 250 µl of immunoprecipitation buffer (25 mM HEPES, pH 7.5; 200 mM NaCl, 1 mM EDTA, 50 µg/ml leupeptin, 10 µg/ml aprotinin, 2 µg/ml soybean trypsin inhibitor, 0.04 mM PMSF and mixture of protein phosphatase inhibitors). Following centrifugation, striatal membrane lysates were immunoprecipitated with immobilized anti-Gαs/olf or -Gαo conjugated with immobilized protein G-agarose beads. The level of MOR in anti-Gαs/olf or -Gαo immunoprecipitates was determined by Western blotting using specific anti-MOR antibodies.

To measure the magnitude of MOR-mediated inhibition of cAMP production, brain slices were incubated with Kreb's-Ringer (basal), 1 µM DAMGO, 1 µM forskolin or 1 µM DAMGO + 1 µM forskolin for 10 minutes at 37°C in the presence of 100 µM of the phosphodiesterase inhibitor IBMX. Tissues were homogenized by sonication and protein precipitated with 1M TCA. The supernatant obtained after centrifugation was neutralized using 50 mM Tris, pH 9.0. The level of cAMP in the brain lysate was measured by a cAMP assay kit (PerkinElmer Life Science, Boston) according to manufacturer's instructions.

| Condition | Gs/olf | Go | Gs/Go-Coupled Ratio |
|---|---|---|---|
| **Vehicle** | | | |
| Average | 330.7 | 1996.4 | 0.173 |
| SEM | 34.6 | 192.0 | 0.34 |
| **Oxycodone,** 10 µM | | | |
| Average | 1425.2 | 900.4 | 1.588 |
| SEM | 77.8 | 26.2 | 0.103 |
| **3333,** 10 µM | | | |
| Average | 1079.0 | 1426.3 | 0.761 |
| SEM | 66.2 | 46.5 | 0.070 |
| **3333,** 100 µM | | | |
| Average | 1064.8 | 1418.8 | 0.756 |
| SEM | 94.2 | 82.9 | 0.077 |

### EXAMPLE 6: Carrageenan-Induced

### Acute Inflammatory Pain

To test the antinociceptive activity of the compounds under acute inflammatory conditions, the latency to paw withdrawal from a noxious thermal stimulus is determined before and 3 hours after injection of a 50 µl solution of 2% carrageenan into the plantar surface of the hindpaw (Mogil et al. 1999 Pain 80:67-82). Animals are placed in plexiglas boxes on top of a glass plate maintained at 30°C and allowed to habituate for two sessions (-24 hours and -1 hour). Each habituation session lasts approximately 45-60 minutes.

For baseline paw withdrawal latencies, an infrared heat source (Ugo Basile model 37370) is applied from under the glass plate onto the plantar surface of the right hind paw with the focus of the light beam no larger than a 3- to 5-mm diameter. The time to withdrawal of the hind paw from the heat source is recorded. A maximum cutoff of 30 seconds is used to prevent tissue damage. The intensity of the beam is set so that baseline latencies are approximately 15 seconds. The post-carrageenan baseline is reestablished 3 hours after the carrageenan injections and only animals with a significant decrease in the latency of hind paw withdrawal from the thermal stimulus (thermal hypersensitivity) are tested. Animals are administered compounds, and hind paw withdrawal latencies are tested at various intervals after injection until the drug response falls below ∼20% MPE.

Antihyperalgesia (thermal hypersensitivity) and antinociception are calculated as follows: percentage activity = 100 [(test paw withdrawal latency - post-carrageenan baseline paw withdrawal latency)/(pre-carrageenan baseline paw withdrawal latency - post-carrageenan baseline paw withdrawal latency)].

Paw edema is determined by use of a plethysmometer (Ugo Basile) in the mice undergoing the thermal latency testing. Paw volumes for the left and right hind paw are measured at the conclusion of the thermal latency testing (120 minutes after drug administration).

### Compound Syntheses

A compound useful herein can be readily synthesized. An illustrative synthetic scheme is shown below that preparation of compounds containing two sulfonyl linkages and one sulfonyl and one carbonyl linkage. That scheme can be readily adapted for the preparation of compounds containing two carbonyl linkages and one carbonyl and one sulfonyl linkage in the opposite configurations from those shown. More detailed syntheses are set out hereinafter.

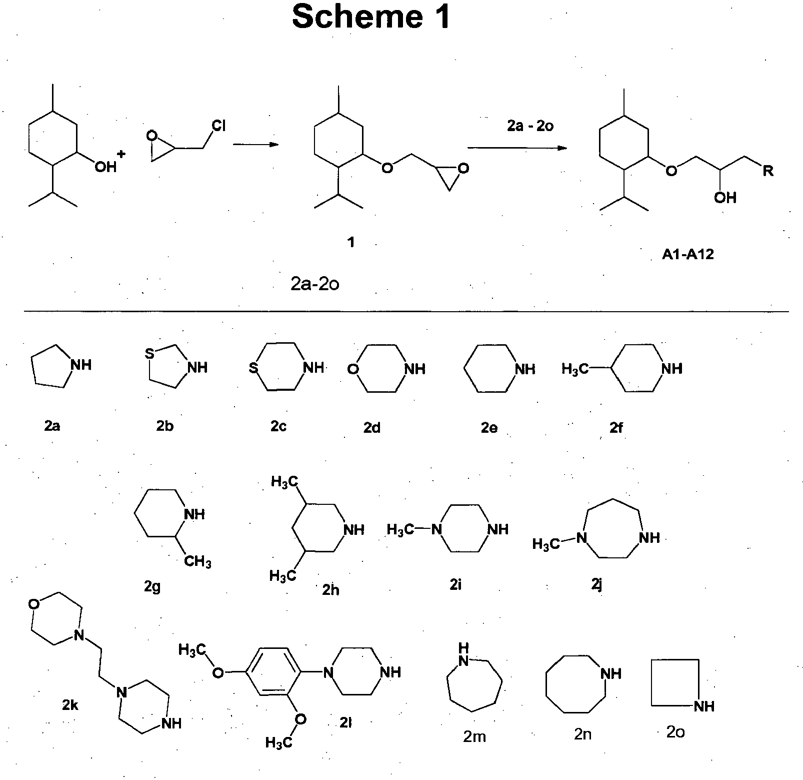

### Preparation of compound 1

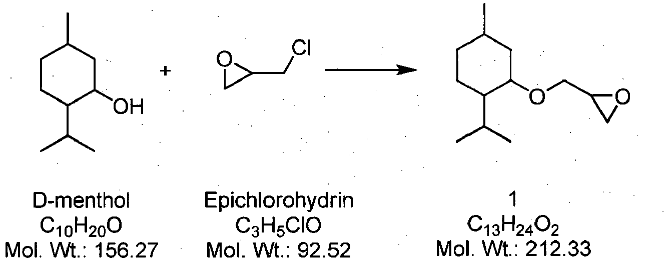

A flask was charged with D-menthol (10 g, 64 mmol), 40 mL toluene, and AlCl₃ (0.68 g, 5.12 mmol). The temperature of the mixture was raised to 160°C. Then, epichlorohydrin (5.9 g, 64 mmol) was added with stirring for 1 hour. Next, NaOH (50%) (10.24 g, 128 mmol) was added with stirring at a temperature of 75°C overnight (about 18 hours). Following this treatment, 5 mL of water was added into the mixture. Next, the mixture was extracted with ethyl acetate three times (15 mL total of ethyl acetate) and the extracted organic phase was combined, dried, and concentrated to obtain the crude product. The crude product was purified by silica gel column to obtain the purified product, a colorless oil (TLC confirmed, 12.8 g, yield:94%).

¹H NMR (400MHz, CDCl₃) δ: 0.793~1.019 (m, 13H), 1.237~1.402 (m, 3H), 1.602~1.684 (m, 1H), 2.054~2.124 (m, 1H), 2.227~2.275 (m, 1H), 2.605~2.644 (m, 1H), 2.801~2.839 (m, 1H), 3.098~3.185 (m, 2H), 3.367~3.409 (m, 0.5H), 3.583~3.603 (m, 1H), 3.801~3.837 (m, 0.5H).

### Preparation of compound 3333

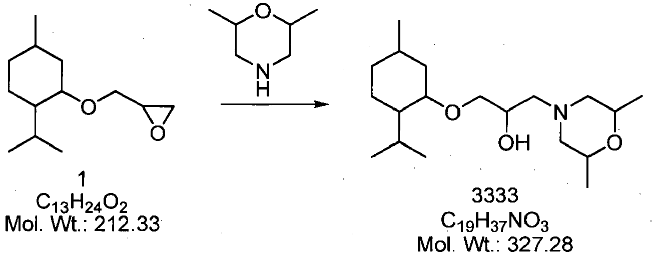

A mixture of compound **1** (490 mg, 2.311 mmol), 2,6-dimethylmorpholine (532 mg, 4.632 mmol) and H₂O (0.4 ml) was stirred overnight (about 18 hours) at room temperature. The resulting mixture was extracted with ethyl acetate, washed with brine, dried with anhydrous sodium sulfate, and concentrated under vacuum to afford 701 mg of crude product as a yellow liquid. The crude product was purified via column chromatography to afford 290 mg of the desired product (yield: 38.4 %) and 111.9 mg of an unidentified isomer (yield: 14.7%).

### Preparation of compound A0001

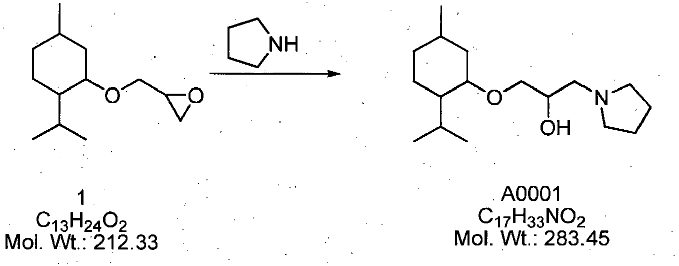

A mixture of compound **1** (200 mg, 0.94 mmol) and pyrrolidine (66.9 mg, 0.94 mmol) in 0.1 mL of water was stirred overnight (about 18 hours) at room temperature. Then 3 mL of H₂O and 5 mL of ethyl acetate was added into the mixture, the organic phase was separated and dried and concentrated to obtain an oily substance. Then, the crude product was purified by silica gel column (methylene chloride : methanol = 30:1) to give 126 mg of title product (¹H NMR and MS confirmed, yield 47%).

¹H NMR (400 MHz, CDCl₃): 3.844-3.833 (m, 1H); 3.667∼3.589 (m, 1H); 3.322 ~3.270 (m, 1H); 3.09~3.03 (td, J = 10.8, 4 Hz, 1H); 2.695~2.655 (m, 2H); 2.542~2.511 (m, 2H); 2.466~2.425 (m, 1H); 2.224~2.107 (m, 2H); 1.795~1.602 (m, 6H); 1.381~1.234 (m, 3H); 0.977~0.762 (m, 12H). MS (ESI) calcd for C₁₇H₃₃NO₂ (m/z): 283.25. found: 284.4 [M+1]⁺

### Preparation of compound A0002

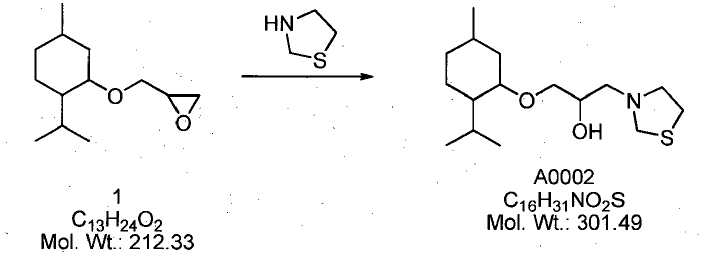

A mixture of compound **1** (200 mg, 0.94 mmol) and thiazolidine (83.81 mg, 0.94 mmol) in 0.1 mL of water was stirred overnight (about 18 hours) at room temperature. Then, 3 mL of H₂O and 5 mL of ethyl acetate was added into the mixture. The organic phase was separated, dried, and concentrated to obtain an oily substance. The crude product was purified by silica gel column (methylene chloride: methanol =30:1) to give 126 mg of title product (¹H NMR and MS confirmed, yield 41%).

¹H NMR (400 MHz, CDCl₃): 4.091 (s, 2H); 3.876 (br, 1H); 3.704~3.581 (m, 1H); 3.371 ~3.286 (m, 1H); 3.184∼3.059 (m, 3 H); 2.952~2.843 (m, 3 H); 2.578~2.539 (m, 1 H); 2.428~2.371 (m, 1H); 2.173~2.072 (m, 2H); 1.664~1.602 (m, 2H); 1.423~1.186 (m, 3H); 0.983~0.765 (m, 12H). MS (ESI) calcd for C₁₆H₃₁NO₂S (m/z): 301.21. found: 302.3 [M+1]⁺

### Preparation of compound A0003

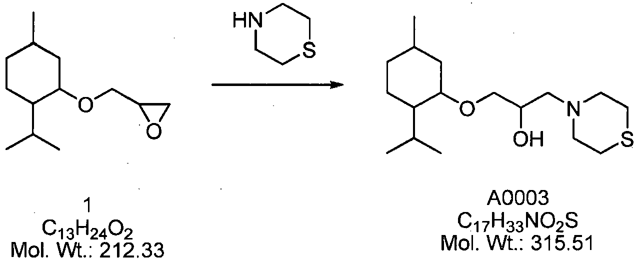

A mixture of compound **1** (200 mg, 0.94 mmol) and thiomorpholine (97.2 mg, 0.94 mmol) in 0.1 mL of water was stirred overnight (about 18 hours) at room temperature. Then, 3 mL of H₂O and 5 mL of ethyl acetate was added into the mixture. The organic phase was separated, dried, and concentrated to obtain an oily substance. The crude product was purified by silica gel column (methylene chloride:methanol =30:1) to give 125 mg of title product (¹H NMR and MS confirmed, yield 76%).

¹H NMR (400 MHz, CDCl₃): 3.83~3.80 (m, 1H); 3.63~3.60 (m, 1H); 3.30 ~2.26 (m, 1H); 3.10~3.03 (td, J = 10.8, 4.0 Hz, 1H); 2.90~2.85 (m, 2H); 2.73~2.65 (m, 5H); 2.48~2.36 (m, 2H); 2.18~2.16 (m, 1H); 2.10~2.06 (m, 1H); 1.16~1.58(m, 2H); 1.26~1.24(m, 3H); 0.97~0.76 (m, 12H). MS (ESI) calcd for C₁₇H₃₃NO₂S (m/z): 315.22. found: 316.4 [M+1]⁺

### Preparation of compound A0004

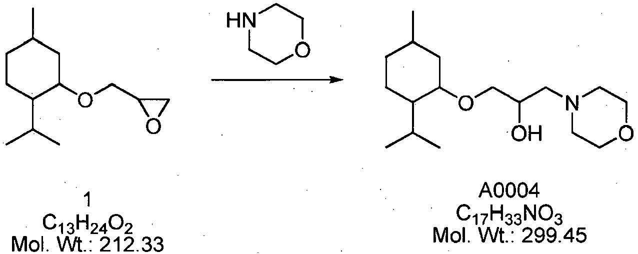

A mixture of compound **1** (200 mg, 0.94 mmol) and morpholine (81.9 mg, 0.94 mmol) in 0.1 mL of water was stirred overnight (about 18 hours) at room temperature. Then, 3 mL of H₂O and 5 mL of ethyl acetate was added into the mixture. The organic phase was separated, dried, and concentrated to obtain an oily substance. The crude product was purified by silica gel column (methylene chloride : methanol =30:1) to give 124 mg of title product (¹H NMR and MS confirmed, yield 44%).

¹H NMR (400 MHz, CDCl₃): 3.92~3.83 (m, 1H); 3.72~3.60 (m, 5 H); 3.32~3.30 (m, 1H); 3.09~3.03 (td, J = 10.8, 4.0 Hz, 1H); 2.68~2.59 (m, 2H); 2.50~2.42 (m, 4H); 2.24~2.16 (m, 1H); 2.12~2.07 (m, 1H); 1.67~1.59 (m, 2H); 1.38~1.20 (m, 3H); 0.98~0.76 (m, 12H) .MS (ESI) calcd for C₁₇H₃₃NO₃ (m/z): 299.25. found: 300.4 [M+1]⁺

### Preparation of compound A0005

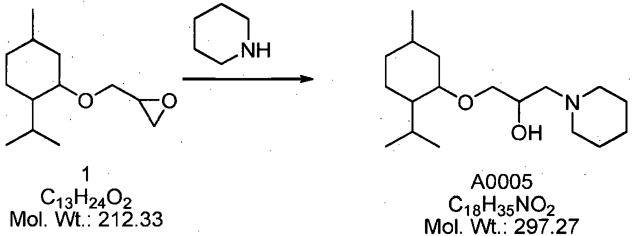

A mixture of compound **1** (200 mg, 0.94 mmol) and piperidine (80 mg, 0.94 mmol) in 0.1 mL of water was stirred overnight (about 18 hours) at room temperature. Then 3 mL of H₂O and 5 mL of ethyl acetate were added into the mixture. The organic phase was separated, dried, and concentrated to obtain an oily substance. The crude product was purified by silica gel column (methylene chloride : methanol =30:1) to give 154 mg of title product (¹H NMR and MS confirmed, yield 55%).

¹H NMR (400 MHz, CDCl₃): 3.92~3.73 (m, 1H); 3.66~3.63 (m, 1H); 3.34 ~2.23 (m, 1H); 3.09~2.95 (td, J = 10.8, 4.0 Hz, 1H); 2.60 (br, 2H) 2.44~2.37 (m, 4H); 2.21~2.10 (m, 2H); 1.78 (br, 1H); 1.67~1.59 (m, 5H); 1.50~1.45 (m, 2H); 1.28~1.25 (m, 3H); 1.00~0.78 (m, 12H). MS (ESI) calcd for C₁₈H₃₅NO₂ (m/z): 2.97.27. found: 2.98.4 [M+1]⁺

### Preparation of compound A0006

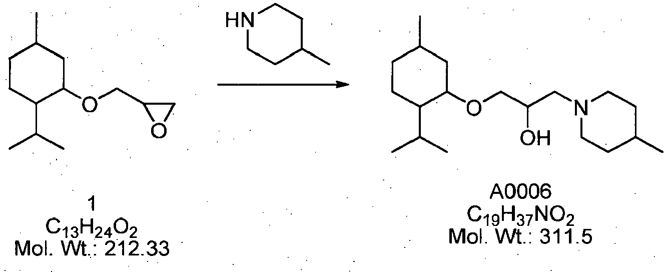

A mixture of compound **1** (200 mg, 0.94 mmol) and 4-methylpiperidine (93.21 mg, 0.94 mmol) in 0.1 mL of water was stirred overnight (about 18 hours) at room temperature. Then 3 mL of H₂O and 5 mL of ethyl acetate was added into the mixture. The organic phase was separated, dried, and concentrated to obtain an oily substance. The crude product was purified by silica gel column (methylene chloride:methanol =30:1) to give 104 mg of title product (¹H NMR and MS confirmed, yield 56%).

¹H NMR (400 MHz, CDCl₃): 3.85 (m, 1H); 3.67~3.62 (m, 1H); 3.34 ~3.29 (m, 1H); 3.09~3.03 (td, J = 10.8, 4 Hz, 1H); 2.97~2.95 (m, 1H); 2.82~2.80 (m, 1H); 2.44~2.39 (m, 2H); 2.24~2.20 (m, 2H); 2.13~2.10 (d, *J* = 12 Hz, 1H); 1.96 (t, *J* = 12 Hz, 1H); 1.69~1.61 (m, 4H); 1.40~1.21 (m, 6H); 1.00~0.79 (m, 15H). MS (ESI) calcd for C₁₉H₃₇NO₂ (m/z): 311.28. found: 312.2 [M+1]⁺

### Preparation of compound A0007

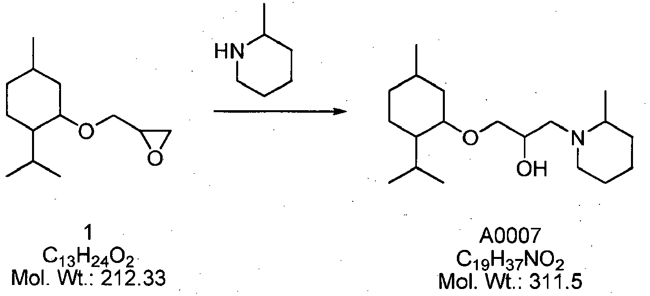

A mixture of compound **1** (200 mg, 0.94 mmol) and 2-methylpiperidine (93.2 mg, 0.94 mmol) in 0.1 mL of water was stirred overnight (about 18 hours) at room temperature. Then, 3 mL of H₂O and 5 mL of ethyl acetate was added into the mixture. The organic phase was separated, dried, and concentrated to obtain an oily substance. The crude product was purified by silica gel column (methylene chloride : methanol =30:1) to give 78 mg of title product (¹H NMR and MS confirmed, yield 27%).

¹H NMR (400 MHz, CDCl₃): 3.84~3.73 (m, 1H); 3.67~3.59 (m, 1H); 3.33 ~3.24 (m, 1H); 3.09~2.95 (m , 1H); 2.80~2.59 (m, 1H); 2.45~2.05 (m, 4H); 1.80~1.45 (m, 8H); 1.40~1.20 (m, 4H); 1.06~1.04 (m, 3H); 0.99~0.77 (m, 12H). MS (ESI) calcd for C₁₉H₃₇NO₂ (m/z): 311.28. found: 312.1 [M+1]⁺

### Preparation of compound A0008

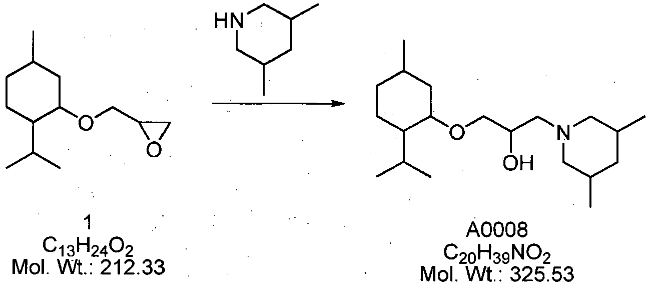

A mixture of compound **1** (200 mg, 0.94 mmol) and 3,5-dimethylpiperidine (106.6 mg, 0.94 mmol) in 0.1 mL of water was stirred overnight (about 18 hours) at room temperature. Then 3 mL of H₂O and 5 mL of ethyl acetate was added into the mixture. The organic phase was separated, dried, and concentrated to obtain an oily substance. The crude product was purified by silica gel column (ethyl acetate : petroleum ether = 1:100) to give 191 mg of title product (¹H NMR and MS confirmed, yield 62.4%).

¹H NMR (400 MHz, CDCl₃): 3.87~3.85 (m, 1H); 3.67∼3.63 (m, 1H); 3.34 ~3.27.(m, 1H); 3.09-2.95 (td, J = 10.8, 4 Hz, 1H) 2.92~2.89 (m, 1H); 2.77~2.75 (m, 1H); 2.43~2.38 (m, 2 H); 2.29~2.18 (m, 1H); 2.03~2.10 (m, 1H); 1.76~1.61 (m, 6 H); 1.50~1.45 (m, 1H); 1.42~1.21 (m, 3H); 1.06~0.54 (m, 18H); 0.56~0.54 (m, 1H). MS (ESI) calcd for C₂₀H₃₉NO₂ (m/z): 325.30, found: 326.5 [M+1]⁺

### Preparation of compound A0009

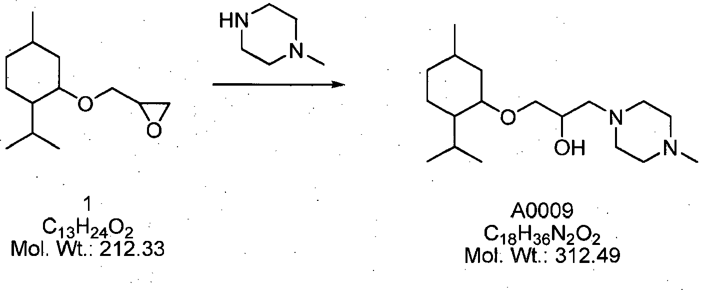

A mixture of compound **1** (200 mg, 0.94 mmol) and 1-methylpiperazine (94.2 mg, 0.94 mmol) in 0.1 mL of water was stirred overnight (about 18 hours) at room temperature. Then, 3 mL of H₂O and 5 mL of ethyl acetate was added into the mixture. The organic phase was separated, dried, and concentrated to obtain an oily substance. The crude product was purified by silica gel column (methylene chloride : methanol =30:1) to give 56 mg of title product (¹H NMR and MS confirmed, yield 19%).

¹H NMR (400 MHz, CDCl₃): 3.89~3.86 (m, 1H); 3.68~3.60 (m, 1H); 3.34~3.30(m, 1H); 3.11~3.05 (td, *J* =10.8, 4.4 Hz, 1H); 2.70 (m, 2 H); 2.53~2.41 (m, 7H); 2.31 (s, 3H); 2.23~2.10 (m , 3H); 1.68~1.61 (m, 2H); 1.37~1.24 (m, 3H); 1.00~0.79 (m, 12H); MS (ESI) calcd for C₁₈H₃₆N₂O₂ (m/z): 312.28,. found: 313.5 [M+1]⁺

### Preparation of compound A0010

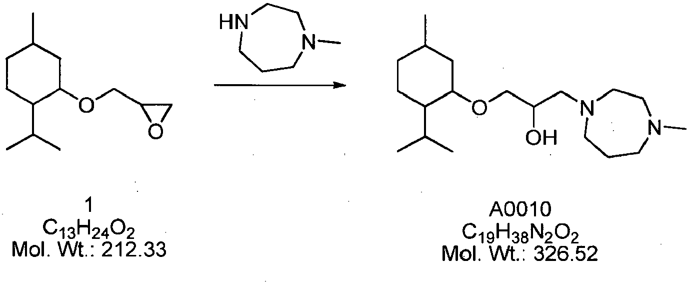

A mixture of compound **1** (200 mg, 0.94 mmol) and 1-methyl-1,4-diazepane (107 mg, 0.94 mmol) in 0.1 mL of water was stirred overnight (about 18 hours) at room temperature. Then, 3 mL of H₂O and 5 mL of ethyl acetate was added into the mixture. The organic phase was separated, dried, and concentrated to obtain an oily substance. The crude product was purified by silica gel column (methylene chloride : methanol=30:1) to give 216 mg of title product (¹H NMR and MS confirmed, yield 70%).

¹H NMR (400 MHz, CDCl₃): 3.86~3.79 (m, 1H); 3.66~3.58 (m, 1H); 3.30~3.26 (m, 1H); 3.11~3.05 (td, *J* = 10.8, 4.4 Hz, 1H); 2.90~2.85 (m, 2H); 2.53~2.41(m, 8H); 2.37~2.51 (m, 1H); 2.357 (s, 3H); 2.22~2.12 (m , 1H); 2.11~2.07 (m, 1H); 1.66~1.58 (m, 5H); 1.37~1.19 (m, 3H); 0.94~0.76 (m, 12H); MS (ESI) calcd for C₁₉H₃₈N₂O₂ (m/z): 326.29,. found: 327.5 [M+1]⁺

### Preparation of compound A0011

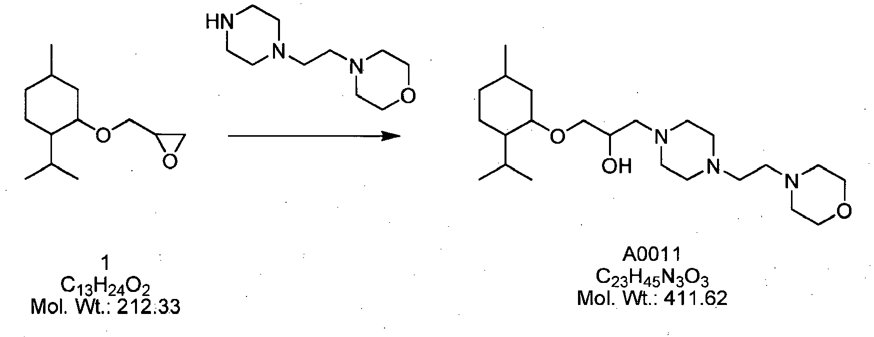

A mixture of compound **1** (200 mg, 0.94 mmol) and 1-(2-morpholinoethyl)piperazine (187.06 mg, 0.94 mmol) in 0.1 mL of water was stirred overnight (about 18 hours) at room temperature. Then 3 mL of H₂O and 5 mL of ethyl acetate was added into the mixture. The organic phase was separated, dried, and concentrated to obtain an oily substance. The crude product was purified by silica gel column (methylene chloride : ethanol=30:1) to give 125 mg of title product (¹H NMR and MS confirmed, yield 35%).

¹H NMR (400 MHz, CDCl₃): 3.87~3.83 (m, 1H); 3.72~3.69 (m, 6H); 3.65~3.60 (m, 1H); 3.30~3.28 (m, 1H); 3.07~3.01 (td, *J* = 10.8, 4.4 Hz; 1H); 2.67 (br, 2 H); 2.53~2.40(m, 9H); 2.23~2.15 (m,3H); 1.67~1.58 (m, 4H); 1.39~1.12 (m, 3H); 0.94~0.76 (m, 15H). MS (ESI) calcd for C₂₄H₄₇N₃O₃ (m/z): 411.35. found: 412.5 [M+1]⁺

### Preparation of compound A0012

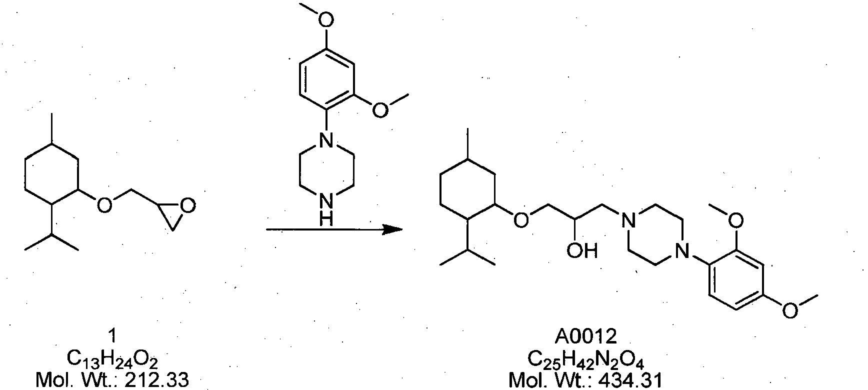

A mixture of compound **1** (100 mg, 0.47 mmol) and 1-(2,4-dimethoxyphenyl) piperazine (105 mg, 0.47 mmol) in 0.1 mL water was stirred overnight (about 18 hours) at room temperature. Then, 3 mL of H₂O and 5 mL of ethyl acetate was added into the mixture. The organic phase was separated, dried, and concentrated to obtain an oily substance. The crude product was purified by silica gel column (methylene chloride : methanol = 30:1) to give 98 mg of the desired product (¹H NMR and MS confirmed, yield 48%).

¹H NMR (400 MHz, CDCl₃): 6.87-6.85 (d, *J* = 8.8 Hz, 1H); 6.48~6.47 (d, *J* = 3.2 Hz, 1H); 6.42 (dd, *J* = 8.4, 2.4 Hz, 1H); 3.86∼3.79 (m, 1H); 3.78 (s, 3 H); 3.66 (s, 3 H); 3.69~3.60 (m, 1H); 3.32~3.24 (m, 1H); 3.08~3.02 (m, 4H); 2.83 (br, 2H); 2.64 (br, 2H); 2.51~2.49 (m, 2H); 2.23~2.15 (m, 1H); 2.12~2.06 (m 1H); 1.64~1.61 (m, 2H); 1.38~1.21 (m, 3H); 0.93~0.78 (m, 12H); MS (ESI) calcd for C₂₅H₄₂N₂O₄ (m/z): 434.31,. found: 435.3 [M+1]⁺

### Preparation of compound A0013

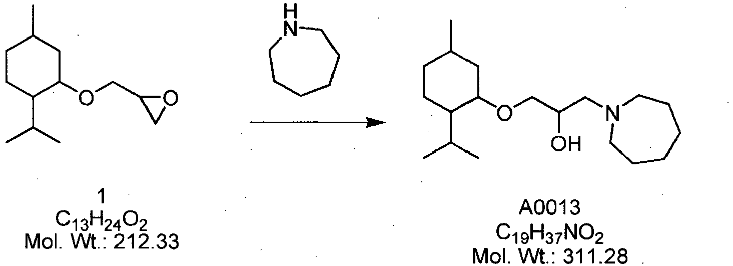

A mixture of compound **1** (150 mg, 0.71 mmol) and azepane (70 mg, 0.71 mmol) in 0.1 mL of water was stirred overnight (about 18 hours) at room temperature. Then 3 mL H₂O and 5 mL ethyl acetate was added into the mixture. The organic phase was separated, dried, and concentrated to obtain an oily substance. The crude product was purified by silica gel column(petroleum ether : ethyl acetate =30:1) and get 72 mg of title product (¹H NMR and MS confirmed, yield 33%).

¹H NMR (400 MHz, CDCl₃): 3.82-3.75 (m, 1H); 3.66~3.61 (m, 1H); 3.34~3.28 (m, 1H); 3.11~3.03 (td, *J* = 10.8, 4.4 Hz, 1H); 2.79~2.75 (m, 2 H); 2.70~2.63 (m, 3H); 2.50~2.45 (m, 1H); 2.20 (m, 1H); 2.12~2.08 (m, 1H); 1.79~1.54 (m, 9H); 1.39~1.20 (m, 3H); 0.99~0.77 (m, 12H); MS (ESI) calcd for C₁₉H₃₇NO₂ (m/z): 311.28, found: 312.6 [M+1]⁺

### Preparation of compound A0014

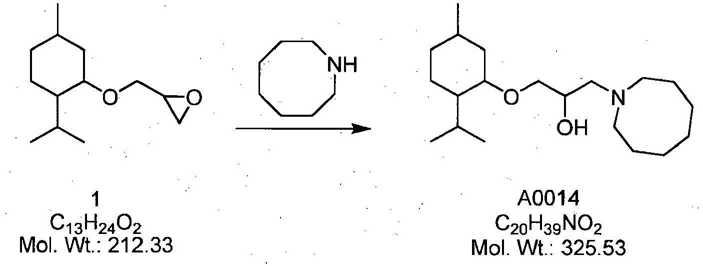

A mixture of compound **1** (100 mg, 0.94 mmol) and azocane (53 mg, 0.47 mmol) in 0.1 mL water was stirred overnight (about 18 hours) at room temperature. Then, 3 mL H₂O and 5 mL ethyl acetate was added into the mixture. The organic phase was separated, dried, and concentrated to obtain an oily substance. The crude product was purified by silica gel column (petroleum ether : ethyl acetate =30:1) and get 64 mg of title product (¹H NMR and MS confirmed, yield 42%).

¹H NMR (400 MHz, CDCl₃): 3.76~3.73 (m, 1H); 3.67~3.62 (m, 1H); 3.35~3.30 (m, 1H); 3.11~3.03 (td, *J* = 10.8, 4.4 Hz, 1H); 2.79~2.65 (m, 2 H); 2.60~2.54,(m, 3H); 2.50~2.41 (m, 1H); 2.22 (m, 1H); 2.12~2.08 (m, 1H); 1.79~1.54 (m, 12H); 1.39~1.19 (m, 3H); 1.07~0.67 (m, 12H). MS (ESI) calcd for C₂₀H₃₉NO₂ (m/z): 325.30. found: 326.5 [M+1]⁺

### Preparation of compound A0015

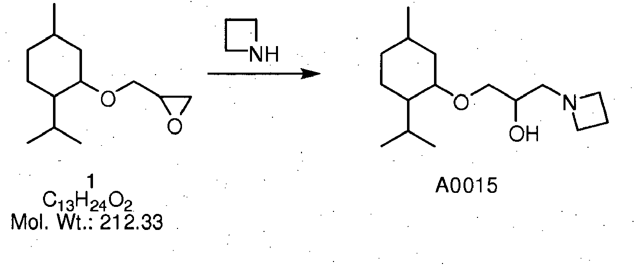

A mixture ot compound **1** (123 mg, 0.58 mmol) and azetidine (33 mg, 0.58 mmol) in 0.1 mL of water was stirred at room temperature overnight (about 18 hours) at room temperature. Next, 3 mL of H₂O and 5 mL ethyl acetate was added into the mixture. Next, the organic phase was separated, dried, and concentrated to obtain an oily residue. The crude product was purified by silica gel column (methylene chloride: methanol=20:1) to give 72 mg of purified product (LC- MS confirmed, yield 46%).

¹H NMR (400 MHz, CDCl₃): 3.79~3.68 (m, 1H); 3.62~3.52 (m, 1H); 3.36~3.15 (m, 2H); 3.05~2.99 (m, 1H); 2.64~2.36 (m, 5 H); 2.18~1.00 (m, 3H); 1.63~1.54 (m, 2H), 1.30~1.17 (m, 3H); 0.98~0.74 (m, 12 H); MS (ESI) calcd for C₁₆H₃₁NO₂ (m/z): 269.24,found: 270.4 [M+1]⁺

### Preparation of compound A0016

### a. Preparation of compound A0034-1

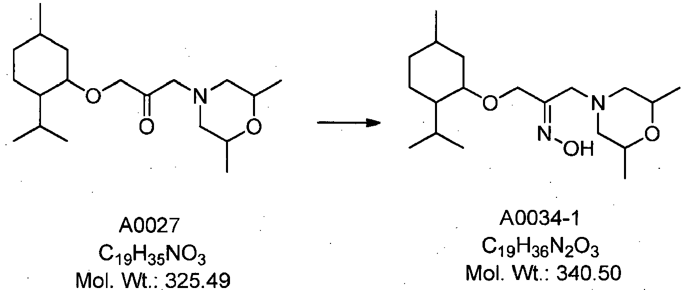

To a mixture of compound **A0027** (100 mg, 0.307 mmol) in 3 mL methanol was added into NH₂OH (HCl) (26 mg, 0.37 mmol) and then triethylamine (40 mg, 0.4 mmol), the mixture was stirred overnight (about 18 hours) at room temperature. Then the solvent was removed and then 5 mL H₂O was added into the mixture, then extracted with ethyl acetate (5 mL x 3), the combined organic phase was then dried and concentrated to get crude title product as a light yellow oil (103 mg TLC and LCMS confirmed, yield 98%).

### b. Preparation of compound A0016

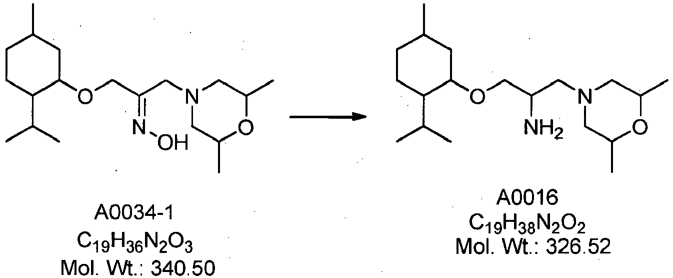

To a solution of LiAlH₄ (25 mg, 0.64 mmol) in 2 ml of tetrahydrofuran(THF) was added a solution of **A0034-1**(100 mg, 0.29 mmol) in 1 ml of THF at room temperature. The mixture was refluxed for 5 hours. 0.5 ml of saturated Na₂SO₄ solution was added and stirred for 30 minutes. Then, 0.5 g Na₂SO₄ was added and the mixture dried over 1 hour. The mixture was filtered and the filtrate was concentrated to get yellow oil (90 mg). Purification by column chromatography (dichloromethane/methanol=10:1) to get yellow oil (20 mg, ¹H-NMR and LC-MS show it was **A0016**, yield 21%, purity 96.6% by ELSD).

¹H NMR (400 MHz, CDCl₃) : 4.789 (br, s, 2H) ; 3.69~3.64 (m, 3H); 3.45~3.27 (m, 2H); 3.09~3.02 (m, 1H); 2.82~2.67 (m, 2H); 2.528 (t, J = 8.0 Hz, 1H); 2.396 (m, 1H); 2.146~1.948 (m, 5H); 1.77~1.60 (m, 3H); 1.34~1.00 (m, 6H), 1.00~0.76 (m, 12H); MS (ESI) calcd for C₁₉H₃₈N₂₀₂ (m/z): 326.29. found: 327.4 [M+1]⁺

### Preparation of compound A0017

### a. Synthesis of compound 2

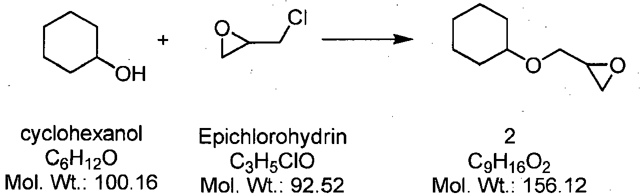

Under nitrogen atmosphere, into a reaction flask were added cyclohexanol (1 g, 10 mmol) and toluene (4 ml) at room temperature. Anhydrous aluminum chloride (0.1 g, 0.8 mmol) was added with stirring to obtain dissolution, and the temperature was raised to 116°C. Epichlorohydrin (1.37 g, 10 mmol) in toluene (2 ml) was added dropwise into the solution. After the addition, the mixture was stirred at the same temperature for 1 hour. Thereafter, the reaction mixture was cooled to 50°C. Under nitrogen atmosphere, a 50% aqueous sodium hydroxide solution (1.6 g) was added and stirred at 75°C overnight (about 18 hours). The mixture was washed with water, and the solvent was removed by evaporation to obtain the crude product. The crude product was purified by silica gel (eluted by petroleum ether) to afford 987 mg of purified product (¹H NMR confirmed, yield 63.2%).

### b. Synthesis of compound A0017

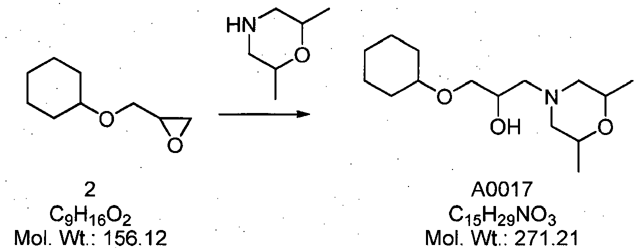

A mixture of compound **2** (987 mg, 6.3 mmol) and 2,6-dimethylmorphine (728 mg, 6.3 mmol) in 0.1 mL of water was stirred at room temperature overnight (about 18 hours). Then 10 mL of H₂O and 5 mL of ethyl acetate were added into the mixture, the organic phase was separated, dried, and concentrated to obtain an oily residue which was purified by silica gel column (dichloromethane:methanol = 20:1) to get 145 mg of the title product (MS confirmed, yield 8.4%).

¹H NMR (400 MHz, CDCl₃): 3.88~3.82 (m, 1H); 3.67~3.64 (m, 2H); 3.48 ~3.38 (m, 2H); 3.24 (m, 1H); 2.80~2.78 (d, *J* = 10.8 Hz, 1H); 2.68~2.65 (d, *J* = 10.8 Hz, 1H); 2.45~2.32 (m, 2H); 1.99~1.94 (t, *J* = 10.8Hz, 1H); 1.87 (m, 2H); 1.75~1.68 (m, 3H); 1.513 (m, 1H); 1.28~1.12 (m, 12H). MS (ESI) calcd for C₁₅H₂₉NO₃ (m/z): 271.21, found: 272.4 [M+1]⁺

### Preparation of compound A0020

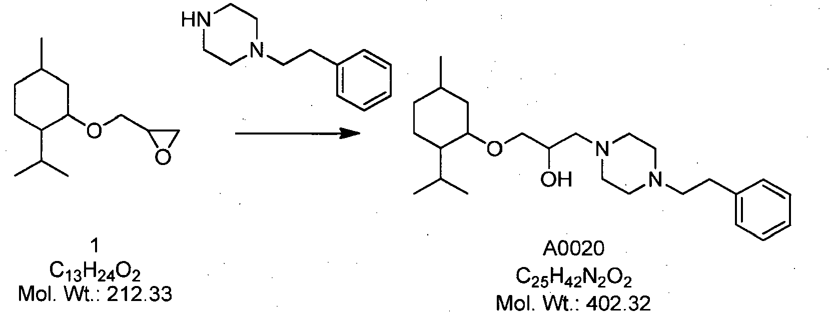

A mixture of compound **1** (200 mg, 0.942 mmol) and 1-phenethylpiperazine (180 mg, 0.942 mmol) in 0.1 mL of water was stirred overnight (about 18 hours) at room temperature. Then 5 mL of H₂O and 10 mL of ethyl acetate was added into the mixture, the organic phase was separated and dried and concentrated to obtain the crude product, then the crude product was purified by silica gel column to get 210 mg of title product (¹H NMR and MS).

¹H NMR (400 MHz, CDCl₃): 7.27~7.17 (m, 5H); 3.83~3.81 (m, 1H); 3.61~3.57 (m, 1 H); 3.31~3.27 (m, 1H); 3.05~3.04 (m, 1 H); 2.80~2.76 (m, 2H); 2.67~2.41 (m, 10H); 2.09~2.06 (m, 1H); 2.02~2.04 (m, 1H); 1.64~1.57 (m , 3H); 1.64~1.61 (m, 2H); 1.38~1.15 (m, 2H); 0.94~0.75 (m, 12H). MS (ESI) calcd for C₂₅H₄₂N₂O₂ (m/z): 402.32, found: 404.2 [M+1]⁺.

### Preparation of compound A0021

### a. Synthesis of compound 3

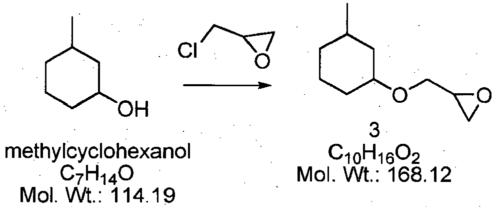

Under nitrogen atmosphere and at room temperature, 3-methylcyclohexanol (1 g, 8.76 mmol) was dissolved in toluene (20 ml). Next, anhydrous aluminum chloride (93 mg, 0.7 mmol) was added to the solution and allowed to dissolve under stirring, and the temperature was raised to 116°C. Into the solution, epichlorohydrin (0.81 g, 8.76 mmol) dissolved in toluene (10 ml) was added dropwise. After the addition, the reaction solution was maintained at the same temperature for 1 hour. After 1 hour, the reaction mixture was cooled to 50 °C and a 50% aqueous sodium hydroxide solution (1.4 g) was added and the reaction solution was maintained at 75 °C overnight (about 18 hours). The mixture was washed with water (20 mL x 2) and then the solvent was removed by evaporation to obtain an oily substance. The crude product was purified by silica gel (eluted by petroleum ether) and 0.8 g of title product was obtained. (yield 53.7 %).

### b. Synthesis of compound A0021

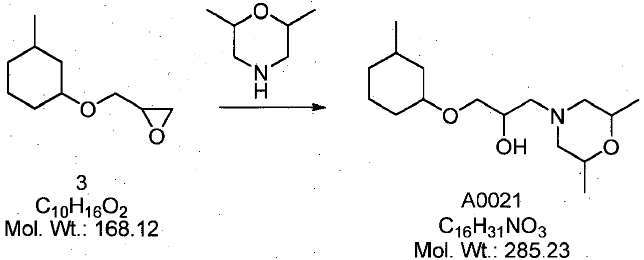

A mixture of compound **3**(800 mg, 4.7 mmol) and 2,6-dimethylmorpholine (542 mg, 4.7 mmol) in 0.2 mL of water was stirred at room temperature overnight (about 18 hours). Then 5 mL of H₂O and 10 mL of ethyl acetate were added into the mixture, the organic phase was separated and dried and concentrated to obtain an oily substance. The crude product was purified by silica gel column and get 165 mg of title product (yield 15.3%, confirmed by ¹H NMR and MS).

¹H NMR (400 MHz, CDCl₃): 3.88~3.82 (m, 1H); 3.67~3.62 (m, 2H); 3.48 ~3.42 (m, 2H); 3.20~3.18 (m, 1H); 2.79~2.75 (m, 1H) 2.67~2.64 (m, 1H); 2.40~2.35 (m, 2H); 2.0~1.93 (m, 2H); 1.75~1.70 (m, 2H); 1.59~1.56 (m, 2H); 1.30~1.41 (m, 1H); 1.23~1.01 (m, 9H); 0.91~0.79 (m, 5H); MS (ESI) calcd for C₁₆H₃₁NO₃ (m/z): 285.23, found: 285.9 [M+1]⁺

### Preparation of compound A0022

### a. Synthesis of compound 4

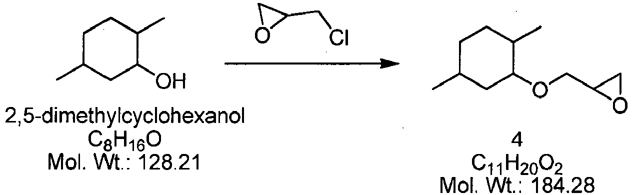

To a mixture of compound epichlorohydrin (145 mg, 1.56 mmol) in NaOH (50% w/w) (1.04 g, 13 mmol) was added compound 2,5-dimethylcyclohexanol (200 mg, 1.56 mmol) and Bu₄HSO₄ (22 mg, 0.06 mmol), the mixture was stirred at room temperature overnight (about 18 hours). Then, 3 mL of H₂O was added into the mixture, extracted with ethyl acetate (5 mL x 3) and the combined organic phase was dried and concentrated to get crude product which was purified by silica gel column (eluted with dichloromethane) to afford 120 mg of title product (yield: 41.7 %, confirmed by thin-layer chromatography).

### b. Synthesis of compound A0022

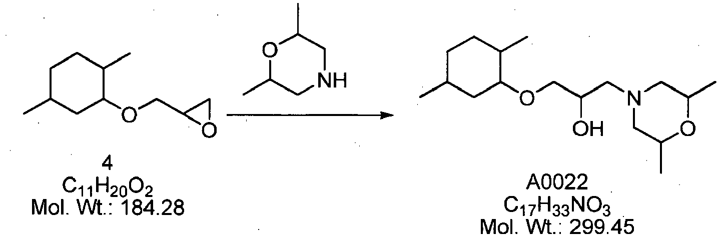

A mixture of compound **4** (120 mg, 0.65 mmol) and 2,6-dimethylmorpholine (75 mg, 0.65 mmol) in 0.1 mL was stirred overnight (about 18 hours) at room temperature. Then, 5 mL of H₂O and 8 mL of ethyl acetate was added into the mixture, the organic phase was separated, dried, and concentrated to obtain an oily substance. Then, the crude product was purified by silica gel column (methylene chloride : methanol=30:1) to afford 48 mg of title product (confirmed by ¹H NMR and MS, yield:10.3%, HPLC: 96.7%)

¹H NMR (400 MHz, CDCl₃): 3.88~3.82 (m, 1H); 3.67~3.61 (m, 3H); 3.47~3.45 (m, 1H); 3.26~3.12 (m, 2H); 2.80~2.77 (m, 1H) 2.69~2.65 (m, 1H); 2.43~2.33 (m, 2H); 2.15 (br, 0.42 H); 2.0~1.96 (m, 1H); 1.75~1.33 (m, 6H); 1.22~1.20 (m, 1H); 1.14~1.12 (d, J = 6.8 Hz, 6H); 0.96~0.83 (m, 7H). MS (ESI) calcd for C₁₇H₃₃NO₃ (m/z): 299.25, found: 300.4 [M+1]⁺

### Preparation of compound A0023

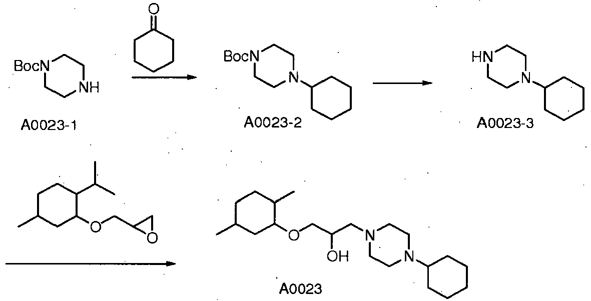

### a) Preparation of compound A0023-2

To a solution of compound **A0023-1** (1 g, 5.35 mmol) and 0.53 g of cyclohexanone in 15 mL dichloromethane was added NaBH(OAc)₃ (1.7 g) and acetic acid (0.322 g). The mixture was stirred overnight (about 18 hours) at room temperature under an Argon atmosphere. Then the mixture was quenched with 1N NaOH (10 mL) and extracted with ethyl acetate (10 mL x 3). The combined organic phase was dried and concentrated to get 1.38 g of title product (yield 96%, LC-MS confirmed).

### b) Preparation of compound A0023-3

A solution of comp **A0023-2** (1.38 g, 5.15 mmol) in ethanol/hydrochloric acid (15 mL) was stirred overnight (about 18 hours) at room temperature. Then, the solvent was removed and 5 mL of H₂O was added into the mixture. The mixture was washed with ethyl acetate (5 ml x 3) and the aqueous phase was neutralized to pH=7~8 and extracted with ethyl acetate (10 ml x 3). The combined organic phase was dried and concentrated and to yield 0.78 g of the title product (LC-MS and TLC confirmed, yield: 90.1%).

### c) Preparation of compound A0023

A mixture of compound **1** (200 mg, 0.94 mmol) and compound A0023-2 (159 mg, 0.94 mmol) in 0.2 mL of water was stirred at overnight (about 18 hours) at room temperature. then 5 mL of H₂O and 10 mL of ethyl acetate was added into the mixture, the organic phase was separated, dried, and concentrated to obtain an oily crude product. The crude product was purified by silica gel column to get the title product (123 mg, yield: 34%, HPLC: 98.3% by ELSD).

¹H NMR (400 MHz, CDCl₃) : 3.79~3.71 (m, 1H); 3.67~3.58 (m, 1H); 3.30~3.26 (m, 1H); 3.07-3.01 (td, *J* = 10.8, 4.4 Hz, 1H); 2.59 (m, 4 H); 2.48~2.35 (m, 4H); 2.22~2.17 (m, 2H); 2.10~2.07 (m, 1H); 1.89~1.77 (m, 5H); 1.64~1.58 (m, 3H); 1.36~1.04 (m, 8H); 0.97~0.75 (m, 13H). MS (ESI) calcd for C₂₃H₄₄N₂₀₂ (m/z): 380.34. found: 381.3 [M+1]⁺

### Preparation of compound A0025

### a. Preparation of compound 5

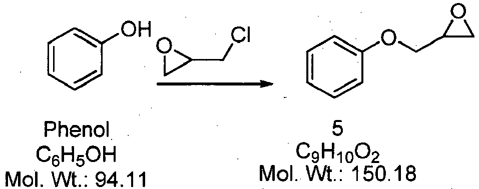

To a mixture of compound epichlorohydrin (0.98 g, 10.6 mmol) in NaOH (50%) (7 g, 88 mmol) was added phenol (1 g, 10.6 mmol) and NaBu₄HSO₄ (143 mg, 3.52 mmol), the mixture was stirred overnight (about 18 hours) at room temperature. Then, the reaction mixture was extracted with ethyl acetate (10 ml X 2). The combined organic phase was dried and concentrated to get crude product and the crude product was purified by silica gel column to afford 1.38 g of the title product. (yield 86.8%, ¹H NMR confirmed).

### b. Preparation of compound A0025

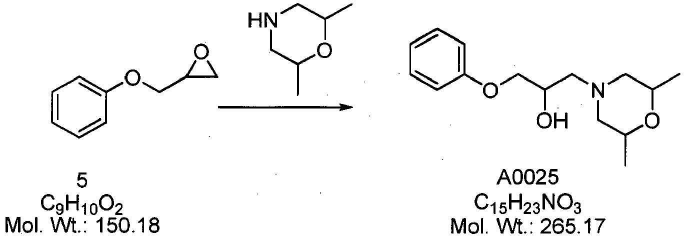

The mixture of 2 X 6-dimethylmorpholine (813 mg, 7.07 mmol), **5** (530 mg, 3.53 mmol) and H₂O (0.5 mL) was stirred overnight (about 18 hours) at room temperature. TLC suggested the reaction complete.

The reaction mixture was extracted with ethyl acetate and washed with brine, dried with anhydrous sodium sulfate, concentrated under vacuum to afford 1.037 g of crude product as yellow liquid (crude yield: 110 %). The crude product was purified via column chromatography to afford 222 mg of target product (yield: 24 %) and 130 mg of the isomer (yield: 14 %). The structure of target product was confirmed by ¹H NMR & MS. Purity: 96% by HPLC.

¹H NMR (400 MHz, CDCl₃): 7.30-7.25 (m, 2H) ; 6.97-6.91 (m, 3H); 4.14-4.10 (m, 1H); 3.98 (d, J = 5.2 Hz, 2H); 3.73-3.65 (m, 2H); 2.85 (d, J = 11.2 Hz, 1H); 2.70 (d, J = 10.8 Hz, 1H); 2.59-2.48 (m, 2H); 2.05 (t, J = 10.4 Hz, 1H); 1.80 (t, J = 10.4 Hz, 1H); 1.17 (dd, J = 6.0, 1.2 Hz, 6H); calcd for C₁₅H₂₃NO₃ (m/z): 265.17, found: 266.1 [M+1]⁺.

### Preparation of compound A0026

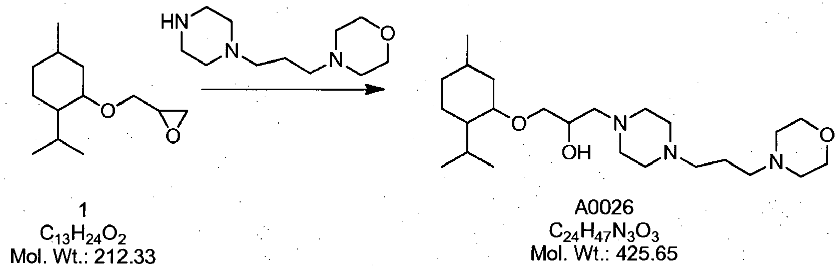

A mixture of compound **1** (200 mg, 0.94 mmol) and 4-(3-piperazin-1-ylpropyl)morpholine (201 mg, 0.94 mmol) in 0.2 mL of water was stirred overnight (about 18 hours) at room temperature. Then, 5 mL of H₂O and 10 mL of ethyl acetate was added into the mixture, the organic phase was separated, dried and concentrated to obtain an oily crude product that was purified by silica gel column (methylene chloride : ethanol=30:1) to give 165 mg of the desired product (confirmed by ¹H NMR, yield:41.2%).

¹H NMR (400 MHz, CDCl₃): 3.86~3.80 (m, 1H); 3.72~3.69 (m, 5H); 3.66~3.59 (m, 1H); 3.30~3.26 (m, 1H); 3.07~3.01 (td, *J* = 10.8, 4.4 Hz; 1H); 2.67~2.66 (br, 2 H); 2.50~2.34 (m, 11H); 2.23~2.12 (m, 1H); 2.11~2.07 (m , 1H); 1.72~1.58 (m, 6H); 1.39~1.12 (m, 3H); 0.94~0.76 (m, 14H). MS (ESI) calcd for C₂₄H₄₇N₃O₃. (m/z): 425.36, found: 426.4 [M+1]⁺

### Preparation of compound A0027

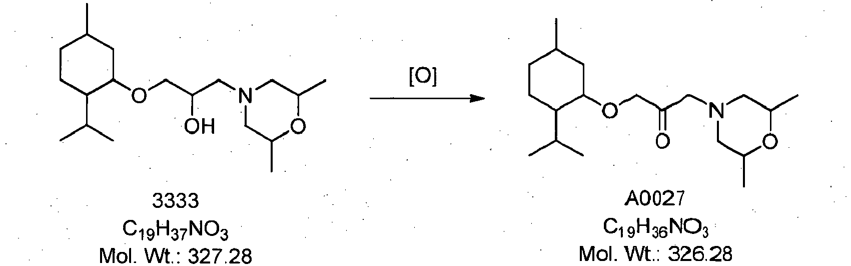

To a solution of oxalyl chloride (0.34 mL, 4 mmol) in dichloromethane (3 mL) at -78 °C was treated with a solution of dimethylsulfoxide (DMSO) (0.52 mL,7.3 mmol) in dichloromethane (0.5 mL) and the resulting mixture was warmed to -60 °C for 20 min. Then a solution of compound **3333** (532 mg) in dichloromethane (4 mL) was added and the mixture was stirred at -40 °C for 1.5 hours. Trimethylamine (2.3 mL) was added and the mixture was stirred at -40 °C for 30 min. The mixture was warmed to room temperature and water was added and the mixture was extracted with dichloromethane and the combined extracts were washed brine, dried over Na₂SO₄. Concentrated and purified by silica gel (petroleum ether:petroleum ether: ethyl acetate =30:1~15:1~10:1) to give compound **A0027** as colorless oil (219 mg, 42% yield, confirmed by ¹H NMR, LC-MS and HPLC).

### Preparation of compound A0028

### a. Preparation of compound 6

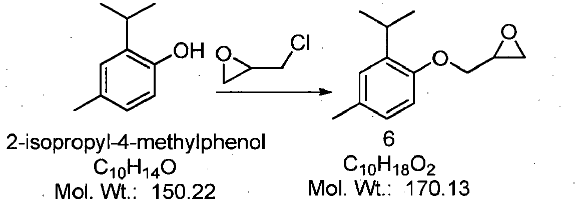

To a solution of NaOH (6.6 g, 166 mmol) in H₂O (7 mL) was added 2-isopropyl-5-methylphenol (3 g, 20 mmol), (n-C₄H₉) ₄N⁺HSO₄⁻ (271 mg, 0.8 mmol) and 2-(chloromethyl)oxirane (6.2 mL, 80 mmol) in portions at 0 °C. The reaction mixture was stirred overnight (about 18 hours) at room temperature. Thin-layer chromatography suggested the reaction complete. The reaction mixture was extracted with ethyl acetate, combined the organic layers, dried with anhydrous sodium sulfate, concentrated under vacuum to afford **5.3 g** of crude product as a yellow liquid. The crude product (2.2 g) was purified via column chromatography to afford 1.023 g of the desired product as colorless liquid (yield: 59%) **b. Preparation of compound A0028**

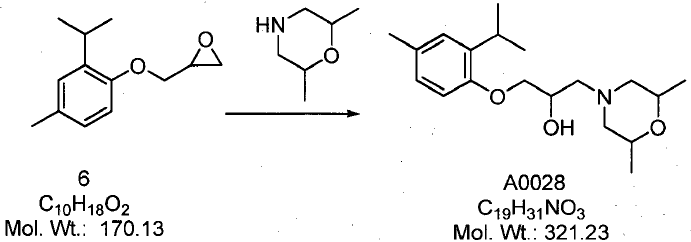

The mixture of 3, 6-dimethylmorpholine (478 mg, 4.157 mmol), **6**(500 mg, 2.427 mmol) and H₂O (0.4 mL) was stirred overnight (about 18 hours) at room temperature. TLC suggested the reaction complete; the reaction mixture was extracted with ethyl acetate and washed with brine, dried with anhydrous sodium sulfate, concentrated under vacuum to afford 675 mg of crude product as a yellow liquid. The crude product was purified via column chromatography to afford 2 products: 150 mg of confirmed by ¹H NMR and MS to be the desired product, 96.7% purity by HPLC.

¹H NMR (400 MHz, CDCl₃): 7.10 (d, J = 7.6Hz, 1H); 6.76 (d, J = 7.6 Hz, 1H); 6.67 (s, 1H); 4.16-4.11 (m, 1H); 4.03-3.94 (m, 2H); 3.75-3.66 (m, 2H); 3.30-3.23 (m, 1H); 2.86 (d, J = 10.8 Hz, 1H); 2.70 (d, J = 10.8 Hz, 1H); 2.61-2.51 (m, 2H); 2.32 (s, 3H); 2.06 (t, J = 10.8 Hz, 1H); 1.81 (t, J = 10.8 Hz, 1H) ; 1.22 -1.17 (m, 12H) ; MS (ESI) calcd for C₁₉H₃₁NO₃ (m/z): 321.23, found: 322.3 [M+1]⁺.

### Preparation of compound A0029

### a. Preparation of compound 7

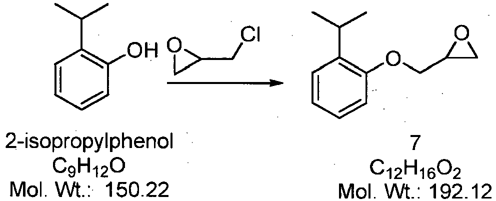

To a solution of NaOH (2.5 g, 60.9 mmol) in H₂O (3 mL) was added 2-isopropylphenol (1 g, 7.34 mmol), (n-C₄H₉)₄N⁺HSO₄⁻ (99 mg, 0.29 mmol) and 2-(chloromethyl)oxirane (2.4 mL, 30 mmol) in portions at 0 °C. The reaction mixture was stirred overnight (about 18 hours) at room temperature. TLC suggested the reaction complete. The reaction mixture was extracted with ethyl acetate, combined the organic layers, dried with anhydrous sodium sulfate, concentrated under vacuum to afford 1.293 g of crude product as a yellow liquid. The crude product was purified via column chromatography to afford 176 mg of the desired product as a colorless liquid (yield: 12%).

### b. Preparation of compound A0029

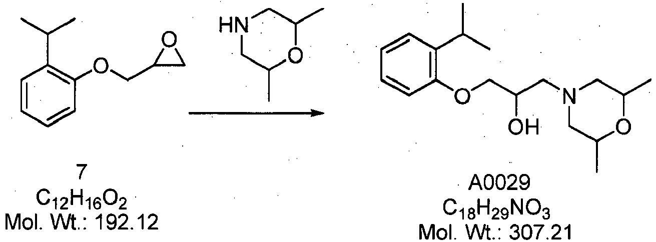

The mixture of 2,6-dimethylmorpholine (210 mg, 1.833 mmol), **7** (176 mg, 0.917 mmol) and H₂O (0.2 mL) was stirred overnight at room temperature. The reaction mixture was extracted with ethyl acetate and washed with brine, dried with anhydrous sodium sulfate, concentrated under vacuum to afford 293 mg of crude product as yellow liquid (yield : 104 %). The crude product was purified via column chromatography to afford 79 mg of the desired product (yield: 28 %, ¹H NMR & MS confirmed, Purity 95.7% by HPLC).

¹H NMR (400 MHz, CDCl₃): 7.22 (d, J = 8.0 Hz, 1H); 7.15 (t, J = 8.0 Hz, 1H); 6.94 (t, J = 7.6Hz, 1H); 6.84 (d, J = 8.4 Hz, 1H); 4.16-4.13 (m, 1H); 4.05-3.96 (m, 2H); 3.74-3.66 (m, 2H); 3.36-3.28 (m, 1H); 2.86 (d, J = 11.2 Hz, 1H); 2.70 (d, J = 10.4 Hz, 1H); 2.61-2.51 (m, 2H); 2.06 (t, J = 10.4 Hz, 1H); 1.81 (t, J = 10.6 Hz, 1H); 1.23 - 1.17 (m, 12 H); calcd for C₁₈H₂₉NO₃ (m/z): 307.21, found: 308.3 [M+1]⁺.

### Preparation of compound A0030

### a. Preparation of compound 8

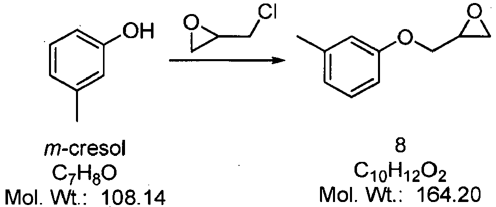

To the mixture of compound m-cresol (3 g, 27.7 mmol), 50% w/w NaOH aqueous (10 mL) and (n-C₄H₉)₄N+HSO₄⁻ was added 2-(chloromethyl)oxirane (10 mL, 129 mmol) in portions at 0 °C. The reaction mixture was stirred at room temperature overnight (about 18 hours). TLC suggested the reaction complete. The reaction mixture was extracted with ethyl acetate, the organic layers combined, dried with anhydrous sodium sulfate, and concentrated under vacuum to afford 3.33 g of crude product as a colorless liquid (yield: 72%). The crude product (1.566 g) was purified via column chromatography to afford 1.319 g of final product (yield: 60.6 %).

### b. Preparation of compound A0030

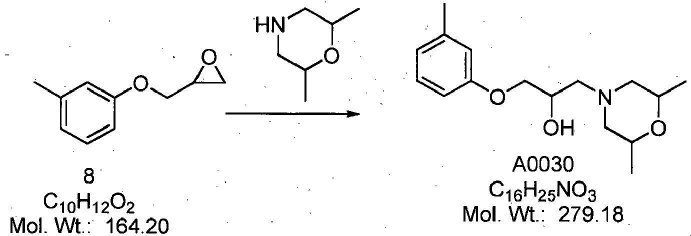

The mixture of 2,6-dimethylmorpholine (420 mg, 3.659 mmol), **8** (500 mg, 3.049 mmol) and H₂O (0.4 mL) was stirred overnight at room temperature. TLC suggested the reaction complete. The reaction mixture was extracted with ethyl acetate and washed with brine, dried with anhydrous sodium sulfate, concentrated under vacuum to afford 703 mg of crude product as yellow liquid (yield : 83 %). The crude product was purified via column (yield: 72 %), ¹H NMR and MS confirmed it was the desired product, 97.4%. purity by HPLC).

¹H NMR (400 MHz, CDCl₃): 7.16 (t, J = 8.0 Hz, 1H); 6.79-6.72 (m, 3H); 4.14-4.08 (m, 1H); 3.98 (d, J = 4.8 Hz, 2H); 3.75-3.64 (m, 2H); 2.85 (d, J = 10.8 Hz, 1H); 2.71 (d, J = 11.2 Hz, 1H); 2.59-2.48 (m, 2H); 2.33 (s, 3H); 2.05 (t, J = 11.2 Hz, 1H); 1.81 (t, J = 10.0 Hz, 1H); 1.18 (s, 3H); 1.14 (s, 3H); calcd for C₁₆H₂₅NO₃ (m/z) : 279.18, found: 280.3 [M+1]⁺.

### Preparation of compound A0031

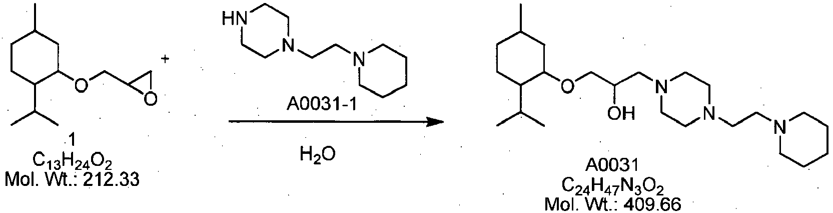

A mixture of A0031-1 100 mg, 0.508 mmol), compound **1** (86 mg, 0.406 mmol) and H₂O (0.2 mL) was stirred overnight (about 18 hours) at room temperature. TLC showed that some of the start materials remained. The reaction mixture was then heated to 40°C and stirred overnight (about 18 hours). The reaction mixture was extracted with ethyl acetate, washed with water and brine, dried with anhydrous sodium sulfate and concentrated under vacuum to afford 70 mg of crude product as a yellow liquid (yield:41.9 %). To the crude product, 0.5 ml of diethyl ether-HCl was added and a white solid appeared. The white solid was washed with petroleum ether:ethyl acetate 7:1 two times. The pH of a solution containing the solid was adjusted to pH = 2 with Na₂CO₃, and that solution was extracted with dichloromethane. The organic layers were combined, washed with water and brine, dried with anhydrous. sodium sulfate and concentrated under vacuum to afford 67 mg of the purified product (yield 40.3%, confirmed by ¹H NMR & MS, purity 91.8% by HPLC).

¹H NMR (400 MHz, CDCl₃): 3.85-3.81 (m, 1H); 3.65-3.58 (m, 1H); 3.30-3.25 (m, 1H); 3.04 (td, J =10.0, 3.6 Hz, 1H); 2.64 (s, 1H); 2.54-2.36 (m, 13H); 2.22-2.15 (m, 2H); 2.08 (d, J = 11.2 Hz, 2H); 1.65-1.54 (m, 6H); 1.43-1.42 (m, 2H); 1.34-1.29 (m, 2H); 1.25-1.20 (m, 2H); 0.75-1.00 (m, 13 H); calcd for C₂₄H₄₇N₃O₂ (m/z): 409.37, found: 410.4 [M+1]⁺.

### Preparation of compound A0032

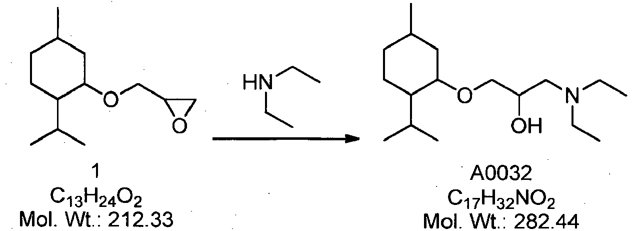

A suspension of compound **1** (100 mg, 0.47 mmol) and compound diethylamine (69 mg, 0.94 mmol) in 0.1 mL H₂O stirred overnight (about 18 hours) at room temperature. Then 5 mL of H₂O and 5 mL of ethyl acetate were added into the mixture, then the organic phase was separated, dried and concentrated to get crude product. Purification by column chromatography (petroleum ether:ethyl acetate=1:1) to get the desired product as pale yellow oil (37 mg, yield 27%, ¹H NMR and MS confirmed, purity: 100% by ELSD).

### Preparation of compound A0033

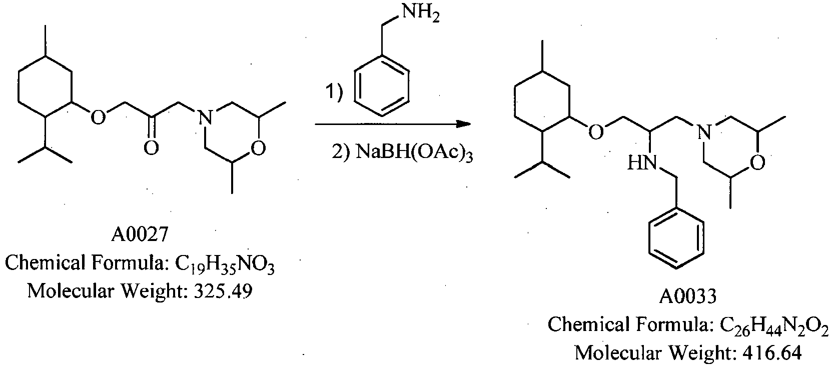

Phenylmethanamine (48 mg, 0.44 mmol) followed by NaBH(OAc)₃(109 mg, 0.52 mmol) and acetic acid (22 mg,0.37 mmol ) was added to a mixture of compound **A0027** (120 mg, 0.37 mmol) in 5 mL dichloromethane. The mixture was stirred overnight (about 18 hours) at room temperature. Then the mixture was quenched with 3 mL 1N NaOH and 5 mL H₂O was added into the mixture. Next, the mixture was extracted with ethyl acetate (5 mL x 3) and the combined organic phase was then dried and concentrated to get crude product. The crude product was purified to get 108 mg of title product.

### Preparation of compound A0035

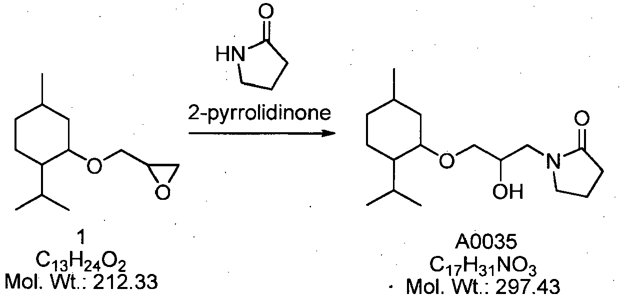

To a solution of compound 2-pyrrolidinone (72 mg, 0.85 mmol) in 3 ml of dioxane was added 60%NaH (26 mg, 1.08 mmol), with stirring for 30 minutes. Next, compound **1** (100 mg, 0.47 mmol) in 2 ml of dioxane was added to the mixture with stirring at 100°C overnight (about 18 hours). Ethyl acetate (8 ml) was added into the mixture, the mixture was washed three times with H₂O (5 ml), and the organic phase was concentrated to get crude product (168 mg). The crude product was then purified to a colorless oil (38 mg, ¹H NMR and LCMS confirmed, yield:1.3%, purity 96.6% by HPLC-ELSD)

### Preparation of compound A0036

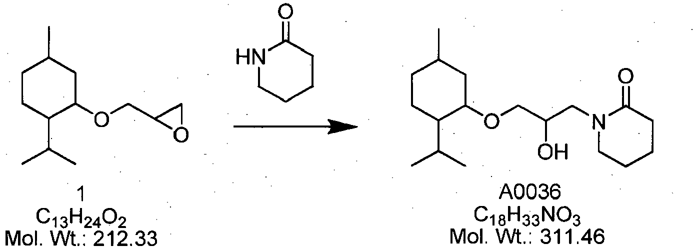

About 42 mg of NaH, as a 60% dispersion in mineral oil was suspended in 2 ml absolute dimethylsulfoxide(DMSO), was stirred at room temperature for 10 minutes, admixed with 2-piperidone (51.4 mg, 0.519 mmol) and stirred for 1 hour. Next, a solution of compound **1** (100 mg, 0.472 mmol) in 2 ml absolute DMSO was added drop-wise. The mixture was then stirred overnight (about 18 hours) and then heated to 55°C and stirred for 4 additional hours. Next, the reaction mixture was dissolved in ethyl acetate (EA), washed with water and brine, dried with anhydrous sodium sulfate and concentrated under vacuum to afford 90 mg of crude product. The crude product was then purified via column chromatography two times (petroleum ether:ethyl acetate 20:1 to ethyl acetate) to afford 30 mg of the purified product (yield:20%). The structure was confirmed by ¹H NMR & MS, purity 93.6% by HPLC.

¹H NMR (400 MHz, CDCl₃): 4.10-4.04 (m, 1H); 3.90-3.84 (m, 1H); 3.57-3.43 (m, 2H); 3.36-3.30 (m, 2H); 3.24-3.16 (m, 1H); 3.07 (td, J = 10.4, 4.0 Hz, 1H); 2.41 (t, J = 6.0 Hz, 2H); 2.11-2.01 (m, 2H); 1.78-1.72 (m, 3H); 1.60-1.52 (m, 2H); 1.31-1.11 (m, 4 H) ; 0.76-0.73 (m, 12H); calcd for C₁₈H₃₃NO₃ (m/z) : 311.25, found: 312.4 [M+1]⁺.

### Preparation of compound A0037

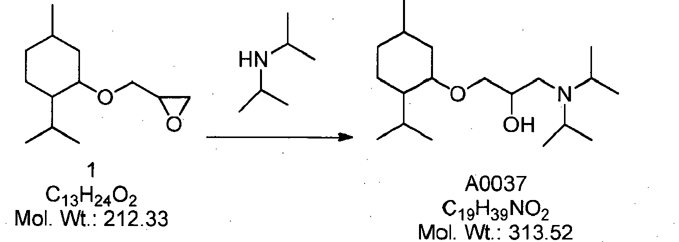

A suspension of compound 1 (100 mg, 0.47 mmol) and diisopropylamine (48 mg, 0.47 mmol) in 0.2 mL H₂O were stirred at 50 °C overnight (about 18 hours). Then 5 mL H₂O and 5 mL ethyl acetate were added into the mixture and the organic phase was separated, dried and concentrated to get crude product. The crude product was purified yielding the title product as a colorless oil (28 mg, yield :19%, ¹H NMR and LCMS confirmed).

### Preparation of compound A0038

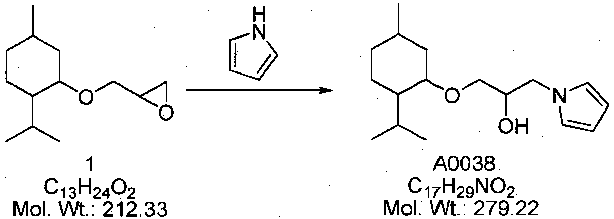

About 13 mg of NaH as 60% dispersion in mineral oil was suspended in 3 ml absolute dimethylformamide (DMF), stirred at room temperature for 10 minutes, then admixed with pyrrole (34.8, 0.519 mmol) for 1 hour. Next, a solution of compound **1** (100 mg, 0.472 mmol) in 2 ml absolute DMF was added drop-wise with stirring overnight (about 18 hours). The solution was heated to 45°C and stirred for three more hours. Then, 20 ml water was added to the reaction mixture followed by extraction with diethyl ether. The organic layers were saved and combined, then washed with 1 N HCl, then brine, and dried with anhydrous Na₂SO₄. The crude product was concentrated under vacuum to afford 108 mg of crude product and was purified via column chromatography (petroleum ether/ethyl acetate =50/1 to petroleum ether/ethyl acetate =20/1) to afford 16 mg of purified product (yield: 13.7 %).

¹H NMR (400 MHz, CDCl₃): 6.70-6.68 (m, 2H); 6.18-6.16 (m, 2H); 4.06-3.92 (m, 3H); 3.65-3.50 (m, 1H); 3.30-3.15 (m, 1H); 3.12-3.05 (m, 1H); 2.36-2.32 (m, 1H); 2.22-2.14 (m, 1H); 2.10-2.05 (m, 1H); 1.69-. 1.61 (m, 2H); 1.40-1.23 (m, 2H); 1.03- 0.78 (m, 12H); calcd. for C₁₇H₂₉NO₂ (m/z): 279.22, found: 280.2 [M+1]⁺.

### Preparation of compound A0039

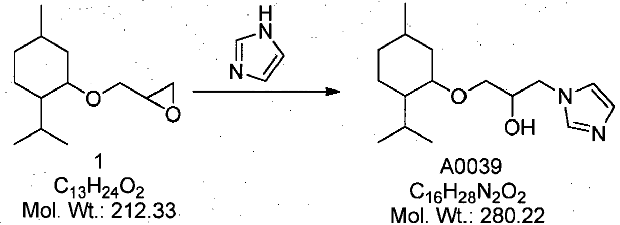

A mixture of compound **1** (100 mg, 0.472 mmol), imidazole (64 mg, 0.943 mmol) and H₂O (0.2 ml) was stirred at room temperature overnight (about 18 hours). The reaction mixture was heated to 40 °C and stirred for 4 hours. After the reaction was complete, the reaction mixture was extracted with ethyl acetate and washed with brine, dried with anhydrous sodium sulfate, concentrated under vacuum to afford 116 mg of crude product as a yellow liquid. (yield: 87.7%). The crude product was purified via column chromatography (ethyl acetate ethanol = 10:1) to afford 70 mg of the desired product (yield 53%, confirmed by ¹H NMR & MS. Purity: 97% by HPLC).

### Preparation of compound A0040

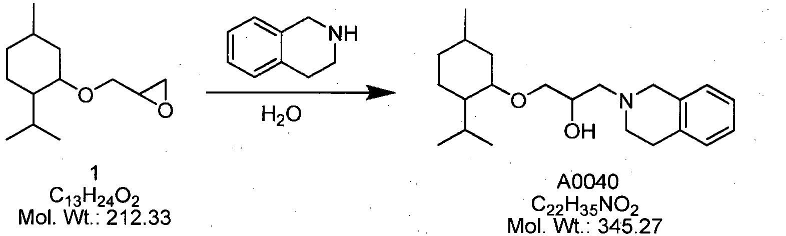

A mixture of compound 1,2,3,4-tetrahydroisoquinoline (133 mg, 0.944 mmol), compound **1** (100 mg, 0.472 mmol) and H₂O (0.2 ml) was stirred overnight (about 18 hours) at room temperature. The reaction mixture was extracted with ethyl acetate, washed with water and brine, dried with anhydrous sodium sulfate, and concentrated under vacuum to afford 221 mg of crude product. The crude product was purified via column chromatography (petroleum ether/ethyl acetate =50/1 to petroleum ether/ethyl acetate =10/1) to afford 100 mg of purified product (yield:61.4%). The structure was confirmed by ¹H NMR & MS, purity 94.6% by HPLC.

¹H NMR (400 MHz, CDCl₃): 7.17-7.09 (m, 3H); 7.03-7.01 (m, 1H); 3.99-3.96 (m, 1H); 3.83-3.79 (m, 1H); 3.72-3.63 (m, 2H); 3.38-3.34 (m, 1H); 3.11-3.04 (m, 1H); 2.97-2.87 (m, 3H); 2.80-2.72 (m, 1H); 2.69-2.57 (m, 2H); 2.26-2.18 (m, 1H); 2.13-2.10 (m, 1H); 1.68-1.60 (m, 2H); 1.41-1.22 (m, 2H); 1.03-0.65 (m, 13H); calcd for C₂₂H₃₅NO₂ (m/z) : 345.27, found: 346.3 [M+1]⁺.

### Preparation of compound A0041

### a. Preparation of compound 9

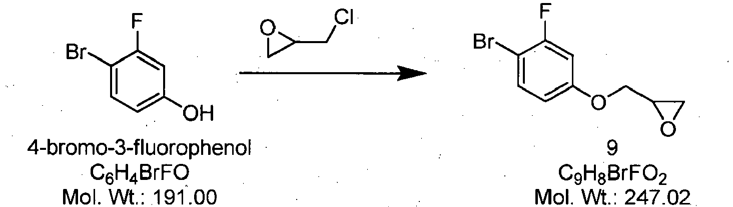

To a solution of NaOH (1.72 g, 43.1 mmol) in H₂O (2.3 ml) was added 4-bromo-3-fluorophenol (1 g, 5.2 mmol), (n-C₄H₉)₄N⁺HSO₄ (70 mg, 0.208 mmol) and 2-(chloromethyl) oxirane (1.91,g, 20.8 mmol) at 0°C. The reaction mixture was then stirred overnight (about 18 hours) at room temperature. After the reaction was complete, 30 ml of water was added to the reaction and then extracted with chloroform. The organic layers were combined, washed with brine, dried with anhydrous sodium sulfate, and concentrated to provide the crude product. The crude product was purified via column chromatography (petroleum ether:CH₂Cl₂ =2:1) to provide 700 mg of purified product (yield: 54%).

### b. Preparation of compound A0041

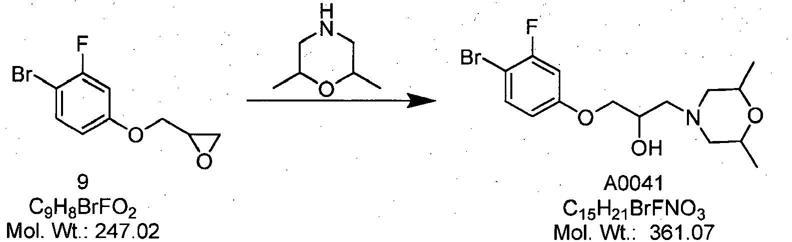

A mixture of 2,6-dimethylmorpholine (200 mg, 1.7 mmol), compound **9** (210 mg, 0.85 mmol) and H₂O (0.2 ml) was stirred overnight (about 18 hours) at room temperature. After the reaction was complete, the reaction mixture was then extracted with CHCl₃, washed with water and brine, dried with anhydrous sodium sulfate, and concentrated under vacuum to afford the crude product. The crude product was then purified via column chromatography (petroleum ether:ethyl acetate = 20:1 to petroleum ether:ethyl acetate = 1:1) to afford 132 mg of purified product (yield:42.7%) confirmed by ¹H NMR & LC-MS, purity 97.2% by HPLC).

¹H NMR (400 MHz, CDCl₃): 7.40 (t, *J* = 8.0 Hz, 1H); 6.73 (dd, *J* = 2.8, 10.4 Hz, 1H); 6.65-6.62 (m, 1H); 4.13-4.07 (m, 1H).; 3.96-3.91 (m, 2H); 3.73-3.64(m, 2H); 2.83(d, *J* = 11.2 Hz, 1H); 2.68(d, *J* = 11:2 Hz, 1H); 2.57-2.45 (m, 2H); 2.06 (t, *J* = 11.6 Hz, 1H); 1.80 (t, *J* = 10.8 Hz, 1H); 1.18 (d, *J* = 1.2 Hz, 3H); 1.17 (d, *J* = 0.8 Hz , 3H). MS (ESI) calcd for C₁₅H₂₁BrFNO₃ (m/z) : 361.07, 363.07, found: 362.1 [M+1]⁺, 363.1 [M+1]⁺

### Preparation of compound A0042

### a. Preparation of compound 10

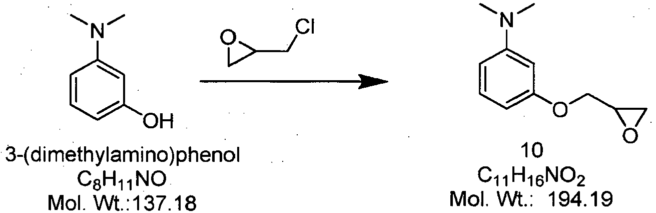

To a solution of NaOH (1.21 g, 30.29 mmol) in H₂O (1.5 ml) was added 3-(dimethylamino)phenol (500 mg, 3.65 mmol), (n-C₄H₉)₄N+HSO₄⁻ (50mg, 0.146 mmol) and 2-(chloromethyl)oxirane (1.35 g, 14.60 mmol) at 0°C. The reaction mixture was then stirred overnight (about 18 hours) at room temperature. After the reaction was complete, 10 ml of water was added to the reaction mixture, then extracted with ethyl acetate. The organic layers were combined, washed with brine, dried with anhydrous sodium sulfate, and concentrated to afford 320 mg of crude product. The crude product was then purified via column chromatography (petroleum ether:ethyl acetate=50:1 to petroleum ether:ethyl acetate=10:1) to afford 180 mg of the purified product (yield: 25.5%)

### b. Preparation of compound A0042

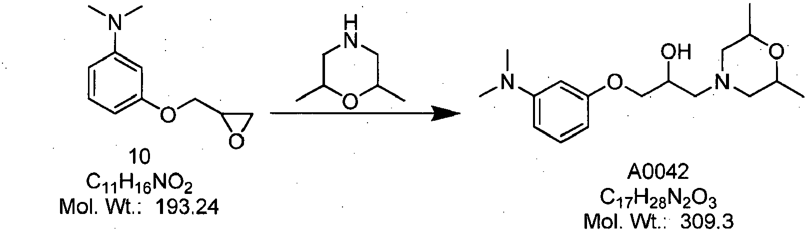

A mixture of **10** (160 mg, 0.83 mmol), 2,6-dimethylmorpholine (0.2 ml, 1.66 mmol) and H₂O (0.3 mL) was stirred overnight (about 18 hours) at room temperature. After the reaction was complete, the reaction mixture was then extracted with ethyl acetate, washed with water and brine, dried with anhydrous sodium sulfate, and concentrated under vacuum to afford 220 mg of crude product as yellow oil. The crude product was purified via column chromatography to obtain 100 mg of the purified product as yellow oil (yield: 39.2%, confirmed by ¹H NMR, purity 97.4% by HPLC).

¹H NMR (400 MHz, CDCl₃) : 7.13 (t, *J* = 8.4 Hz, 1H); 6.36 (d, *J* = 8.4 Hz, 1H); 6.30-6.27 (m, 2H); 4.15-4.11 (m, 1H); 4.10-3.94 (m, 2H); 3.75-3.65 (m, 2H); 2.93 (s, 6H); 2.85(d, *J* = 11.2 Hz, 1H); 2.71 (d, *J* = 11.2 Hz, 1H); 2.56-2.51 (m, 2H); 2.05 (t, *J* = 10.8 Hz, 1H); 1.80 (t, *J* = 11.2 Hz, 1H); 1.17 (s, 3H); 1.16 (s, 3H). MS (ESI) calcd for C₁₇H₂₈N₂O₃ (m/z) : 308.21, found: 309.3 [M+1]⁺.

### Preparation of compound A0043

### a. Preparation of compound 11

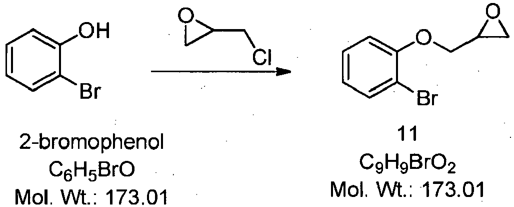

To 1.5 ml of NaOH (50%) was added 2-bromophenol (850 mg, 4.91 mmol), (n-C₄H₉)₄NHSO₄ (66 mg, 0.1965 mmol) and 2-(chloromethyl)oxirane (1.5 ml, 19.65 mmol) at 0°C. The reaction mixture was then stirred at room temperature overnight (about 18 hours). Next, 1N NaOH was added and the solution was extracted with dichloromethane, the organic layers were combined, dried with anhydrous Na₂SO₄, and concentrated under vacuum. The crude product was purified via column chromatography to obtain 400 mg of purified product as a colorless oil (yield:35.7%, confirmed by ¹H NMR).

### b. Preparation of compound A0043

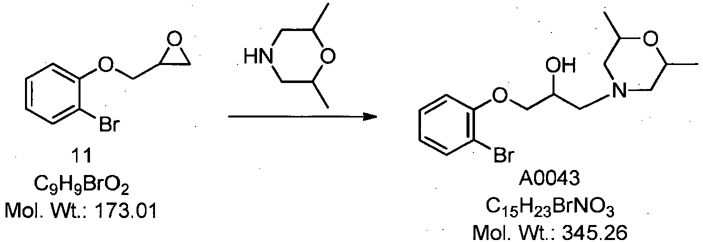

The mixture of **11** (228 mg, 1 mmol), 2,6-dimethylmorpholine (0.18 ml, 1.5 mmol) and H₂O (0.2 mL) was stirred overnight (about 18 hours) at room temperature. The reaction mixture was extracted with ethyl acetate and washed with water and brine, dried with anhydrous sodium sulfate, concentrated under vacuum to afford 350 mg of crude product as yellow oil. The crude product (150 mg) was purified by preparative thin-layer chromatography to obtain 80 mg of the title product as white solid (yield: 23%)

### Preparation of compound 0044

### a. Preparation of compound 12

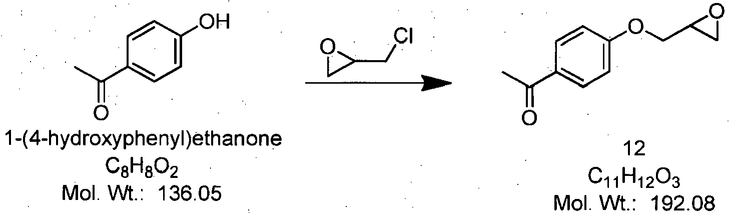

To a solution of 1-(4-hydroxyphenyl)-ethanone (500 mg, 3.67 mmol), (n-C₄H₉)₄N⁺HSO₄⁻ (50 mg, 0.147 mmol) and 2-(chloromethyl)oxirane (0.87 mL, 11 mmol) was added a solution of NaOH (1.2 g, 30.46 mmol) in H₂O (1.2 mL) at 0°C. The reaction mixture was then stirred overnight (about 18 hours) at room temperature. The reaction mixture was then poured into water (50 mL), extracted with dichloromethane, and the organic layers were combined, dried with anhydrous sodium sulfate, and concentrated under vacuum to afford 1 g of crude product as a red liquid. The crude product was purified by silica gel to obtain 350 mg of purified product as a colorless liquid (yield: 49%, confirmed by ¹H NMR).

### b. Preparation of compound A0044

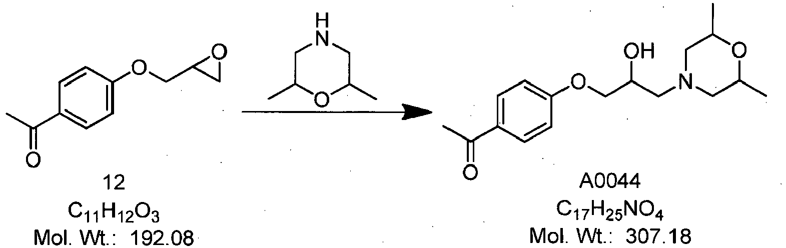

A mixture of 2, 6-dimethylmorpholine (0.44 mL, 3.64 mmol), compound **12** (350 mg, 1.82 mmol) and H₂O (0.6 mL) was stirred at room temperature overnight (about 18 hours). TLC suggested that the reaction was complete. The reaction mixture was then extracted with dichloromethane, washed with brine, dried with anhydrous sodium sulfate, and concentrated under vacuum to the crude product as a yellow liquid. The crude product was then purified by silica gel to obtain 200 mg of the purified product as a white solid and 130 mg of the isomer as yellow liquid (yield:35%, confirmed by ¹H NMR & MS, purity 98.4% by HPLC).

¹H NMR (400 MHz, CDCl₃) : 7.93 (dt, *J* = 8.4, 3.2 Hz, 1H); 6.96 (dt, *J* = 6.8, 2.8 Hz, 1H); 4.15-4.12 (m, 1H); 4.06-4.04 (m, 2H); 3.74-3.65 (m, 2H); 2.85(d, *J* = 11.2 Hz, 1H); 2.71 (dt, *J* = 10.8, 1.6 Hz, 1H); 2.57-2.47 (m, 5H); 2.07 (t, *J* = 10.4 Hz, 1H); 1.80 (t, *J* = 10.0 Hz, 1H); 1.18 (s, 3H); 1.16 (s, 3H). MS (ESI) calcd for C₁₇H₂₅NO₄ (m/z): 307.18, found: 308.2 [M+1]⁺.

### Preparation of compound A0045

### a. Preparation of compound 13

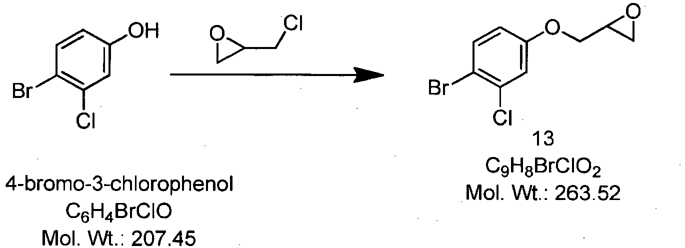

To a solution of 4-bromo-3-chlorophenol (500 mg, 2.41 mmol) , (n-C₄H₉)₄N⁺HSO₄⁻ (33 mg, 0.0964 mmol) and 2-(chloromethyl)oxirane (0.57 mL, 7.23 mmol) was added a solution of NaOH (0.8 g, 20 mmol) in H₂O (0.8 mL) at 0°C. The reaction mixture was then stirred at room temperature overnight (about 18 hours). The reaction mixture was then poured into water (50 mL), extracted with dichloromethane, and the organic layers were combined. The organic layers were dried with anhydrous sodium sulfate and concentrated under vacuum to afford 0.6 g of crude product as a yellow liquid. The crude product was then purified by silica gel to obtain 280 mg of the purified product as a colorless oil (yield: 44%, confirmed by ¹H NMR).

### b. Preparation of compound A0045

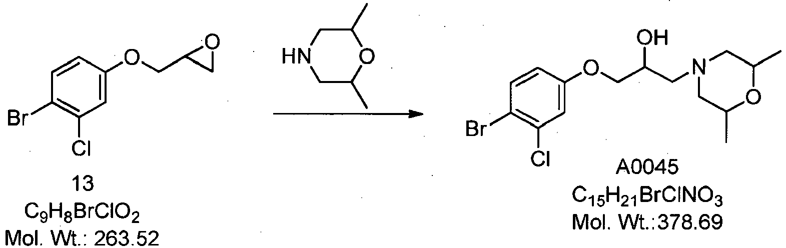

The mixture of 2,6-dimethylmorpholine (0.17 mL, 1.43 mmol), **13** (250 mg, 0.95 mmol) and H₂O (0.2 mL) was stirred overnight (about 18 hours) at room temperature. Thin-layer chromatography suggested the reaction complete. The reaction mixture was extracted with dichloromethane and washed with brine, dried with anhydrous sodium sulfate, concentrated under vacuum to the crude product as yellow liquid. The crude product was purified by silica gel to obtain 150 mg of the title product as white solid and 70 mg of the isomer as yellow liquid (41%, confirmed by ¹H NMR & MS, purity 98.2 % by HPLC).

### Preparation of compound A0046

### a. Preparation of compound 14

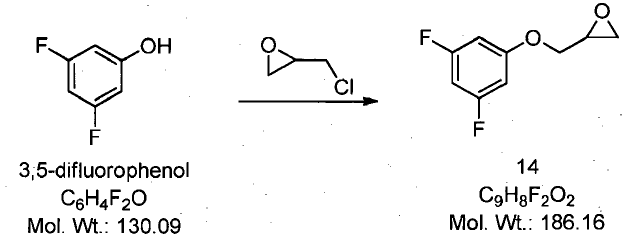

To 1.3ml of NaOH (50%) was added 3,5-difluorophenol (0.528 g, 4.0 mmol), (n-C₄H₉)₄N⁺HSO₄⁻ (54 mg, 0.16 mmol) and 2-(chloromethyl)oxirane (1.48 g, 16 mmol) at 0°C. The reaction mixture was stirred overnight (about 18 hours) at room temperature. Next, 1N NaOH was added and the solution was extracted with dichloromethane. The organic layers were combined, dried with anhydrous Na₂SO4, and concentrated under vacuum. The crude product was purified via column chromatography to obtain 300 mg of the purified product as colorless oil (yield: 40%, confirmed by ¹H NMR).

### b. Preparation of compound A0046

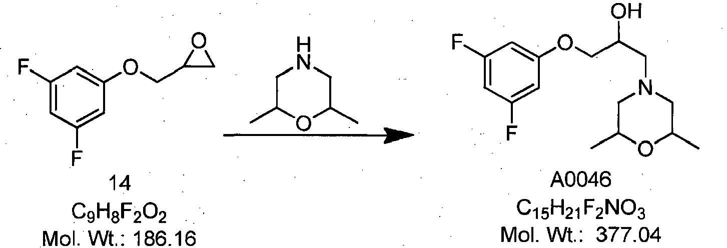

A mixture of **14** (156 mg, 0.83 mmol), 2,6-dimethylmorpholine (191 mg, 1.66 mmol) and H₂O (0.2 ,mL) was stirred overnight (about 18 hours) at room temperature. The reaction mixture was then extracted with ethyl acetate, washed with water and brine, dried with anhydrous sodium sulfate, and concentrated under vacuum. The crude product was purified via column chromatography to obtain the purified product (132 mg) as a colorless oil (yield:52.8%). The structure was confirmed by ¹H NMR & MS, purity 97.4% by HPLC.

¹H NMR (400 MHz, CDCl₃) : 6.46-6.42 (m, 3H); 4.12-4.07 (m, 1H); 3.98-3.91 (m, 2H); 3.74-3.64 (m, 2H); 2.83 (d, *J* = 10.8 Hz, 1H); 2.69 (d, *J* = 11.2 Hz, 1H); 2.56-2.44 (m, 2H); 2.06 (t, *J* = 11.2 Hz, 1H); 1.80 (t, *J* = 10.4 Hz, 1H); 1.18 (s, 3H); 1.16 (s, 3H). MS (ESI) calcd for C₁₅H₂₁F₂NO₃ (m/z): 377.04, 379.04, found: 377.9 [M+1]⁺, 379.9 [M+1]⁺.

### Preparation of compound A0047

### a. Preparation of compound 15

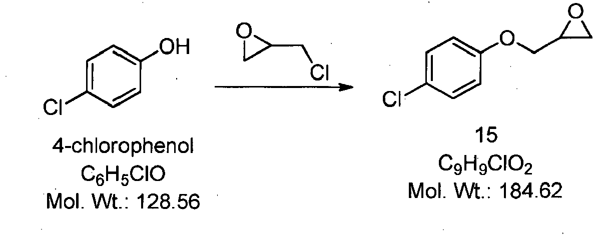

To 1.3 ml of NaOH (50%) was added 4-chlorophenol (500 mg, 3.9 mmol), (n-C₄H₉)₄N⁺HSO⁴⁻ (53 mg, 0.156 mmol) and 2-(chloromethyl)oxirane (902 mg, 9.75 mmol) at 0°C. The reaction mixture was then stirred at room temperature overnight (about 18 hours). Next, 1N NaOH was added and the solution was extracted with dichloromethane, the organic layers were combined, dried with anhydrous Na₂SO₄ and concentrated under vacuum. The crude product was purified via column chromatography to obtain 370 mg of the purified product as a yellow oil (yield: 51.5%).

### b. Preparation of compound A0047

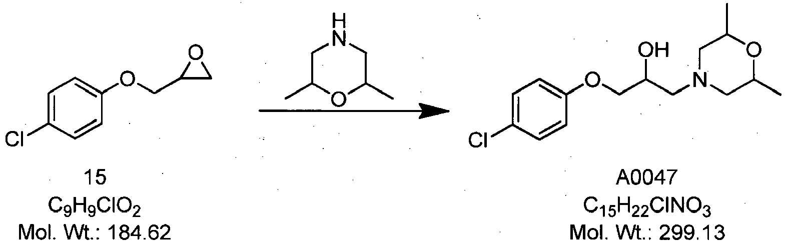

A mixture of compound **15** (332 mg, 1.8 mmol), 2,6-dimethylmorpholine (0.44 ml, 3.6 mmol) and H₂O (0.6 mL) was stirred at room temperature overnight (about 18 hours). After the reaction was complete, the reaction mixture was extracted with ethyl acetate, washed with water and brine, dried with anhydrous sodium sulfate and concentrated under vacuum to afford 520 mg of crude product as yellow oil (yield 96.6%). About 120 mg of the crude product was purified by preparative thin-layer chromatography to obtain 62 mg of the purified product (yield:51.6%, confirmed by ¹H NMR & MS, purity 98.4% by HPLC).

¹H NMR (400 MHz, CDCl₃): 7.23 (d, *J* = 7.2 Hz, 1H); 6.85 (dt, *J* = 7.2 Hz, 1H); 4.13-4.07 (m, 1H); 3.97-3.92 (m, 2H); 3.73-3.63 (m, 2H); 2.83(d, *J* = 12.0 Hz, 1H); 2.69 (d, *J* = 11.6 Hz, 1H); 2.57-2.45 (m, 2H); 2.06 (t, *J* = 11.2 Hz, 1H); 1.79 (t, *J* = 11.2 Hz, 1H) ; 1.18 (s, 3H); 1.16 (s, 3H). MS (ESI) calcd for C₁₅H₂₂ClNO₃ (m/z) : 299.13, found: 300.3 [M+1]⁺.

### Preparation of compound A0048

### a. Preparation of compound 16

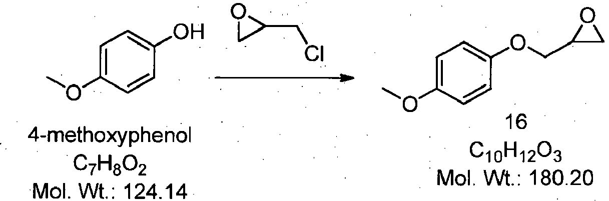

To 1.3 ml of NaOH (50%) was added 4-methoxyphenol (500 mg, 4.03 mmol), (n-C₄H₉)₄N⁺HSO₄⁻ (54 mg, 0.16 mmol) and 2-(chloromethyl)oxirane (1.26 ml, 16.13 mmol) in portions at 0 °C. The reaction mixture was stirred overnight (about 18 hours) at room temperature. Then, 1N NaOH was added and the solution was extracted with dichloromethane, combined the organic layers, dried with anhydrous Na₂SO₄, concentrated under vacuum. The residue was purified via column chromatography to obtain 555 mg of the desired product as yellow oil (yield: 76.5%).

### b. Preparation of compound A0048

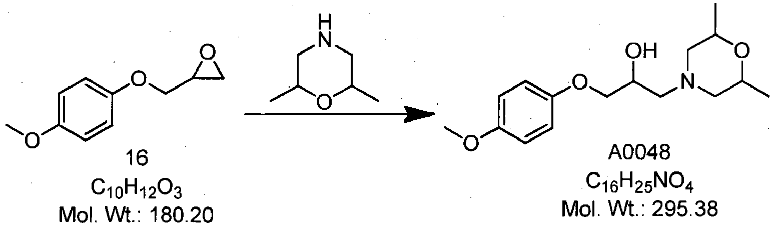

The mixture of **16** (200 mg, 1.1 mmol), 2,6-dimethylmorpholine (0.27 ml, 2.2 mmol) and H₂O (0.3 mL) was stirred overnight (about 18 hours) at room temperature. After the reaction was complete, the reaction mixture was extracted with dichloromethane and washed with water and brine, dried with anhydrous sodium sulfate, concentrated under vacuum to afford 324 mg of crude product as colorless oil (yield: 98.90). The crude product (120 mg) was purified by preparative thin-layer chromatography to obtain 48 mg of the title product (yield: 40%). The structure was confirmed by ¹H NMR & MS, purity 96.6 % by HPLC.

### Preparation of compound A0049

### a. Preparation of compound 17

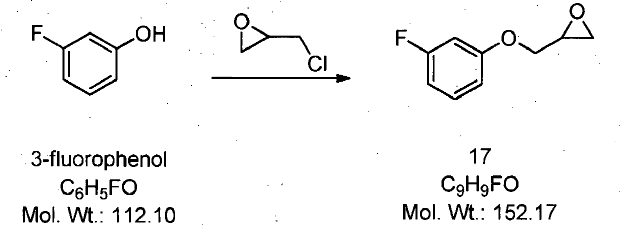

To a solution of NaOH (1.480 g, 37.01 mmol) in H₂O (2 ml) was added 3-fluorophenyl (500 mg, 4.46 mmol), (n-C₄H₉)₄N⁺HSO₄⁻ (60 mg, 0.178 mmol) and 2-(chloromethyl)oxirane (1.659 g, 17.85 mmol) 0°C. The reaction mixture was then stirred overnight (about 18 hours) at room temperature. When the reaction was deemed complete, 10 ml of water was added to the reaction mixture, which was then extracted with ethyl acetate. The organic layers were combined, washed with brine, dried with anhydrous sodium sulfate, and concentrated to afford 641 mg of crude product. The crude product was purified via column chromatography (petroleum ether/ethyl acetate=100/1 to petroleum ether/ethyl acetate=50/1) to afford 421 mg of purified product (yield:56.2%). The structure was confirmed by H NMR, shown as follows.

### b. Preparation of compound A0049

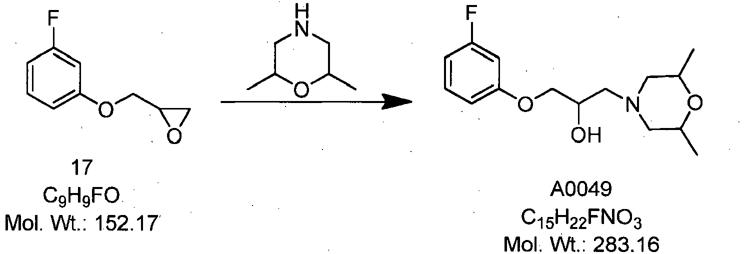

A mixture of 2,6-dimethylmorpholine (137 mg, 1.190 mmol), **17** (100 mg, 0.595 mmol) and H₂O (0.2 ml) was stirred overnight (about 18 hours) at room temperature. When the reaction was complete, the reaction mixture was extracted with ethyl acetate, washed with water and brine, dried with anhydrous sodium sulfate, and concentrated under vacuum to afford 220 mg of the crude product. The crude product was purified via column chromatography (petroleum ether:ethyl acetate=50:1 to petroleum ether:ethyl acetate=10:1) to afford 101 mg of purified product (yield:59.9%, confirmed by ¹H NMR, purity 96.8% by HPLC).

¹H NMR (400 MHz, CDCl₃): 7.25-7.19 (m, 1H); 6.72-6.62 (m, 3H); 4.15-4.09 (m, 1H); 3.97 (d, *J* = 4.8 Hz, 2H); 3.76- 3.64 (m, 2H); 2.85 (d, *J* = 11.6 Hz, 1H); 2.70 (d, *J* = 11.6 Hz, 1H); 2.59-2.47 (m, 2H); 2.07 (t, *J* = 10.6 Hz, 1H); 1.81 (t, *J* = 10.6 Hz, 1H) ; 1.18 (s, 3H); 1.17 (s, 3H); calcd for C₁₅H₂₂FNO₃ (m/z): 283.16. found: 284.2 [M+1]⁺.

### Preparation of compound A0050

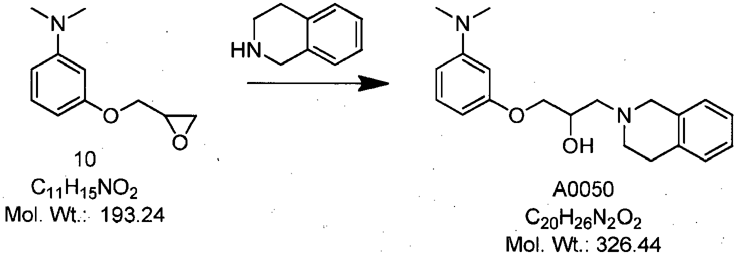

The mixture of 1,2,3,4-tetrahydro-isoquinoline (133 mg, 1.036 mmol), **10** (100 mg, 0.518 mmol) and H₂O (0.2 ml) was stirred overnight (about 18 hours) at room temperature. Once the reaction was complete, the reaction mixture was extracted with ethyl acetate and washed with water and brine, dried with anhydrous sodium sulfate, concentrated under vacuum to afford 280 mg of crude product. The crude product was purified via column chromatography (petroleum ether:ethyl acetate =20:1) and preparative thin-layer chromatography (ethyl acetate) to afford 50 mg of final product (yield: 30.0%; confirmed by ¹H NMR & MS, purity 97.9 % by HPLC).

### Preparation of compound 51

### a. Preparation of compound 18

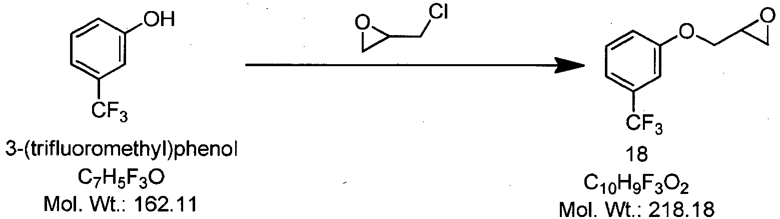

To 1.0 ml of NaOH (50%) was added 3-(trifluoromethyl) phenol (486 mg, 3.0 mmol), (n-C₄H₉)4N⁺HSO₄⁻ (40 mg, 0.12 mmol) and 2-(chloromethyl)oxirane (0.92 ml, 12 mmol) in portions at 0 °C. The reaction mixture was stirred overnight (about 18 hours) at room temperature. Then, 1N NaOH was added and the solution was extracted with dichloromethane, combined the organic layers, dried with anhydrous Na2SO₄, and concentrated under vacuum. The residue was purified via column chromatography to obtain 160 mg of final product as colorless oil (yield: 24.5%).

### b. Preparation of compound A0051

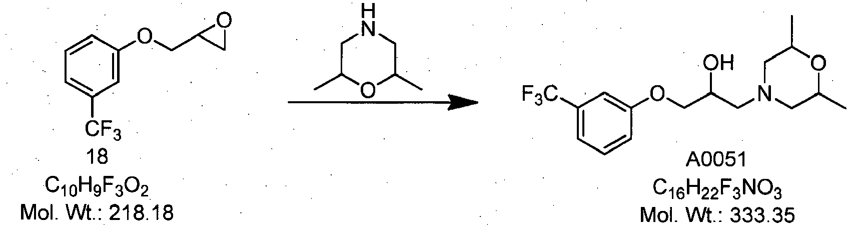

The mixture of **18** (160 mg, 0.73 mmol), 2,6-dimethylmorpholine (168 mg, 1.46 mmol) and H₂O (0.2 mL) was stirred overnight (about 18 hours) at room temperature. The reaction mixture was extracted with ethyl acetate and washed with water and brine, dried with anhydrous sodium sulfate, concentrated under vacuum and purified via column chromatography to obtain **A0051** as white solid (126 mg, yield: 52%). The product (70 mg) was purified by preparative thin-layer chromatography to give 30 mg of A0051 as white solid (yield: 22%). The structure was confirmed by ¹H NMR & MS, purity 96.6 % by HPLC.

### Preparation of compound A0053

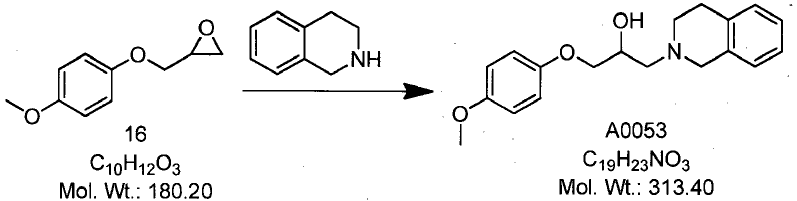

The mixture of **16** (100 mg, 0.56 mmol), 1,2,3,4-tetrahydroisoquinoline (148. mg, 1.1 mmol) and H₂O (0.2 mL) was stirred overnight at room temperature. The reaction mixture was extracted with dichloromethane and washed with water and brine, dried with anhydrous sodium sulfate, concentrated under vacuum to afford 180 mg of crude product as yellow oil. The crude product was purified by preparative thin-layer chromatography to obtain 141 mg of the target product (yield: 80.4%). The structure was confirmed by ¹H NMR & MS, purity 99.2 % by HPLC.

### Preparation of compound A0054

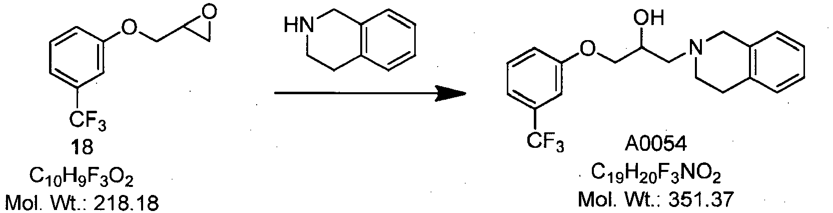

A mixture of 1,2,3,4-tetrahydroisoquinoline (426 mg, 3.2 mmol), **18** (350 mg, 1.6 mmol) and H₂O (0.3 mL) was stirred overnight (about 18 hours) at room temperature. Once the reaction was complete, the reaction mixture was extracted with dichloromethane and washed with brine, dried with anhydrous sodium sulfate, concentrated under vacuum to the crude product as yellow liquid. The crude product was purified by silica gel to obtain 400 mg of the title product as yellow liquid. (Yield: 71%) 120 mg of the crude product was purified by preparative thin-layer chromatography to obtain the title product as colorless liquid (40 mg, yield: 23%). The structure was confirmed by ¹H NMR & MS, purity 97.0 % by HPLC.

### Preparation of compound A0055

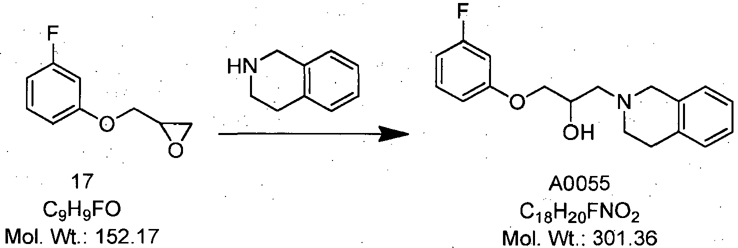

A mixture of 1,2,3,4-tetrahydroisoquinoline (158 mg, 1.190 mmol), **17** (100 mg, 0.595 mmol) and H₂O (0.2 ml) was stirred overnight (about 18 hours) at room temperature. Thin-layer chromatography suggested the reaction complete. The reaction mixture was extracted with ethyl acetate and washed with water and brine, dried with anhydrous sodium sulfate, concentrated under vacuum to afford 286 mg of crude product. The crude product was purified via column chromatography (petroleum ether/ethyl acetate=5/1 and preparative thin-layer chromatography ethyl acetate to afford 51 mg of final product (yield: 25.8%; confirmed by ¹H NMR & MS, purity 98.7% by HPLC).

### Preparation of compound A0056

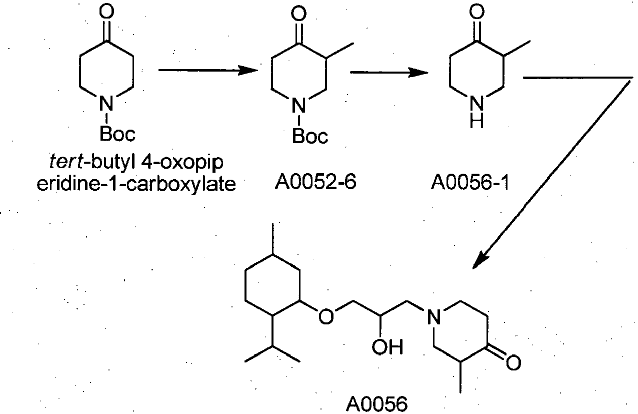

### a) Preparation of compound A0052-6

NaH (634 mg, 15.85 mmol) was added to a solution of tert-butyl 4-oxopiperidine-1-carboxylate (3 g, 15.1 mmol) in 40 mL of tetrahydrofuran at room temperature. The solution was stirred at room temperature for 30 minutes. 1.9 mL of iodomethane was added slowly. The mixture was stirred at room temperature overnight (about 18 hours). Water was added and extracted with ethyl acetate. The combined organic layer was washed with water and brine, dried with anhydrous sodium sulfate, concentrated under vacuum to afford 3.5 g of crude product as yellow oil. The crude product was purified by chromatography eluted with petroleum ether:ethyl acetate=50:1 to 10:1 to obtain 320 mg of the target product as colorless oil (yield: 10%). The structure was confirmed by ¹H NMR

### b. Preparation of compound A0056-1

To the solution of **A0052-6** (300 mg, 1.41 mmol) in 3 ml dichloromethane was added 0.6 mL trifluoroacetic acid and the mixture was kept stirring for 1 hour at room temperature. Then the mixture was added 30 mL of NaOH (20%, aq) and extracted with dichloromethane. The combined organic layer was washed with brine, dried over anhydrous sodium sulfate and concentrated to obtain 122 mg of the crude product as yellow oil. The crude product was used for the next step without any further purification (yield: 75%). The structure was confirmed by ¹H NMR.

### c. Preparation of compound A0056

The mixture of compound **A0056-1** (115 mg, 1.02 mmol), compound **1** (323 mg, 1.53 mmol) and H₂O (0.3 mL) was stirred overnight (about 18 hours) at room temperature. Thin-layer chromatography was used to monitor the reactions progress. The reaction mixture was quenched by extraction with ethyl acetate, followed by a washing with water and brine. The quenched reaction mixture was dried with anhydrous sodium sulfate and concentrated under vacuum to afford 300 mg of crude product as yellow oil. The crude product was purified via column chromatography to obtain 20 mg of the title product as colorless oil (yield: 6%). The structure was confirmed by ¹H NMR and MS.

### Preparation of compound A0057

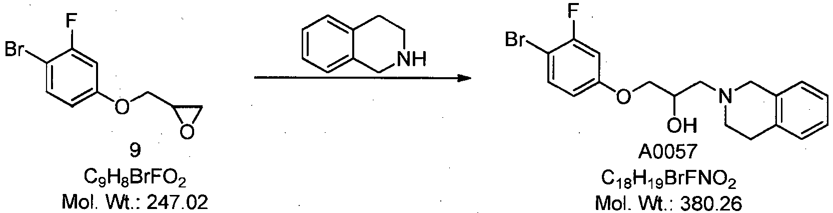

A mixture of compound **9** (100 mg, 0.4 mmol), 1,2,3,4-tetrahydroisoquinoline (107 mg, 0.8 mmol) and H₂O (0.2 mL) was stirred at room temperature overnight (about 18 hours). The reaction mixture was extracted with dichloromethane and washed with water and brine, dried with anhydrous sodium sulfate, concentrated under vacuum to afford the crude product. The crude product was purified by preparative thin-layer chromatography to obtain 75 mg of the title product (yield: 48.7%). The structure was confirmed by ¹H NMR and the purity was 98.8% by HPLC.

¹H NMR (400 MHz, CDCl₃): 7.41 (dd, *J* = 8.0, 8.8 Hz, 1H); 7.17-7.11 (m, 3H); 7.04-7.02 (m, 1H); 6.74 (dd, *J* = 2.4, 10.4 Hz, 1H); 6.64-6.67 (m, 1H); 4.17-4.21 (m, 1H); 4.02-3.99 (m, 2H); 3.87 (d, *J* = 14.4 Hz, 1H); 3.67 (d, *J* = 14.4 Hz, 1H); 2.99-2.91 (m, 3H); 2.81-2.67 (m, 3H); calcd for C₁₈H₁₉BrFNO₂ (m/z): 380.25. found: 380.1 [M+1]⁺.

### Preparation of compound A0058

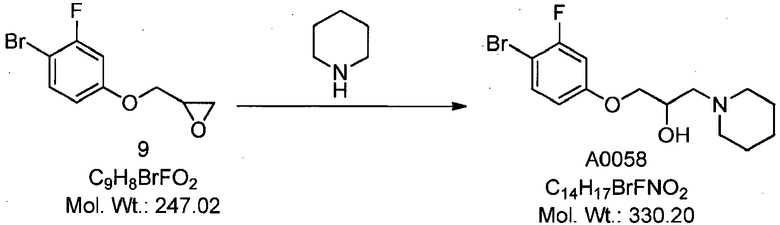

A mixture of **9** (90 mg, 0.36 mmol), piperidine (62 mg, 0.73 mmol) and H₂O (0.2 mL) was stirred at room temperature overnight (about 18 hours). The reaction mixture was extracted with dichloromethane and washed with water and brine, dried with anhydrous sodium sulfate, concentrated under vacuum to afford the crude product. The crude product was purified by preparative thin-layer chromatography to obtain 88 mg of the title product (yield: 73.6%). The structure was confirmed by ¹H NMR and the purity was 98.8% by HPLC.

¹H NMR (400 MHz, CDCl₃): 7.39 (dd, *J* = 8.0, 8.8 Hz, 1H); 6.72 (dd, *J* = 3.2, 10.8 Hz, 1H); 6.65-6.61 (m, 1H); 4.06-4.03 (m, 1H); 3.93-3.92 (m, 2H); 2.61 (m, 2H); 2.46-2.42 (m, 2H); 2.36 (s, 2H); 1.62-1.55 (m, 4H); 1.48-1.44 (m, 2H) ; calcd for C₁₄H₁₉BrFNO₂ (m/z): 332.21. found: 332.1 [M+1]⁺.

### Preparation of compound A0059

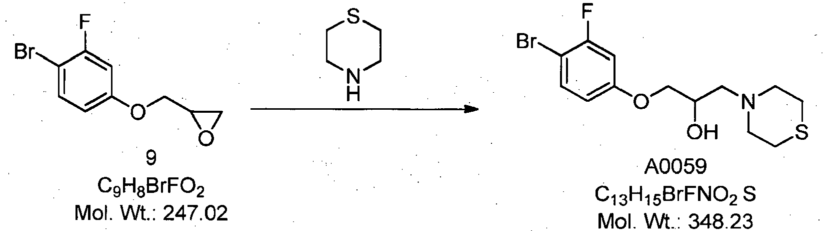

A mixture of **9** (90 mg, 0.36 mmol)), thiomorpholine (75 mg, 0.73 mmol) and H₂O (0.2 mL) was stirred overnight (about 18 hours) at room temperature. Thin-layer chromatography was used to monitor the reaction's progress. The precipitate that formed was collected by filtration and washed with H₂O to obtain 96 mg of the title product (yield: 76.1%).

¹H NMR (400 MHz, CDCl₃): 7.40 (dd, *J* = 8.4, 8.8 Hz, 1H); 6.72 (dd, *J* = 2.8, 10.0 Hz, 1H); 6.63 (dd, *J* = 2.8, 8.8 Hz, 1H); 4.07-4.03 (m, 1H); 3.94-3.90 (m, 2H); 3.46 (s, 1H); 2.96-2.91 (m, 2H); 2.76-2.67 (m, 6H); 2.59 (dd, *J* = 4.0, 12.8 Hz, 1H); 2.49 (dd, *J* = 9.6, 12.8 Hz, 1H) ; calcd for C₁₃H₁₇BrFNO₂S (m/z): 350.25. found: 352.2 [M+1]⁺.

### Preparation of compound A0060

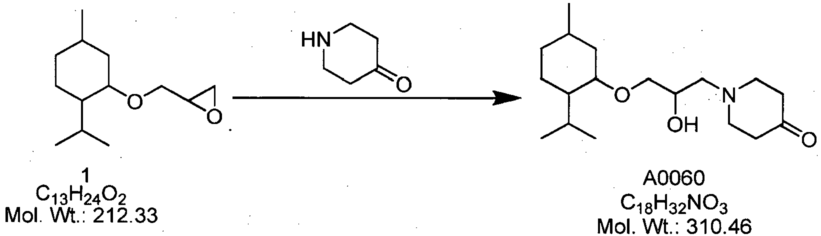

A mixture of compound **1** (200 mg, 0.943 mmol) and piperidin-4-one (112 mg, 1.132 mmol) in H₂O (0.5 ml) was stirred 3 days. The mixture was dissolved in ethyl acetate and washed with brine, dried and concentrated under vacuum to afford 259 mg of crude product. The mixture was dissolved in ethyl acetate and was washed with brine, dried, and concentrated under vacuum to afford 259 mg of crude product. The crude product was purified via column chromatography (dichloromethane/methanol=200/1-dichloromethane/methanol=50/1) to afford 80 mg of final product (yield: 27 %). The structure was confirmed by ¹H NMR & LC-MS. Purity 100% by HPLC.

¹H NMR (400 MHz, CDCl₃): 3.92-3.86 (m, 1H); 3.72-3.62 (m, 1H); 3.36-3.30 (m, 1H); 3.07 (td, *J* = 10.8, 4.0 Hz, 1H); 2.97-2.90 (m, 2H); 2.81-2.75 (m, 2H); 2.64-2.41 (m, 6H); 2.22-2.14(m, 1H); 2.12-2.07 (m, 1H); 1.67-1.58 (m, 2H); 1.36-1.20 (m, 3H); 1.02-0.77 (m, 13H). MS (ESI) calcd for C₁₈H₃₃NO₃ (m/z): 311.46. found: 312.4 [M+1]⁺.

### Preparation of compound A0061

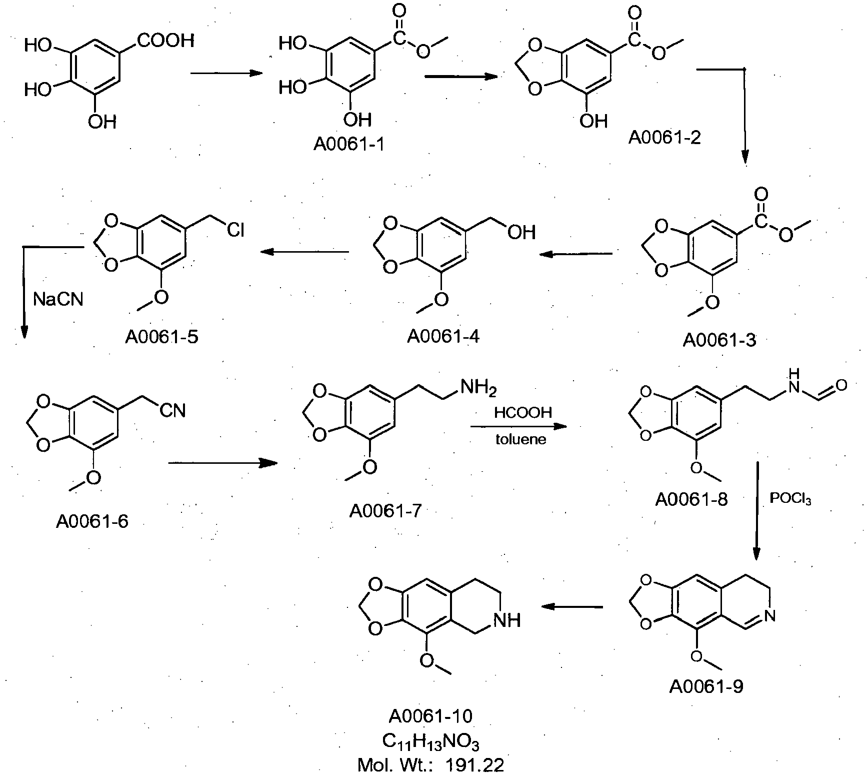

### a. Preparation of compound A0061-10

### i. Synthesis of compound A0061-1

To a solution of 3,4,5-trihydroxybenzoic acid (3 g, 17.6 mmol) in methanol (30 ml) was added concentrated sulfuric acid (0.9 ml) and the mixture was stirred under reflux for 1.5 hours. The reaction vessel was cooled to room temperature and the reaction mixture was neutralized with saturated sodium bicarbonate solution at 0 °C. The organic solvent was removed under reduced pressure. The concentrated residue was dissolved in ethyl acetate, washed with saturated sodium bicarbonate solution and brine, dried over anhydrous Na₂SO₄, concentrated and dried under vacuum to give compound A0061-1. (2.424 g, yield: 74.8%) as a white solid.

### ii. Synthesis of compound A0061-2

To a solution of compound **A0061-1** (1 g, 5.43 mmol) in dimethyl sulfoxide (DMSO) (25 ml) was added potassium hydrogen carbonate (KHCO₃) (0.54 g, 5.43 mmol) followed by dibromomethane (0.4 ml)) and the mixture was heated at 60 °C for 1.5 hours under nitrogen. The reaction was cooled and poured into water (50 ml). The mixture was extracted with ether. The organic layers were combined, dried over anhydrous Na₂SO₄ and concentrated to yield a crude oil which was further purified by column chromatography on silica gel (petroleum ether:ethyl acetate=5:1) to yield compound **A0061-2.** (580 mg, yield: 55%, NMR confirmed) as a white solid.

### iii. Synthesis of compound A0061-3

To a suspension of potassium carbonate (K₂CO₃) (211 mg, 1.53mmol) in dimethylsulfoxide (DMSO) (5 mL) was added a solution of compound **A0061-2** (200 mg, 1.02 mmol) in DMSO(5 mL) and the mixture was stirred at room temperature for 30 minutes. Methyl iodide (217 mg, 1.53 mmol) was added, and the reaction mixture was stirred for another 4 hours. Methyl iodide was removed under reduced pressure and the residue obtained was extracted with ethyl acetate. The organic layer was dried over anhydrous sodium sulfate and concentrated under reduced pressure to yield compound **A0061-3.** (180mg, yield: 84%, ¹H NMR confirmed) as a yellow solid.

### iv. Synthesis of compound A0061-4

Compound **A0061-3** (56 mg, 0.27 mmol) was added dropwise to a suspension of lithium aluminum hydride (LiAlH₄) (40 mg, 1.08 mmol ) in THF (5 mL) at 0 °C. The reaction mixture was stirred at 0 °C for 30 minutes, followed by stirring at room temperature for 1 hour. The reaction was quenched with cold water (10 ml) and extracted with ethyl acetate. The organic layer was dried over anhydrous Na₂SO₄ and concentrated under reduced pressure to yield compound **A0061-4.** (48 mg, yield: 97.7%, ¹H NMR confirmed) as yellow oil.

### v. Synthesis of compound A0061-5

To a mixture of compound **A0061-4** (1.15 g, 5.5 mmol) in THF (20 ml) was added thionyl chloride (0.8 ml) and the reaction mixture was stirred at room temperature for 3.5 hours. The reaction was quenched with water and extracted with ethyl acetate. The organic layer was dried over Na₂SO₄ and concentrated under reduced pressure to give compound **A0061-5.** (1.056 g, yield: 95.5%, ¹H NMR confirmed) as a yellow solid.

### vi. Synthesis of compound A0061-6

A solution of compound **A0061-5** (1.056 g, 5.25 mmol) and sodium cyanide (NaCN) (0.52 g, 10.5 mmol) in dimethylformamide (DMF) (25 mL) was stirred at 100 °C for 4 hours whereupon the color of the reaction mixture changed from yellow to black. The reaction was diluted with water and extracted with ethyl acetate three times. The combined layers of ethyl acetate were washed with water, dried over anhydrous Na₂SO₄ and concentrated under reduced pressure to give compound **A0061-6.** (630 mg, yield: 62.8%, ¹H NMR confirmed) as a brown solid.

### vii. Synthesis of compound A0061-7

To a mixture of ethanol (EtOH) (20 mL), water (8 mL) and 1N HCl (2 mL) was added PtO₂ (200 mg) and compound **A0061-6** (630 mg, 3.56 mmol) and the reaction mixture was hydrogenated overnight (about 18 hours) under 40 psi of H₂ at room temperature. Thin-layer chromatography showed the reaction was complete. The solution was concentrated under reduced pressure. To the residue was added water and 1M NaOH until the solution reached pH=13∼14. The resulting mixture was extracted with CH₂Cl₂. The organic layer was dried over Na₂SO₄ and concentrated to give compound **A0061-7.** (670mg, yield: 96.5%, ¹H NMR confirmed) as brown oil.

### viii. Synthesis of compound A0061-8

A mixture of compound **A0061-7** (670 mg, 3.44 mmol) and formic acid (0.62 mL) in toluene (20 mL) was refluxed for 4 hours following which the reaction was partitioned between water and toluene and the aqueous layer was extracted with toluene three times. The combined organic layers were washed with water and brine and concentrated to obtain compound **A0061-8.** (539 mg, yield: 70.3%, ¹H NMR confirmed) as a brown solid.

### ix. Synthesis of compound A0061-9

To a solution of compound **A0061-8** (500 mg, 2.24 mmol) in CH₂Cl₂ (10 ml) was added POCl₃ (0.5 ml) and the reaction mixture was refluxed at 75 °C for 3 hours. The reaction mixture was concentrated under reduced pressure and to the residue was added water (20 ml), toluene (20 ml) and 20% NaOH (5 ml). The mixture was stirred at 100 °C for 1 hour and cooled. The layers were separated and the aqueous layer was extracted with ethyl acetate. The combined organic layers were washed with water and brine and concentrated to obtain the crude product (394 mg) which was further purified by column chromatography over silica gel to obtain compound **A0061-9.** (320 mg, yield: 69.7%, ¹H NMR confirmed) as a white solid.

### x. Synthesis of compound A0061-10

To a solution of compound **A0061-9** (2.16 g, 10.5 mmol) in methanol (45 ml) was added NaBH₄ (2.0 g, 52.7 mmol) at 0 °C. The reaction mixture was stirred overnight (about 18 hours) at room temperature. TLC suggested the reaction complete. The reaction mixture was concentrated to remove the solvent and the residue was dissolved in ethyl acetate and washed with H₂O and brine, dried, concentrated under vacuum to afford 2.1g of crude product. The crude product was dissolved in methanol (5 ml) and to the mixture methanol/hydrochloric acid was added until pH 1∼2. The reaction mixture was concentrated to remove the solvent, diethyl ether was added to the residue and a white solid appeared. The solid was filtered and was washed with diethyl ether three times and dried to afford 1.708 g of final product (yield: 67 %).

### b. Synthesis of compound A0061

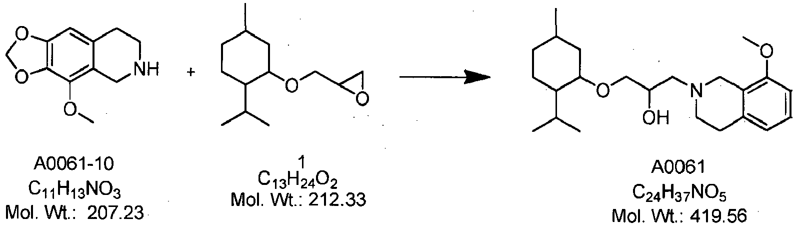

A mixture of **1** (171 mg, 0.805 mmol) and **19** (200 mg, 0.966 mmol) in H₂O (0.4 ml) was stirred overnight (about 18 hours). Thin-layer chromatography suggested the reaction complete. The mixture was dissolved in ethyl acetate and was washed with brine, dried, and concentrated under vacuum to afford 405 mg of crude product. The crude product was purified via column chromatography (dichloromethane : methanol = 200:1- dichloromethane : methanol=100:1) to afford 60 mg of final product (yield: 15%) and 227 mg of mixture product. The structure was confirmed by ¹H NMR & LC-MS. Purity 95.8% by HPLC.

### Preparation of compound A0062

### a. Preparation of compound 20

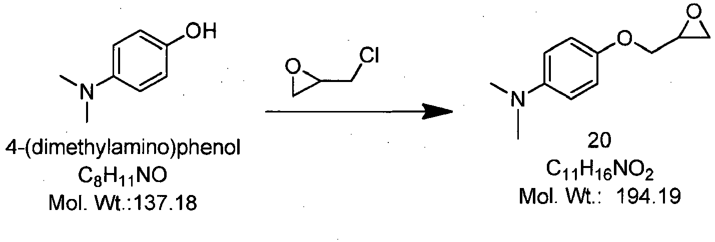

To 1.2ml of NaOH (50%) was added 4-(dimethylamino)phenol (500 mg, 3.65 mmol), (n-C₄H₉)₄N⁺HSO₄⁻ (50 mg, 0.14 mmol) and 2-(chloromethyl)oxirane (1.14 ml, 1.4.6 mmol) in portions at 0 °C to form a reaction mixture. The reaction mixture was stirred overnight (about 18 hours) at room temperature. Then, 1N NaOH was added and the solution was extracted with dichloromethane, combined the organic layers, dried with anhydrous Na₂SO₄, concentrated under vacuum: The residue was purified via column chromatography to obtain 210 mg of final product as yellow oil (yield: 29.8%).

### b. Preparation of compound A0062

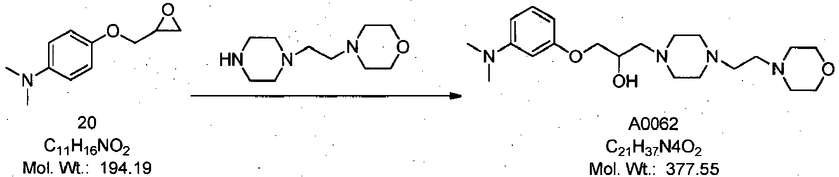

A mixture of compound **20** (100 mg, 0.52 mmol), 1-(2-morpholinoethyl)piperazine (206 mg, 1.04 mmol) and H₂O (0.2 mL) was stirred overnight (about 18 hours) at room temperature. Thin-layer chromatography showed the starting materials remained. The reaction mixture was diluted with dichloromethane and washed with water and brine, dried with anhydrous sodium sulfate, concentrated under vacuum to afford the crude product as yellow oil. The crude product was purified by preparative thin-layer chromatography to obtain 5 mg of the title product (yield: 2.5%).

### Preparation of compound A0063

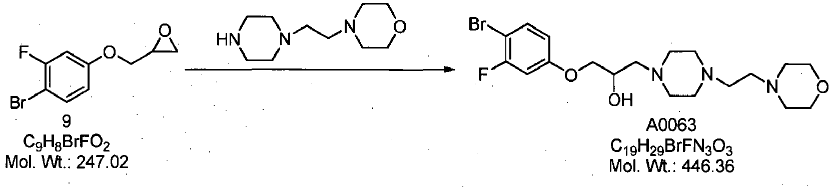

A mixture of compound **9** (100 mg, 0.4 mmol), 1-(2-morpholinoethyl)piperazine (159 mg, 0.8 mmol) and H₂O (0.2 mL) was stirred overnight at room temperature (about 18 hours). Thin-layer chromatography was to monitor the reaction's progress. Once the reaction was deemed complete, the reaction mixture was diluted with dichloromethane and washed with water and brine, dried with anhydrous sodium sulfate, and concentrated under vacuum to afford the crude product as yellow oil. The crude product was purified by preparative thin-layer chromatography to obtain 26 mg of the title product (yield: 14.6%, confirmed by ¹H NMR & LC-MS. Purity 98.7% by HPLC).

¹H NMR (400 MHz, CDCl₃): 7.38 (t, *J* = 8.0 Hz, 1H); 6.72 (d, *J* = 10.0 Hz, 1H); 6.63 (d, *J* = 9.2 Hz, 1H); 4.08-4.06 (m, 1H); 3.94-3.93 (m, 2H); 3.71 (t, *J* = 4.0 Hz, 4H); 2.72-2.70 (m, 2H); 2.58-2.44 (m, 16H); calcd for C₁₉H₂₉BrFN₃O₃ (m/z) : 446.35. found: 446.2 [M+1]⁺.

### Preparation of compound A0065

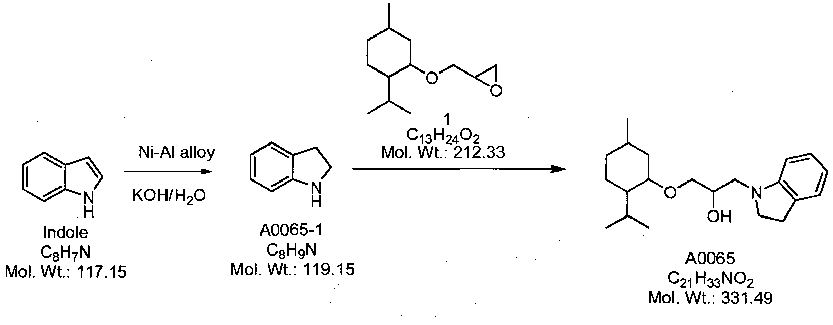

### a. Synthesis of compound A0065-1

To a solution of indole (1.0 g, 8.5 mmol) in absolute ethanol (20 ml), was added Raney Nickel (100 mg). The mixture was stirred at room temperature under 10 atm H₂ overnight (about 18 hours). Then the mixture was filtered and washed with ethanol. The filtrate was concentrated and purified by silica gel to obtain the title product as colorless liquid (824 mg, Yield: 81%).

### b. Synthesis of compound A0065

A mixture of compound **1** (148 mg, 0.7 mmol), **A0065-1** (125 mg, 1.05 mmol) and H₂O (0.2 mL) was stirred overnight (about 18 hours) at room temperature. The reaction mixture was extracted with ethyl acetate and washed with water and brine, dried with anhydrous sodium sulfate, and concentrated under vacuum to afford the crude product. The crude product was purified by silica gel to obtain the title product (102 mg, yield: 44%). The structure was confirmed by ¹H NMR and the purity was 98.6% by HPLC, shown as follows.

¹H NMR (400 MHz, CDCl₃): 7.10-7.08 (m, 2H); 6.70-6.69 (m, 1H); 6.55-6.53 (m, 1H); 4.02 (m, 1H); 3.80-3.68 (m, 1H); 3.53-3.33 (m, 3H); 3.25-3.08 (m, 3H); 3.00 (t, J = 8.4 Hz, 2H); 2.61 (t, *J* = 3.6Hz, 1H); 2.25-2.11 (m, 2H); 1.66-1.55 (m, 2H); 1.37-1.30 (m, 1H); 1.03-0.77 (m, 13H). LCMS (ESI) calcd for C₂₁H₃₃NO₂ (m/z) : 331.49. found: 332.6 [M+1]⁺.

### Preparation of compound A0066

### a. Synthesis of compound A0066-1

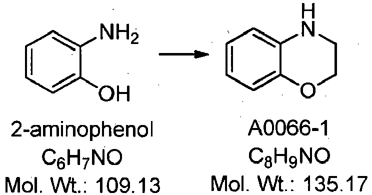

1,2-Dibromoethane (1.51 g,13.87 mmol) was dissolved in 100 mL of acetone and a solution of 3.19 g of K₂CO₃ in 30 mL of water was added. A solution of 2-aminophenol in 10 mL of acetone suspension was added slowly to the suspension. The solution was heated to reflux for 24 hours. The acetone was evaporated to dryness. The residue was dissolved in water and extracted with dichloromethane. The combined organic layer was washed with brine, dried over Na₂SO₄ and evaporated to dryness. The residue was purified by chromatography eluted with petroleum ether:ether acetate=50:1 to 10:1 to obtain 450 mg of the target product as brown oil (yield: 29%,).

### b. Synthesis of compound A0066

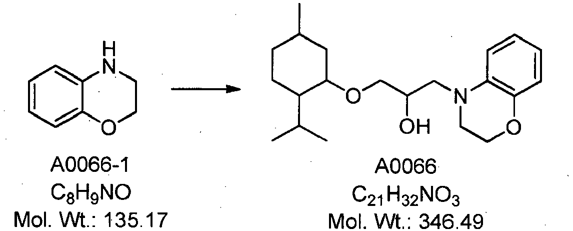

A mixture of **A0066-1** (135 mg, 1 mmol), **1** (254 mg, 1.2 mmol) and H₂O (0.3 mL) was stirred overnight (about 18 hours) at room temperature. Thin-layer chromatography showed no reaction. The mixture was stirred at 50°C for 1 day. The reaction mixture was extracted with ethyl acetate and washed with water and brine, dried with anhydrous sodium sulfate, concentrated under vacuum to afford 300 mg of crude product as yellow oil. The crude product was purified by chromatography eluted with petroleum ether : ethyl acetate=10:1 to obtain **A0066** as yellow oil (25 mg, yield: 7.2%). ¹H-NMR

¹H NMR (400 MHz, CDCl₃): 6.84 (d, *J* = 7.6 Hz, 1H); 6.81 (d, *J* = 7.2 Hz, 1H); 6.73 (t, *J* = 8.4 Hz, 1H); 6.64 (t, *J* = 8.0 Hz, 1H); 4.23 (t, *J* = 4.3 Hz, 2H);4.05 (s, 1H); 3.71 (m, 1H), 3.51 - 3.25 (m, 5H); 3.11 (m, 1H); 2.47 (s, 1H); 2.19 (m, 1H); 2.11 (d, *J* = 12.0 Hz, 1H); 1.68-1.61 (m, 3H), 1.29 (m, 3H); 1.02 - 0.78 (m, 13H); MS (ESI) calcd for C₂₁H₃₃NO₃ (m/z): 347.49. found: 348.4 [M+1]⁺.

### Preparation of compound A0068

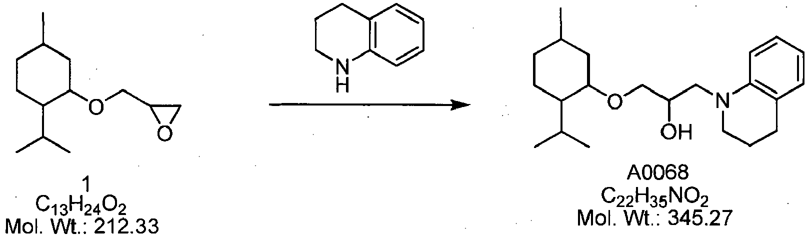

A mixture of compound **1** (500 mg, 2.35 mmol) and 1,2,3,4-tetrahydroquinoline (470 mg, 3.54 mmol) in H₂O (0.6 ml) was stirred for 2 days. The mixture was dissolved in ethyl acetate and dried with Na₂SO₄, and concentrated under vacuum to afford 950 mg of crude product. The crude product (200 mg) was purified via preparative thin-layer chromatography to obtain the product (20 mg, Yield: 12%). The product (750 mg) was further purified via column chromatography (petroleum ether:ethyl acetate=100:1-petroleum ether:ethyl acetate=10:1) to obtain the final product. (450 mg, Yield: 56 %) ¹H-NMR

¹H NMR (400 MHz, CDCl₃): 7.05 (t, *J* = 7.6 Hz, 1H); 6.96 (d, *J* = 7.6 Hz, 1H); 6.67 (t, *J* = 8.4 Hz, 1H); 6.60 (t, *J* = 7.4 Hz, 1H); 4.05 (m, 1H); 3.71 (m, 2H); 3.41 - 3.28 (m, 6H); 3.10 (m, 1H); 2.78 (t, *J* = 6.2 Hz, 2H); 2.43 (m, 1H); 2.26 - 2.07 (m, 2H); 1.99 - 1.91 (m, 2H); 1.65 (m, 2H); 0.96 - 0.77 (m, 13H); MS (ESI) calcd for C₂₂H₃₅NO₂ (m/z): 345.52. found: 346.3 [M+1]⁺.

### Preparation of compound A0069

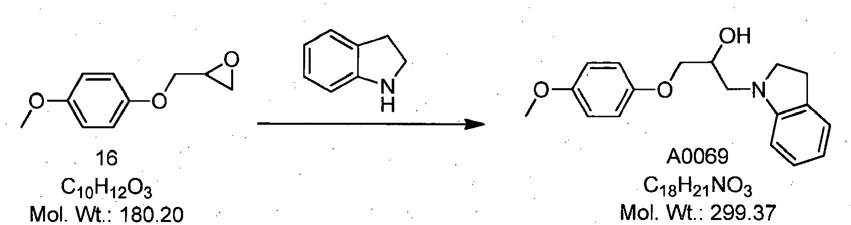

The mixture **16** (144 mg, 0.8 mmol), indoline (143 mg, 1.2 mmol ) and H₂O (0.2 mL) was stirred overnight (about 18 hours) at room temperature. The reaction mixture was extracted with ethyl acetate and the organic layer was washed with water and brine, dried with anhydrous sodium sulfate, concentrated under vacuum to afford the crude product. The crude product was purified by silica gel to obtain the title product (172 mg, yield: 72%). The structure was confirmed by ¹H NMR and the purity was 99.1% by HPLC.

¹H NMR (400 MHz, CDCl₃): 7.11 (d, *J* = 7.2 Hz, 1H), 7.08 (t, *J* = 7.6 Hz, 1H), 6.92-6.83 (m, 4H); 6.70 (t, *J* = 7.6 Hz, 1H); 6.57 (d, *J* = 7.2 Hz, 1H); 4.25 (m, 1H); 4.08-4.00 (m, 2H); 3.79 (s, 3H); 3.52 (dd, *J* = 16.8, 8.8 Hz, 1H); 3.41 (dd, *J* = 18.0, 8.8 Hz, 1H); 3.35 (dd, *J* = 18.0, 7.2 Hz, 1H); 3.23 (dd, J = 13.2, 4.8 Hz, 1H); 3.01 (t, *J* = 8.4 Hz, 2H); 2.67 (s, 1H) ; LCMS (ESI) calcd for C₁₈H₂₁NO₃ (m/z): 299.36. found: 300.3 [M+1]⁺.

### Preparation of compound A0072

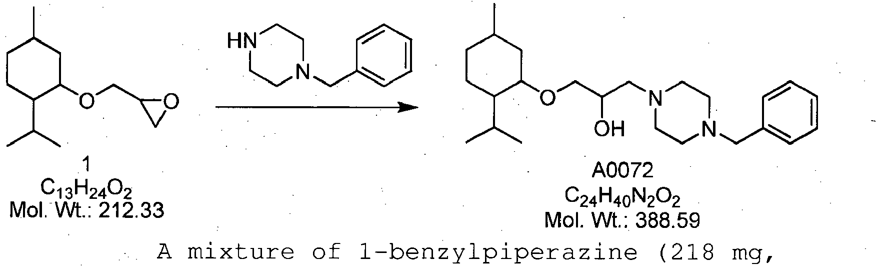

1.2 mmol), compound **1** (212 mg, 1 mmol) and H₂O (0.2 mL) was stirred overnight (about 18 hours) at room temperature. After the reaction was complete, the reaction mixture was extracted with dichloromethane and washed with brine, dried with anhydrous sodium sulfate, concentrated under vacuum to obtain the crude product as yellow liquid. The crude product was purified by chromatograph to obtain target product as yellow liquid (240 mg, yield: 61%). The structure was confirmed by ¹H NMR & LC-MS. Purity: 98.2% by HPLC.

¹H NMR (400 MHz, CDCl₃) : 7.32 (m, 4H);7.27-7.25 (m, 1H); 3.92-3.78 (m, 1H); 3.63 (td, *J* = 9.2, 5.2 Hz, 1H); 3.51 (s, 2H); 3.35-3.25 (m, 1H); 3.05 (td, J = 10.4, 4.0 Hz , 1H); 2.66 (s, 2H); 2.55-2.34 (m, 7H); 2.26-2.14 (m, 1H); 2.08 (m, 1H); 1.64 (m, 2H); 1.28 (m, 3H); 1.05-0.67 (m, 13H); MS(ESI) calcd for C₂₄H₄₀N₂O₂ (m/z): 388.59. found: 389.3 [M+1]⁺.

### Preparation of compound A0073

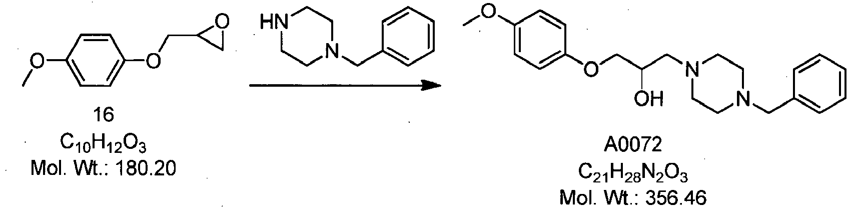

A mixture of 1-benzylpiperazine (218 mg, 1.2 mmol), **16** (180 mg, 1 mmol) and H₂O (0.2 mL) was stirred at room temperature overnight (about 18 hours). Thin-layer chromatography suggested the reaction complete. The reaction mixture was extracted with dichloromethane and washed with brine, dried with anhydrous sodium sulfate, and concentrated under vacuum to obtain 500 mg of the crude product as yellow liquid. One-half of the crude product was purified by preparative thin-layer chromatography to obtain the title product as yellow liquid (80 mg, yield: 220).

¹H NMR (400 MHz, CDCl₃) : 7.33 (m, 4H); 7.29-7.25 (m, 1H); 6.84 (m, 4H), 4.08 (td, *J* = 9.2, 4.6 Hz, 1H); 3.93 (d, *J* = 4.8 Hz, 2H); 3.77 (s, 3H); 3.53 (s, 2H); 2.73 (s, 2H); 2.64-2.37 (m, 8H); MS(ESI) calcd for C₂₁H₂₈N₂O₃ (m/z) : 356.46. found: 357.3 [M+1]⁺.

### Preparation of compound A0078

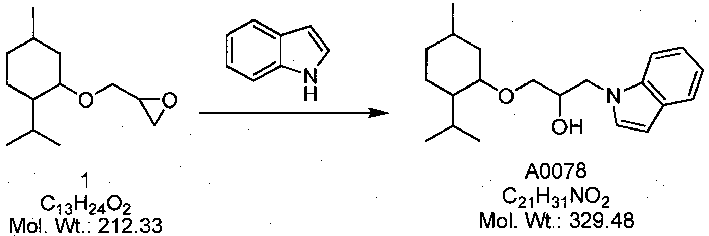

NaH (42 mg as 60% dispersion in mineral oil) was suspended in 2.0 ml of dry dimethylformamide (DMF), stirred at room temperature for 10 minutes and 1H-indole (123 mg, 1.05 mmol) was added. Next, a solution of compound **1** (148 mg, 0.7 mmol) in 1.0 ml of absolute DMF was added drop-wise. The mixture was stirred overnight (about 18 hours). The reaction mixture was heated to 75°C for 3 hours. Water was added and the solution was extracted with ethyl acetate. The organic layer was washed with water, dried over anhydrous Na₂SO₄ and concentrated. The residue was purified by silica gel to obtain the title product as yellow oil (55 mg, Yield: 24%).

¹H NMR (400 MHz, CDCl₃)_{:} 7.77 (d, *J* = 8.4 Hz, 1H); 7.54-7.51 (m, 1H); 7.37-7.33 (t, *J* = 6.4 Hz, 1H); 7.29-7.3-23 (m, 2H); 6.65 (s, 1H); 4.44-4.30 (m, 2H); 4.26 (s, 1H); 3.81-3.65 (m, 1H); 3.47-3.32 (m, 1H); 3.26-3.18 (m, 1H); 2.54 (brs, 1H); 2.39-2.30 (m, 1H); 2.20-1.16 (m, 1H); ; 1.51-1.38 (m, 1H); 1.08-0.91 (m, 13H); LCMS (ESI) calcd for C₂₁H₃₁NO₂ (m/z) : 329.48. found: 330.6 [M+1]⁺.

Each of the patents, patent applications and articles cited herein is incorporated by reference. The use of the article "a" or "an" is intended to include one or more.

The foregoing description and the examples are intended as illustrative and are not to be taken as limiting. Still other variations within the spirit and scope of this invention are possible and will readily present themselves to those skilled in the art.

## Claims

1. A compound of Formula **A** or a
pharmaceutically acceptable salt thereof 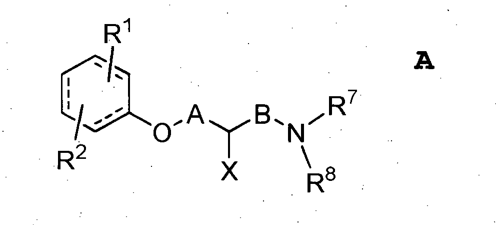 wherein
R¹ and R² are the same or different and are independently H, halogen, C₁-C₁₂ hydrocarbyl, C₁-C₆ acyl, C₁-C₆ hydrocarbyloxy, CF₃ and NR³R⁴, wherein R³ and R⁴ are the same or different and are H, C₁-C₄ hydrocarbyl, C₁-C₄ acyl, C₁-C₄ hydrocarbylsulfonyl, or R³ and R⁴ together with the depicted nitrogen form a 5-7-membered ring that optionally contains 1 or 2 additional hetero atoms that independently are nitrogen, oxygen or sulfur;
**A** and **B** are the same or different and are CH₂, CDH or CD₂;
**X** is OH or NR⁵R⁶, wherein R⁵ and R⁶ are the same or different and are H, C₁-C₄ hydrocarbyl, C₁-C₄ acyl, C₁-C₄ hydrocarbylsulfonyl, or R⁵ and R⁶ together with the depicted nitrogen form a 5-7-membered ring that optionally contains 1 or 2 additional hetero atoms that independently are nitrogen, oxygen or sulfur;
R⁷ and R⁸ together with the depicted nitrogen form a ring structure **W** that contains 4 to 14 atoms in the ring structure including the depicted nitrogen, wherein **W:** a) contains 1, 2 or 3 further hetero atoms that are independently oxygen, nitrogen or sulfur and mixtures thereof, and b) contains one or more substituent groups bonded to one or more ring atoms, in which the one or more substituents contain a total of up to 8 atoms selected from the group consisting of carbon, nitrogen, oxygen and sulfur, and mixtures thereof;
a dotted line (----) represents an optional double bond, with the proviso that R¹ and R² are other than methyl and isopropyl, respectively, when **W** is dimethyl-N-morpholinyl and the optional double bonds are absent.

2. A compound according to claim 1 or pharmaceutically acceptable salt thereof, wherein the compound is of Formula **I** 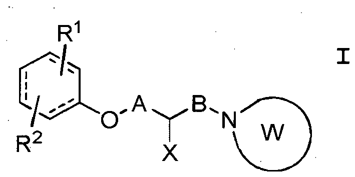
R¹ and R² are the same or different and are independently selected from the group consisting of H, halogen, C₁-C₁₂ hydrocarbyl, C₁-C₆ acyl, C₁-C₆ hydrocarbyloxy, CF₃ and NR³R⁴, wherein R³ and R⁴ are the same or different and are H, C₁-C₄ hydrocarbyl, C₁-C₄ acyl, C₁-C₄ hydrocarbylsulfonyl, or R³ and R⁴ together with the depicted nitrogen form a 5-7-membered ring that optionally contains 1 or 2 additional hetero atoms that independently are nitrogen, oxygen or sulfur;
**A** and **B** are the same or different and are CH₂, CDH or CD₂;
X is OH or NR⁵R⁶ wherein R⁵ and R⁶ are the same or different and are H, C₁-C₄ hydrocarbyl, C₁-C₄ acyl, C₁-C₄ hydrocarbylsulfonyl, or R⁵ and R⁶ together with the depicted nitrogen form a 5-7-membered ring that optionally contains 1 or 2 additional hetero atoms that independently are nitrogen, oxygen or sulfur;
**W** is a ring structure that contains up to 12 atoms in the ring structure including the depicted nitrogen,
said ring structure **W**
a) containing 1, 2 or 3 further hetero atoms that are independently oxygen, nitrogen or sulfur and mixtures thereof, and
b) including one or more substituent groups bonded to one or more ring atoms, said one or more substituent containing a total of up to 8 atoms selected from the group consisting of carbon, nitrogen, oxygen and sulfur, and mixtures thereof;
a dotted line (----) represents 1, 2, or 3 optional double bonds,
with the proviso that R¹ and R² are other than methyl and isopropyl, respectively, when **W** is or dimethyl-N-morpholinyl, and the three optional double bonds are absent.

3. The compound or its pharmaceutically acceptable salt according to claim 1 or 2, wherein said compound has Formula **II** or Formula **III** 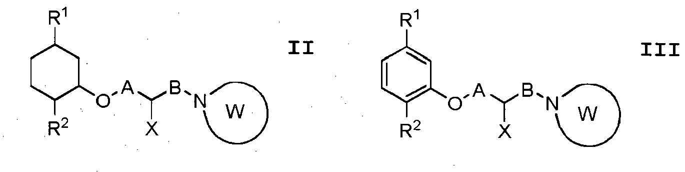 wherein **A, B, X, W,** R¹ and R² are as previously defined.

4. A compound or pharmaceutically acceptable salt thereof according to claim 1 or 2, wherein said compound is of Formula **Ia** 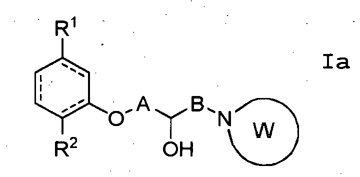 wherein
R¹ and R² are the same or different and are independently H, or C₁-C₆ hydrocarbyl;
**W** is a ring structure that contains 4 to 12 atoms in the ring structure including the depicted nitrogen, and
a) contains 1, 2 or 3 further hetero atoms that are independently oxygen, nitrogen or sulfur, and
b) includes one or more substituent groups bonded to one or more ring atoms, said one or more substituent containing a total of up to 8 atoms selected from the group consisting of carbon, nitrogen, oxygen and sulfur, and mixtures thereof;
a dotted line (----) represents 1, 2, or 3 optional double bonds,
with the proviso that R¹ and R² are other than methyl and isopropyl, respectively, when **W** is or dimethyl-N-morpholinyl and the three optional double bonds are absent.

5. The compound or its pharmaceutically acceptable salt according to any one of claims 1 to 4, wherein **W** is selected from the group consisting of

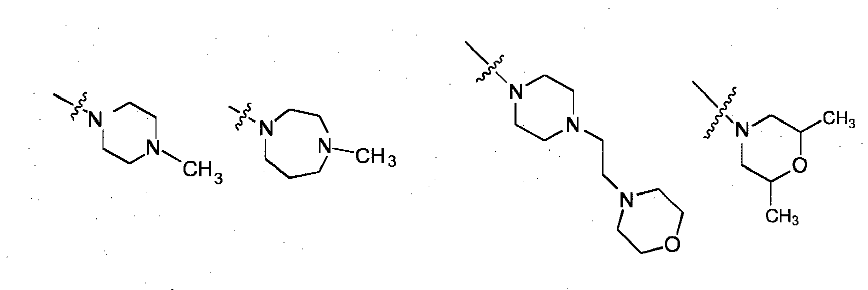

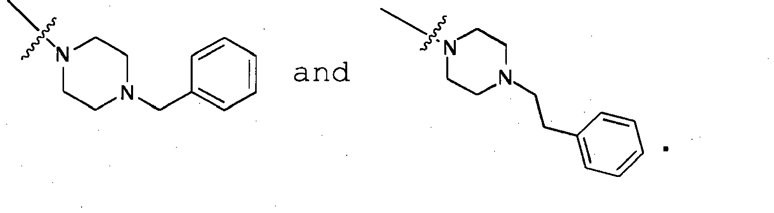

6. The compound or its pharmaceutically acceptable salt according to claim 1, 2 or 5, wherein said compound of Formula I has the structure of Formula **IIa** 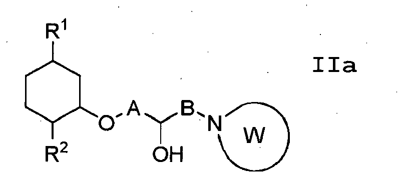 wherein **A, B,** R¹ and R² and **W** are as previously defined.

7. The compound or its pharmaceutically acceptable salt according to claim 6, wherein R¹ is methyl and R² contains 3 to 5 carbon atoms.

8. The compound or its pharmaceutically acceptable salt according to claim 6 that is selected from the group consisting of

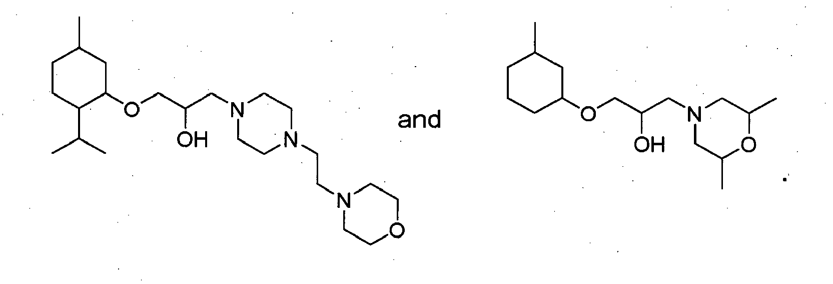

9. The compound or its pharmaceutically acceptable salt according to any one of claims 1 to 5, wherein said compound of Formula **I** has the structure of Formula **IIIa** 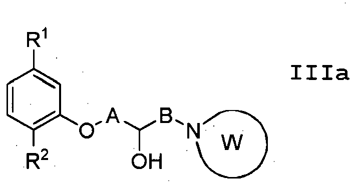 wherein **A, B,** R¹ and R² and **W** are as previously defined.

10. The compound or its pharmaceutically acceptable salt according to any one of claims 1 to 5, wherein when one optional double bond is present, three double bonds are present.

11. The compound or its pharmaceutically acceptable salt according to any one of claims 1 to 5 and 9, wherein said three double bonds are present and one of R¹ and R² is H.

12. The compound or its pharmaceutically acceptable salt according to claim 11, wherein one of R¹ and R² is NR³R⁴.

13. The compound or its pharmaceutically acceptable salt according to any one of claims 1 to 5
and 10, wherein said three double bonds are present and both of R¹ and R² are halogen.

14. The compound or its pharmaceutically acceptable salt according to claim 9, wherein one of R¹ and R² is H, and the other contains 3 to 5 carbon atoms.

15. The compound or its pharmaceutically acceptable salt according to claim 9 that is selected from the group consisting of 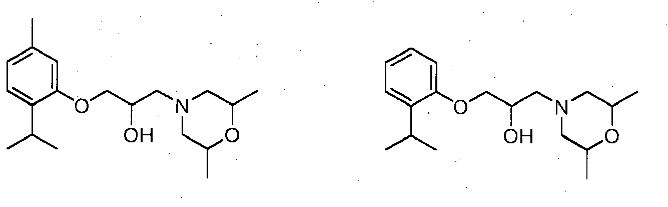 and

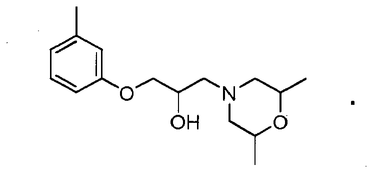

16. A pharmaceutical composition comprising a compound or a pharmaceutically acceptable salt thereof according to any one of claims 1 to 15 dissolved or dispersed in a physiologically tolerable carrier.

17. A pharmaceutical composition comprising a compound or a pharmaceutically acceptable salt thereof according to any one of claims 1 to 15 dissolved or dispersed in a physiologically tolerable carrier, for use in a method of reducing one or both of pain and inflammation in a host mammal.

18. Use of a pharmaceutical composition comprising a compound or a pharmaceutically acceptable salt thereof according to any one of claims 1 to 15 dissolved or dispersed in a physiologically tolerable carrier, in the manufacture of a medicament for use in a method of reducing one or both of pain and inflammation in a host mammal.

19. The composition for use according to claim 17 or the use according to claim 18, wherein said composition is administered a plurality of times over a period of days.

20. A compound or its pharmaceutically acceptable salt, wherein the compound has the structural formula

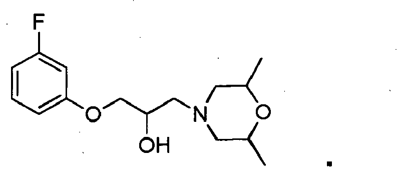

## Patentansprüche

1. Verbindung der Formel **A** oder ein pharmazeutisch unbedenkliches Salz davon 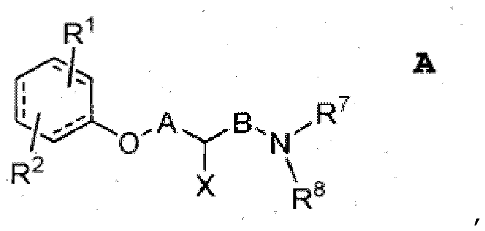 wobei
R¹ und R² gleich oder verschieden sind und unabhängig voneinander für H, Halogen, C₁-C₁₂-Hydrocarbyl, C₁-C₆-Acyl, C₁-C₆-Hydrocarbyloxy, CF₃ oder NR³R⁴ stehen, wobei R³ und R⁴ gleich oder verschieden sind und für H, C₁-C₄-Hydrocarbyl, C₁-C₄-Acyl oder C₁-C₄-Hydrocarbylsulfonyl stehen oder R³ und R⁴ zusammen mit dem gezeigten Stickstoff einen 5- bis 7-gliedrigen Ring bilden, der gegebenenfalls 1 oder 2 zusätzliche Heteroatome enthält, bei denen es sich unabhängig voneinander um Stickstoff, Sauerstoff oder Schwefel handelt;
**A** und **B** gleich oder verschieden sind und für CH₂, CDH oder CD₂ stehen;
**X** für OH oder NR⁵R⁶ steht, wobei R⁵ und R⁶ gleich oder verschieden sind und für H, C₁-C₄-Hydrocarbyl, C₁-C₄-Acyl oder C₁-C₄-Hydrocarbylsulfonyl stehen oder R⁵ und R⁶ zusammen mit dem gezeigten Stickstoff einen 5- bis 7-gliedrigen Ring bilden, der gegebenenfalls 1 oder 2 zusätzliche Heteroatome enthält, bei denen es sich unabhängig voneinander um Stickstoff, Sauerstoff oder Schwefel handelt;
R⁷ und R⁸ zusammen mit dem gezeigten Stickstoff eine Ringstruktur **W** bilden, die einschließlich des gezeigten Stickstoffs 4 bis 14 Atome in der Ringstruktur enthält, wobei **W:** a) 1, 2 oder 3 weitere Heteroatome enthält, bei denen es sich unabhängig voneinander um Sauerstoff, Stickstoff oder Schwefel und Mischungen davon handelt, und b) eine oder mehrere an ein oder mehrere Ringatome gebundene Substituentengruppen enthält, wobei der eine oder die mehreren Substituenten insgesamt bis zu 8 aus der aus Kohlenstoff, Stickstoff, Sauerstoff und Schwefel und Mischungen davon bestehenden Gruppe ausgewählte Atome enthält;
eine gestrichelte Linie (----) für eine gegebenenfalls vorhandene Doppelbindung steht, mit der Maßgabe, dass R¹ und R² nicht für Methyl bzw. Isopropyl stehen, wenn **W** für Dimethyl-N-morpholinyl steht und die gegebenenfalls vorhandenen Doppelbindungen fehlen.

2. Verbindung nach Anspruch 1 oder pharmazeutisch unbedenkliches Salz davon, wobei die Verbindung die Formel **I** aufweist 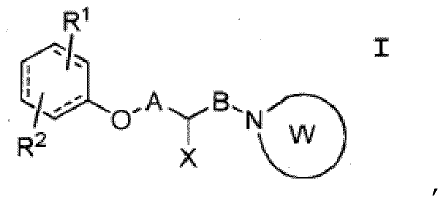
R¹ und R² gleich oder verschieden sind und unabhängig voneinander aus der aus H, Halogen, C₁-C₁₂-Hydrocarbyl, C₁-C₆-Acyl, C₁-C₆-Hydrocarbyloxy, CF₃ und NR³R⁴ bestehenden Gruppe ausgewählt sind, wobei R³ und R⁴ gleich oder verschieden sind und für H, C₁-C₄-Hydrocarbyl, C₁-C₄-Acyl oder C₁-C₄-Hydrocarbylsulfonyl stehen oder R³ und R⁴ zusammen mit dem gezeigten Stickstoff einen 5- bis 7-gliedrigen Ring bilden, der gegebenenfalls 1 oder 2 zusätzliche Heteroatome enthält, bei denen es sich unabhängig voneinander um Stickstoff, Sauerstoff oder Schwefel handelt;
**A** und **B** gleich oder verschieden sind und für CH₂, CDH oder CD₂ stehen;
**X** für OH oder NR⁵R⁶ steht, wobei R⁵ und R⁶ gleich oder verschieden sind und für H, C₁-C₄-Hydrocarbyl, C₁-C₄-Acyl oder C₁-C₄-Hydrocarbylsulfonyl stehen oder R⁵ und R⁶ zusammen mit dem gezeigten Stickstoff einen 5- bis 7-gliedrigen Ring bilden, der gegebenenfalls 1 oder 2 zusätzliche Heteroatome enthält, bei denen es sich unabhängig voneinander um Stickstoff, Sauerstoff oder Schwefel handelt;
**W** für eine Ringstruktur steht, die einschließlich des gezeigten Stickstoffs bis zu 12 Atome in der Ringstruktur enthält,
wobei die Ringstruktur **W**
a) 1, 2 oder 3 weitere Heteroatome enthält, bei denen es sich unabhängig voneinander um Sauerstoff, Stickstoff oder Schwefel und Mischungen davon handelt, und
b) ein oder mehrere Substituentengruppen gebunden an ein oder mehrere Ringatome einschließt, wobei der eine oder die mehreren Substituenten insgesamt bis zu 8 aus der aus Kohlenstoff, Stickstoff, Sauerstoff und Schwefel und Mischungen davon bestehenden Gruppe ausgewählte Atome enthält;
eine gestrichelte Linie (----) für 1, 2 oder 3 gegebenenfalls vorhandene Doppelbindungen steht,
mit der Maßgabe, dass R¹ und R² nicht für Methyl bzw. Isopropyl stehen, wenn **W** für Dimethyl-N-morpholinyl steht und die drei gegebenenfalls vorhandenen Doppelbindungen fehlen.

3. Verbindung oder deren pharmazeutisch unbedenkliches Salz nach Anspruch 1 oder 2, wobei die Verbindung die Formel **II** oder die Formel **III** aufweist 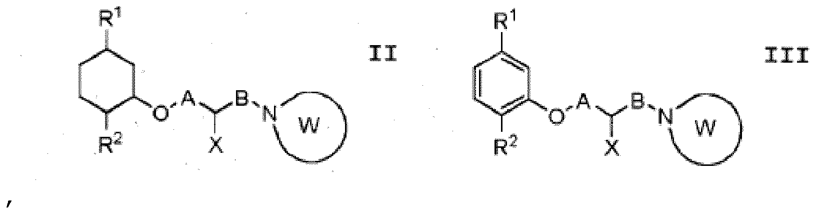 wobei A, B, X, W, R¹ und R² wie oben definiert sind.

4. Verbindung oder pharmazeutisch unbedenkliches Salz davon nach Anspruch 1 oder 2, wobei die Verbindung die Formel **Ia** aufweist 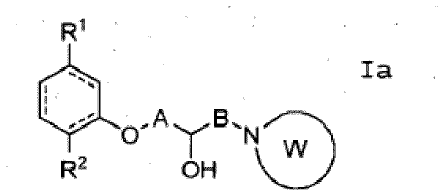 wobei R¹ und R² gleich oder verschieden sind und unabhängig voneinander für H oder C₁-C₆-Hydrocarbyl stehen;
**W** für eine Ringstruktur steht, die einschließlich des gezeigten Stickstoffs 4 bis 12 Atome in der Ringstruktur enthält und
a) 1, 2 oder 3 weitere Heteroatome enthält, bei denen es sich unabhängig voneinander um Sauerstoff, Stickstoff oder Schwefel handelt, und
b) ein oder mehrere Substituentengruppen gebunden an ein oder mehrere Ringatome einschließt, wobei der eine oder die mehreren Substituenten insgesamt bis zu 8 aus der aus Kohlenstoff, Stickstoff, Sauerstoff und Schwefel und Mischungen davon bestehenden Gruppe ausgewählte Atome enthält;
eine gestrichelte Linie (----) für 1, 2 oder 3 gegebenenfalls vorhandene Doppelbindungen steht,
mit der Maßgabe, dass R¹ und R² nicht für Methyl bzw. Isopropyl stehen, wenn **W** für Dimethyl-N-morpholinyl steht und die drei gegebenenfalls vorhandenen Doppelbindungen fehlen.

5. Verbindung oder deren pharmazeutisch unbedenkliches Salz nach einem der Ansprüche 1 bis 4, wobei **W** aus der aus 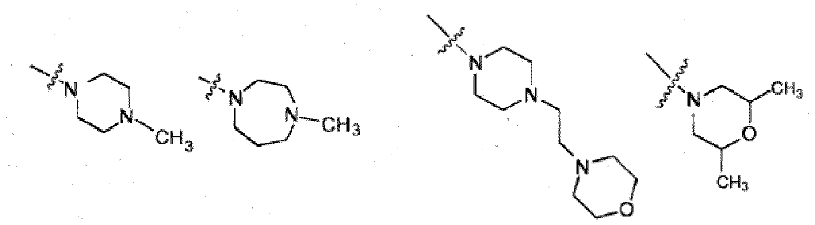 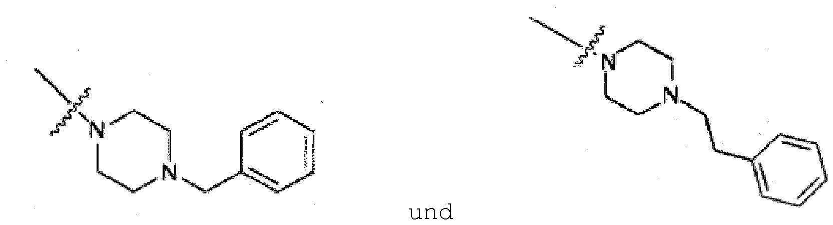 bestehenden Gruppe ausgewählt ist.

6. Verbindung oder deren pharmazeutisch unbedenkliches Salz nach Anspruch 1, 2 oder 5, wobei die Verbindung der Formel **I** die Struktur der Formel **IIa** aufweist 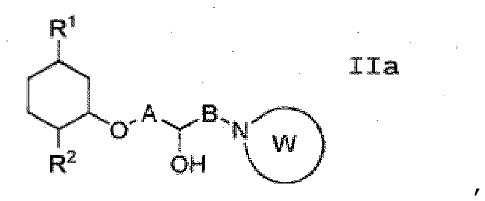 wobei **A, B,** R¹ und R² und **W** wie oben definiert sind.

7. Verbindung oder deren pharmazeutisch unbedenkliches Salz nach Anspruch 6, wobei R¹ für Methyl steht und R² 3 bis 5 Kohlenstoffatome enthält.

8. Verbindung oder deren pharmazeutisch unbedenkliches Salz nach Anspruch 6, ausgewählt aus der Gruppe bestehend aus

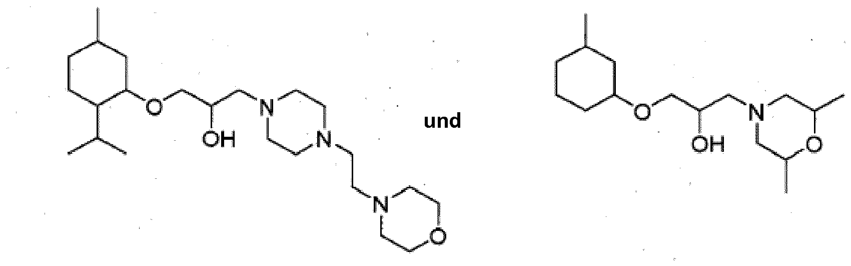

9. Verbindung oder deren pharmazeutisch unbedenkliches Salz nach einem der Ansprüche 1 bis 5, wobei die Verbindung der Formel **I** die Struktur der Formel **IIIa** aufweist 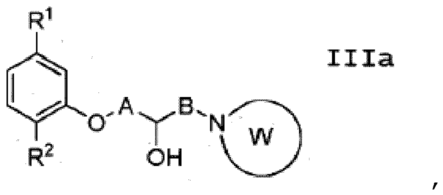 wobei **A, B,** R¹ und R² und **W** wie oben definiert sind.

10. Verbindung oder deren pharmazeutisch unbedenkliches Salz nach einem der Ansprüche 1 bis 5, wobei, wenn eine gegebenenfalls vorhandene Doppelbindung vorhanden ist, drei Doppelbindungen vorhanden sind.

11. Verbindung oder deren pharmazeutisch unbedenkliches Salz nach einem der Ansprüche 1 bis 5 und 9, wobei die drei Doppelbindungen vorhanden sind und einer der Reste R¹ und R² für H steht.

12. Verbindung oder deren pharmazeutisch unbedenkliches Salz nach Anspruch 11, wobei einer der Reste R¹ und R² für NR³R⁴ steht.

13. Verbindung oder deren pharmazeutisch unbedenkliches Salz nach einem der Ansprüche 1 bis 5 und 10, wobei die drei Doppelbindungen vorhanden sind und sowohl R¹ als auch R² für Halogen stehen.

14. Verbindung oder deren pharmazeutisch unbedenkliches Salz nach Anspruch 9, wobei einer der Reste R¹ und R² für H steht und der andere 3 bis 5 Kohlenstoffatome enthält.

15. Verbindung oder deren pharmazeutisch unbedenkliches Salz nach Anspruch 9, ausgewählt aus der Gruppe bestehend aus 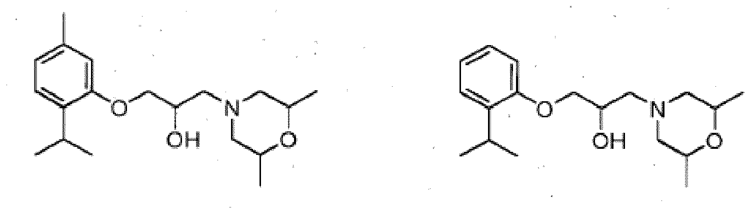 und

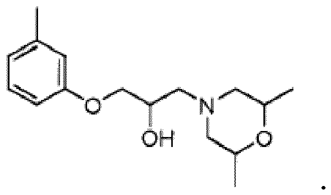

16. Pharmazeutische Zusammensetzung, umfassend eine Verbindung oder ein pharmazeutisch unbedenkliches Salz davon nach einem der Ansprüche 1 bis 15 gelöst oder dispergiert in einem physiologisch tolerierbaren Träger.

17. Pharmazeutische Zusammensetzung, umfassend eine Verbindung oder ein pharmazeutisch unbedenkliches Salz davon nach einem der Ansprüche 1 bis 15 gelöst oder dispergiert in einem physiologisch tolerierbaren Träger, zur Verwendung bei einem Verfahren zur Verminderung von Schmerzen und/oder Entzündung bei einem Wirtssäugetier.

18. Verwendung einer pharmazeutischen Zusammensetzung, umfassend eine Verbindung oder ein pharmazeutisch unbedenkliches Salz davon nach einem der Ansprüche 1 bis 15 gelöst oder dispergiert in einem physiologisch tolerierbaren Träger, zur Herstellung eines Medikaments zur Verwendung bei einem Verfahren zur Verminderung von Schmerzen und/oder Entzündung bei einem Wirtssäugetier.

19. Zusammensetzung zur Verwendung nach Anspruch 17 oder Verwendung nach Anspruch 18, wobei die Zusammensetzung über mehrere Tage mehrmals verabreicht wird.

20. Verbindung oder deren pharmazeutisch unbedenkliches Salz, wobei die Verbindung die
Strukturformel 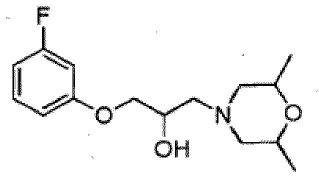 aufweist.

## Revendications

1. Composé de Formule **A** ou l'un de ses sels de qualité pharmaceutique : 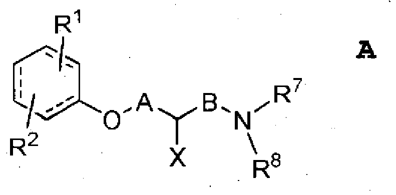 où
chacun des radicaux R¹ et R² est identique ou différent et représente indépendamment H ou un groupement halogéno, hydrocarbyle en C₁-C₁₂, acyle en C₁-C₆, hydrocarbyloxy en C₁-C_{6,} CF₃ et NR³R⁴, où chacun des radicaux R³ et R⁴ est identique ou différent et représente H ou un groupement hydrocarbyle en C₁-C₄, acyle en C₁-C₄, hydrocarbylsulfonyle en C₁-C₄,
ou R³ et R⁴ forment ensemble et avec l'atome d'azote illustré un cycle comportant entre 5 et 7 chaînons ainsi qu'éventuellement 1 ou 2 hétéroatomes supplémentaires représentant indépendamment des atomes d'azote, d'oxygène ou de soufre ;
chacun des radicaux **A** et **B** est identique ou différent et représente CH₂, CDH ou CD₂ ;
**X** représente un groupement OH ou NR⁵R⁶, où chacun des radicaux R⁵ et R⁶ est identique ou différent et représente H ou un groupement hydrocarbyle en C₁-C₄, acyle en C₁-C₄, hydrocarbylsulfonyle en C₁-C₄, ou R⁵ et R⁶ forment ensemble et avec l'atome d'azote illustré un cycle comportant entre 5 et 7 chaînons ainsi qu'éventuellement 1 ou 2 hétéroatomes supplémentaires représentant indépendamment des atomes d'azote, d'oxygène ou de soufre ;
R⁷ et R⁸ forment ensemble et avec l'azote illustré une structure cyclique **W** qui comporte entre 4 et 14 chaînons, y compris l'atome d'azote illustré, où **W :** a)
comporte 1, 2 ou 3 hétéroatomes supplémentaires qui représentent indépendamment des atomes d'oxygène, d'azote ou de soufre ou leurs mélanges, et b)
comporte un ou plusieurs groupements substituants liés à un ou plusieurs atomes de cycle, le ou les substituants comportant un total d'atomes pouvant aller jusqu'à 8 et choisis dans le groupe constitué par le carbone, l'azote, l'oxygène et le soufre, et leurs mélanges ;
un segment en pointillé (----) représente une double liaison optionnelle, à la condition que R¹ et R² soient respectivement différents des groupements méthyle et isopropyle lorsque **W** représente un groupement diméthyl-N-morpholinyle et que les doubles liaisons optionnelles sont absentes.

2. Composé conforme à la revendication 1 ou l'un de ses sels de qualité pharmaceutique, où le composé répond à la Formule **I** 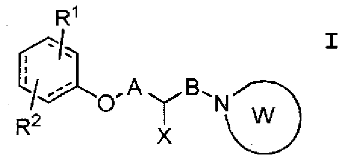 chacun des radicaux R¹ et R² est identique ou différent et est indépendamment choisi dans le groupe constitué par H et les groupements halogéno, hydrocarbyle en C₁-C₁₂, acyle en C₁-C₆, hydrocarbyloxy en C₁-C₆, CF₃ et NR³R⁴, où chacun des radicaux R³ et R⁴ est identique ou différent et représente H ou un groupement hydrocarbyle en C₁-C₄, acyle en C₁-C₄, hydrocarbylsulfonyle en C₁-C₄,
ou
R³ et R⁴ forment ensemble et avec l'atome d'azote illustré un cycle comportant entre 5 et 7 chaînons ainsi qu'éventuellement 1 ou 2 hétéroatomes supplémentaires représentant indépendamment des atomes d'azote, d'oxygène ou de soufre ;
chacun des radicaux **A** et **B** est identique ou différent et représente CH₂, CDH ou CD₂ ;
**X** représente un groupement OH ou NR⁵R⁶, où chacun des radicaux R⁵ et R⁶ est identique ou différent et représente H ou un groupement hydrocarbyle en C₁-C₄, acyle en C₁-C₄, hydrocarbylsulfonyle en C₁-C₄, ou R⁵ et R⁶ forment ensemble et avec l'atome d'azote illustré un cycle comportant entre 5 et 7 chaînons ainsi qu'éventuellement 1 ou 2 hétéroatomes supplémentaires représentant indépendamment des atomes d'azote, d'oxygène ou de soufre ;
**W** représente une structure cyclique comportant jusqu'à 12 chaînons y compris l'atome d'azote illustré,
ladite structure cyclique **W**
a) comportant 1, 2 ou 3 hétéroatomes supplémentaires qui représentent indépendamment des atomes d'oxygène, d'azote ou de soufre et leurs mélanges, et
b) incluant un ou plusieurs groupements substituants liés à un ou plusieurs atomes de cycle, le ou lesdits substituants comportant un total d'atomes pouvant aller jusqu'à 8 et choisis dans le groupe constitué par le carbone, l'azote, l'oxygène et le soufre, et leurs mélanges ;
un segment en pointillé (----) représente 1, 2 ou 3 doubles liaisons optionnelles,
à la condition que R¹ et R² soient respectivement différents des groupements méthyle et isopropyle lorsque **W** représente un groupement diméthyl-N-morpholinyle et que les trois doubles liaisons optionnelles sont absentes.

3. Composé ou son sel de qualité pharmaceutique conforme à la revendication 1 ou 2, où ledit composé répond à la Formule **II** ou à la Formule **III** 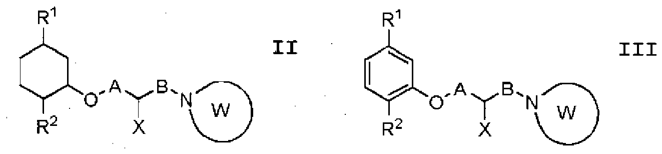 où **A, B, X, W,** R¹ et R² sont tels que définis précédemment.

4. Composé ou l'un de ses sels de qualité pharmaceutique conforme à la revendication 1 ou 2, où ledit composé répond à la Formule **Ia** 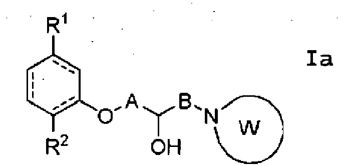 où
chacun des radicaux R¹ et R² est identique ou différent et représente indépendamment H ou un groupement hydrocarbyle en C₁-C₆ ;
**W** représente une structure cyclique comportant entre 4 et 12 chaînons y compris l'atome d'azote illustré, et
a) comporte 1, 2 ou 3 hétéroatomes supplémentaires qui représentent indépendamment des atomes d'oxygène, d'azote ou de soufre, et
b) inclut un ou plusieurs groupements substituants liés à un ou plusieurs atomes de cycle, le ou lesdits substituants comportant un total d'atomes pouvant aller jusqu'à 8 et choisis dans le groupe constitué par le carbone, l'azote, l'oxygène et le soufre, et leurs mélanges ;
un segment en pointillé (----) représente 1, 2 ou 3 doubles liaisons optionnelles,
à la condition que R¹ et R² soient respectivement différents des groupements méthyle et isopropyle lorsque **W** représente un groupement diméthyl-N-morpholinyle et que les trois doubles liaisons optionnelles sont absentes.

5. Composé ou son sel de qualité pharmaceutique conforme à l'une quelconque des revendications 1 à 4, où **W** est choisi dans le groupe constitué par

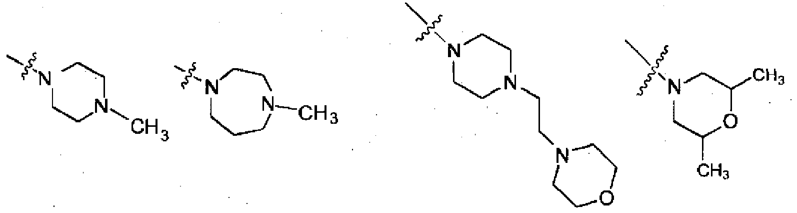

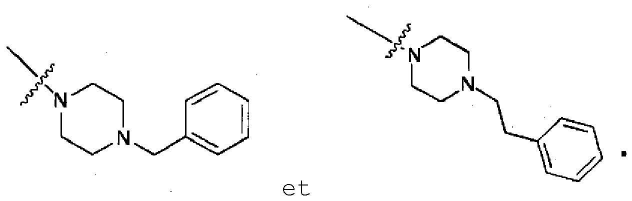

6. Composé ou son sel de qualité pharmaceutique conforme à la revendication 1, 2 ou 5, où ledit composé de Formule I présente la structure de la Formule **IIa** 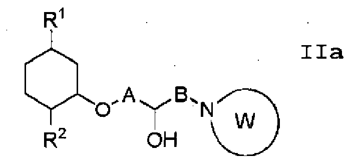 où **A, B,** R¹ et R² et **W** sont tels que définis précédemment.

7. Composé ou son sel de qualité pharmaceutique conforme à la revendication 6, où R¹ représente un groupement méthyle et R² comporte entre 3 et 5 atomes de carbone.

8. Composé ou son sel de qualité pharmaceutique conforme à la revendication 6, choisi dans le groupe constitué par

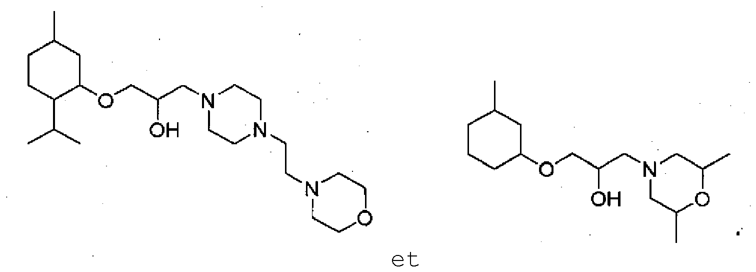

9. Composé ou son sel de qualité pharmaceutique conforme à l'une quelconque des revendications 1 à 5, où ledit composé de Formule **I** présente la structure de la Formule **IIIa** 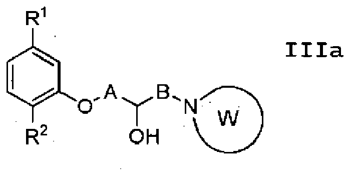 où **A, B,** R¹ et R² et **W** sont tels que définis précédemment.

10. Composé au son sel de qualité pharmaceutique conforme à l'une quelconque des revendications 1 à 5, où lorsqu'une double liaison optionnelle est présente, trois doubles liaisons sont présentes.

11. Composé ou son sel de qualité pharmaceutique conforme à l'une quelconque des revendications 1 à 5 et 9, où lesdites trois doubles liaisons sont présentes et l'un des radicaux R¹ et R² représente H.

12. Composé ou son sel de qualité pharmaceutique conforme à la revendication 11, où l'un des radicaux R¹ et R² représente NR³R⁴.

13. Composé ou son sel de qualité pharmaceutique conforme à l'une quelconque des revendications 1 à 5 et 10, où lesdites trois doubles liaisons sont présentes et les deux radicaux R¹ et R² représentent des groupements halogéno.

14. Composé ou son sel de qualité pharmaceutique conforme à la revendication 9, où l'un des radicaux R¹ et R² représente H et l'autre comporte entre 3 et 5 atomes de carbone.

15. Composé ou son sel de qualité pharmaceutique conforme à la revendication 9, choisi dans le groupe constitué par 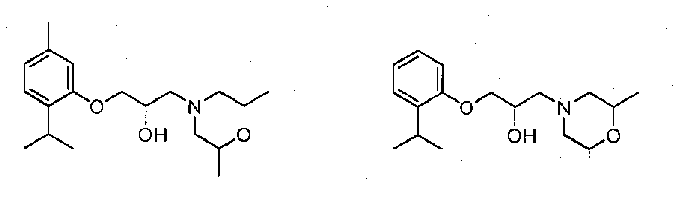 et

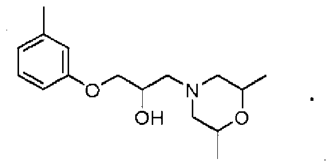

16. Composition pharmaceutique comprenant un composé ou l'un de ses sels de qualité pharmaceutique conforme à l'une quelconque des revendications 1 à 15, dissous ou dispersé dans un vecteur de qualité physiologique.

17. Composition pharmaceutique comprenant un composé ou l'un de ses sels de qualité pharmaceutique conforme à l'une quelconque des revendications 1 à 15 dissous ou dispersé dans un vecteur de qualité physiologique, pour emploi dans une méthode de soulagement de la douleur, de l'inflammation ou des deux chez un mammifère hôte.

18. Emploi d'une composition pharmaceutique comprenant un composé ou l'un de ses sels de qualité pharmaceutique conforme à l'une quelconque des revendications 1 à 15 dissous ou dispersé dans un vecteur de qualité physiologique, dans la fabrication d'un médicament pour emploi dans une méthode de soulagement de la douleur, de l'inflammation ou des deux chez un mammifère hôte.

19. Composition pour emploi conforme à la revendication 17 ou emploi conforme à la revendication 18, où ladite composition est administrée une multitude de fois sur une durée de plusieurs jours.

20. Composé ou son sel de qualité pharmaceutique, où le composé répond à la formule structurelle
